(19) Europäisches Patentamt
European Patent Office
Office européen des brevets

(11) Veröffentlichungsnummer: **0 100 906**
**B1**

(12) # EUROPÄISCHE PATENTSCHRIFT

(45) Veröffentlichungstag der Patentschrift: **13.05.87**

(21) Anmeldenummer: **83106920.8**

(22) Anmeldetag: **14.07.83**

(51) Int. Cl.⁴: **C 07 D 487/04,** C 07 D 487/14, A 61 K 31/55 // C07D209/38, C07D265/26 ,(C07D487/04, 243:00, 235:00),(C07D487/14, 243:00, 235:00,205:00), (C07D487/14, 243:00,235:00, 209:00)

(54) **Tri- und tetracyclische Imidazo(1,5-a)(1,4)Benzodiazepin-1-carbonsäurederivate.**

(30) Priorität: **21.07.82 CH 4460/82**

(43) Veröffentlichungstag der Anmeldung: **22.02.84 Patentblatt 84/8**

(45) Bekanntmachung des Hinweises auf die Patenterteilung: **13.05.87 Patentblatt 87/20**

(84) Benannte Vertragsstaaten: **AT BE CH DE FR GB IT LI LU NL SE**

(56) Entgegenhaltungen:
**EP - A - 0 027 214**
**EP - A - 0 059 386**
**EP - A - 0 059 387**
**EP - A - 0 059 389**
**EP - A - 0 059 391**

(73) Patentinhaber: **F. HOFFMANN-LA ROCHE & CO. Aktiengesellschaft, CH-4002 Basel (CH)**

(72) Erfinder: **Hunkeler, Walter, Dr., Im Stigler 32, CH-4312 Magden (CH)**
Erfinder: **Kyburz, Emilio, Dr., Unterer Rebbergweg 127, CH-4153 Reinach (CH)**

(74) Vertreter: **Lederer, Franz, Dr. et al, Vanderwerth, Lederer & Riederer Patentanwälte Lucile-Grahn-Strasse 22, D-8000 München 80 (DE)**

**EP 0 100 906 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft Imidazobenzodiazepine. Im speziellen betrifft sie tri- und tetracyclische Imidazobenzodiazepine der allgemeinen Formel

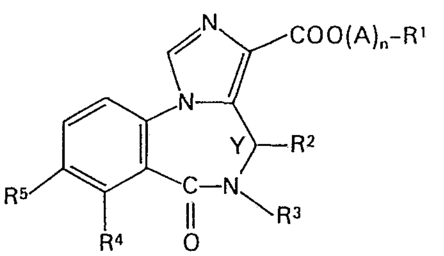

I

worin A niederes Alkylen, n die Zahl 0 oder 1, $R^1$ niederes Alkinyl, niederes Alkenyl, Aryl, jeweils gegebenenfalls durch niederes Alkyl substituiertes $(C_{3-8})$-Cycloalkyl oder $(C_{5-8})$-Cycloalkenyl oder einen gegebenenfalls durch niederes Alkyl substituierten 5- oder 6gliedrigen gesättigten oder ungesättigten Heterocyclus, der als Ringglied ein Sauerstoff- oder Schwefelatom enthält, $R^4$ und $R^5$ je Wasserstoff, Halogen, Trifluormethyl, Cyano, Nitro, Amino oder niederes Alkyl und entweder $R^2$ Wasserstoff und $R^3$ niederes Alkyl oder $R^2$ und $R^3$ zusammen Dimethylen, Trimethylen oder Propenylen bedeuten, wobei die Verbindungen der Formel I, worin $R^2$ und $R^3$ zusammen Dimethylen, Trimethylen oder Propenylen bedeuten, bezüglich des mit γ bezeichneten Kohlenstoffatoms die (S)- oder (R,S)-Konfiguration aufweisen, und pharmazeutisch annehmbare Säureadditionssalze davon.

Diese Verbindungen sind neu und zeichnen sich durch wertvolle pharmakodynamische Eigenschaften aus. Sie können bei der Bekämpfung bzw. Verhütung von Krankheiten verwendet werden.

Gegenstand der vorliegenden Erfindung sind Verbindungen der allgemeinen Formel I und pharmazeutisch annehmbare Säureadditionssalze davon als solche und als pharmazeutische Wirkstoffe, die Herstellung dieser Verbindungen, ferner Arzneimittel, enthaltend eine Verbindung der allgemeinen Formel I oder ein pharmazeutisch annehmbares Säureadditionssalz davon, die Herstellung solcher Arzneimittel und die Verwendung der erfindungsgemässen Stoffe bei der Bekämpfung bzw. Verhütung von Krankheiten.

Der Ausdruck «nieder» in Kombinationen, wie «niederes Alkyl», «niedere Alkylgruppen», «niederes Alkylen», «niederes Alkyloxy», «niederes Alkinyl», «niederes Alkenyl» und dergleichen, bedeutet, dass die so bezeichneten Reste höchstens 7, vorzugsweise höchstens 4 Kohlenstoffatome aufweisen. Die Ausdrücke «Alkyl», «Alkylgruppe» und dergleichen bezeichnen geradkettige oder verzweigte, gesättigte Kohlenwasserstoffreste wie Methyl, Äthyl, n-Propyl, Isopropyl, n-Butyl, s-Butyl, t-Butyl und dergleichen. Der Ausdruck «Alkyloxy» bezeichnet über ein Sauerstoffatom gebundene Alkylgruppe, wie z.B. Methoxy, Äthoxy, Isopropoxy und dergleichen. Der Ausdruck «Alkylen» bezeichnet zweiwertige, geradkettige oder verzweigte, gesättigte Kohlenwasserstoffreste wie Methylen, 1,2-Äthylen, Äthyliden und dergleichen. Der Ausdruck «Alkinyl» bezeichnet geradkettige oder verzweigte Kohlenwasserstoffreste mit einer Dreifachbindung wie Propargyl und dergleichen. Der Ausdruck «Alkenyl» bezeichnet geradkettige oder verzweigte Kohlenwasserstoffreste mit einer Doppelbindung wie Allyl und dergleichen.

Der Ausdruck «$(C_{3-8})$-Cycloalkyl» bezeichnet gesättigte cyclische Kohlenwasserstoffreste mit 3 bis 8 Ringglieder, d.h. Cyclopropyl, Cyclobutyl, Cyclopentyl, Cyclohexyl, Cycloheptyl und Cyclooctyl. Der Ausdruck «$(C_{5-8})$-Cycloalkenyl» bezeichnet ungesättigte cyclische Kohlenwasserstoffreste mit 5 bis 8 Ringglieder wie 2-Cyclohexen-1-yl und dergleichen. Der Ausdruck «Aryl» bezeichnet gegebenenfalls durch Halogen, niederes Alkyloxy, niederes Alkyl, Trifluormethyl oder Nitro substituiertes, vorzugsweise monosubstituiertes Phenyl, wie Phenyl, o-Chlorphenyl, m-Chlorphenyl, p-Chlorphenyl, o-Methoxyphenyl, m-Methoxyphenyl, p-Methoxyphenyl und dergleichen. Der Ausdruck «5- oder 6gliedriger gesättigter oder ungesättigter Heterocyclus, der als Ringglied ein Sauerstoff- oder Schwefelatom enthält» umfasst heterocyclische Reste mit einem Heteroatom, wie Tetrahydro-2H-pyran-4-yl und dergleichen. Der Ausdruck «Halogen» bedeutet Fluor, Chlor, Brom oder Jod.

In einer bevorzugten Ausführungsform bedeutet entweder n die Zahl 0 oder die Zahl 1, wobei A gegebenenfalls durch niederes Alkyl substituiertes Methylen oder 1,2-Äthylen bedeutet. $R^1$ bedeutet vorzugsweise Alkinyl, Phenyl oder jeweils gegebenenfalls durch niederes Alkyl substituiertes $(C_{3-8})$-Cycloalkyl oder $(C_{5-8})$-Cycloalkenyl, wobei die Bedeutungsmöglichkeit $(C_{3-6})$-Cycloalkyl besonders bevorzugt ist. In einer ganz besonders bevorzugten Ausführungsform bedeutet die Gruppe $-(A)_n-R^1$ Cyclohexyl, 2-Cyclohexen-1-yl, Cyclopropylmethyl, 1-Cyclopropyläthyl oder 2-Cyclopropyläthyl.

$R^2$ und $R^3$ bedeuten vorzugsweise zusammen Dimethylen, Trimethylen oder Propenylen, wobei die entsprechenden Verbindungen der Formel I bezüglich des mit γ bezeichneten Kohlenstoffatoms vorzugsweise die (S)-Konfiguration aufweisen. Die bevorzugte Bedeutungsmöglichkeit von $R^4$ ist Wasserstoff, Halogen, Trifluormethyl, Nitro, Cyano oder niederes Alkyl. $R^5$ bedeutet vorzugsweise Wasserstoff oder Halogen.

Eine im Rahmen der vorliegenden Erfindung ganz besonders bevorzugte Verbindung ist:

Cyclopropylmethyl (S)-8-chlor-12,12a-dihydro-9-oxo-9H,11H-azeto[2,1-c]imidazo[1,5-a][1,4]benzodiazepin-1-carboxylat.

Weitere besonders bevorzugte Verbindungen sind:

Cyclohexyl (S)-8-brom-11,12,13,13a-tetrahydro-9-oxo-9H-imidazo[1,5-a]pyrrolo[2,1-c][1,4] benzodiazepin-1-carboxylat,
(R,S)-2-Cyclohexen-1-yl (S)-8-chlor-11,12,13,13a-tetrahydro-9-oxo-9H-imidazo[1,5-a]pyrrolo [2,1-c][1,4]-benzodiazepin-1-carboxylat,
Cyclohexyl (S)-8-chlor-12,12a-dihydro-9-oxo-9H,11H-azeto[2,1-c]imidazo[1,5-a][1,4]benzodiazepin-1-carboxylat,
Cyclohexyl (S)-7-fluor-12,12a-dihydro-9-oxo-9H,11H-azeto[2,1-c]imidazo[1,5-a][1,4]benzodiazepin-1-carboxylat,
Cyclohexyl (S)-8-chlor-7-fluor-11,12,13,13a-tetrahydro-9-oxo-9H-imidazo[1,5-a]pyrrolo [2,1-c][1,4]benzodiazepin-1-carboxylat,
(R,S)-1-Cyclopropyläthyl (S)-8-chlor-12,12a-dihydro-9-oxo-9H,11H-azeto[2,1-c]imidazo [1,5-a][1,4]benzodiazepin-1-carboxylat,
Cyclohexyl (S)-12,12a-dihydro-8-jod-9-oxo-9H,11H-azeto[2,1-c]imidazo[1,5-a][1,4]benzodiazepin-1-carboxylat,
Cyclopropylmethyl (S)-7-fluor-11,12,13,13a-tetrahydro-9-oxo-9H-imidazo[1,5-a]pyrrolo[2,1-c][1,4]benzodiazepin-1-carboxylat,
(R,S)-1-Cyclopropyläthyl (S)-8-chlor-11,12,13,13a-tetrahydro-9-oxo-9H-imidazo[1,5-a] pyrrolo[2,1-c][1,4]benzodiazepin-1-carboxylat,
Cyclopropylmethyl (S)-7-fluor-12,12a-dihydro-9-oxo-9H,11H-azeto[2,1-c]imidazo[1,5-a] [1,4]benzodiazepin-1-carboxylat und
Cyclohexyl (S)-11,12,13,13a-tetrahydro-9-oxo-8-(trifluormethyl)-9H-imidazo[1,5-a]pyrrolo[2,1-c][1,4]benzodiazepin-1-carboxylat.

Weitere bevorzugte, von der allgemeinen Formel I umfasste Verbindungen sind:
Cyclohexyl (S)-8-chlor-11,12,13,13a-tetrahydro-9-oxo-9H-imidazo[1,5-a]pyrrolo[2,1-c][1,4]benzodiazepin-1-carboxylat,
2-Propinyl (S)-8-chlor-11,12,13,13a-tetrahydro-9-oxo-9H-imidazo[1,5-a]pyrrolo[2,1-c][1,4]benzodiazepin-1-carboxylat,
Cyclopropylmethyl 8-chlor-11,13a-dihydro-9-oxo-9H-imidazo[1,5-a]pyrrolo[2,1-c][1,4]benzodiazepin-1-carboxylat,
Cyclopropylmethyl (S)-8-chlor-11,12,13,13a-tetrahydro-9-oxo-9H-imidazo[1,5-a]pyrrolo[2,1-c][1,4]benzodiazepin-1-carboxylat,
Cyclopropylmethyl (S)-8-brom-11,12,13,13a-tetrahydro-9-oxo-9H-imidazo[1,5-a]pyrrolo[2,1-c][1,4]benzodiazepin-1-carboxylat,
Cyclopropylmethyl (S)-11,12,13,13a-tetrahydro-8-jod-9-oxo-9H-imidazo[1,5-a]pyrrolo[2,1-c][1,4]benzodiazepin-1-carboxylat,
2-Cyclopropyläthyl (S)-8-chlor-11,12,13,13a-tetrahydro-9-oxo-9H-imidazo[1,5-a]pyrrolo[2,1-c][1,4]benzodiazepin-1-carboxylat,
Cyclohexyl (S)-12,12a-dihydro-8-methyl-9-oxo-9H,11H-azeto[2,1-c]imidazo[1,5-a][1,4]benzodiazepin-1-carboxylat,
rac-cis-3-Methylcyclohexyl (S)-8-chlor-11,12,13,13a-tetrahydro-9-oxo-9H-imidazo[1,5-a]pyrrolo[2,1-c][1,4]benzodiazepin-1-carboxylat,
Cyclopentyl (S)-12,12a-dihydro-8-jod-9-oxo-9H,11H-azeto[2,1-c]imidazo[1,5-a][1,4]benzodiazepin-1-carboxylat,
Cycloheptyl (S)-8-chlor-11,12,13,13a-tetrahydro-9-oxo-9H-imidazo[1,5-a]pyrrolo[2,1-c][1,4]benzodiazepin-1-carboxylat,
Benzyl (S)-8-chlor-12,12a-dihydro-9-oxo-9H,11H-azeto[2,1-c]imidazo[1,5-a][1,4]benzodiazepin-1-carboxylat,
Cyclopropylmethyl (S)-12,12a-dihydro-8-jod-9-oxo-9H,11H-azeto[2,1-c]imidazo[1,5-a][1,4]benzodiazepin-1-carboxylat,
Cyclohexyl (S)-8-brom-12,12a-dihydro-9-oxo-9H,11H-azeto[2,1-c]imidazo[1,5-a][1,4]benzodiazepin-1-carboxylat,
Cyclopropylmethyl (S)-8-brom-12,12a-dihydro-9-oxo-9H,11H-azeto[2,1-c]imidazo[1,5-a][1,4]benzodiazepin-1-carboxylat,
Cyclohexyl (S)-11,12,13,13a-tetrahydro-8-nitro-9-oxo-9H-imidazo[1,5-a]pyrrolo[2,1-c][1,4]benzodiazepin-1-carboxylat und
Cyclohexyl (S)-8-cyano,11,12,13,13a-tetrahydro-9-oxo-9H-imidazo[1,5-a]pyrrolo[2,1-c][1,4]benzodiazepin-1-carboxylat.

Weitere repräsentative Vertreter der erfindungsgemässen Stoffklasse sind:
Cyclopropylmethyl (S)-11,12,13,13a-tetrahydro-9-oxo-8-(trifluormethyl)-9H-imidazo[1,5-a]pyrrolo[2,1-c][1,4]benzodiazepin-1-carboxylat,
Cyclohexyl (S)-12,12a-dihydro-9-oxo-8-(trifluormethyl)-9H,11H-azeto[2,1-c]imidazo[1,5-a][1,4]benzodiazepin-1-carboxylat,
Cyclopropylmethyl (S)-12,12a-dihydro-9-oxo-8-(trifluormethyl)-9H,11H-azeto[2,1-c]imidazo[1,5-a][1,4]benzodiazepin-1-carboxylat,
Cyclopropylmethyl (S)-8-chlor-7-fluor-11,12,13,13a-tetrahydro-9-oxo-9H-imidazo[1,5-a]pyrrolo[2,1-c][1,4]benzodiazepin-1-carboxylat,
Cyclohexyl (S)-8-chlor-7-fluor-12,12a-dihydro-9-oxo-9H,11H-azeto[2,1-c]imidazo[1,5-a][1,4]benzodiazepin-1-carboxylat,
Cyclopropylmethyl (S)-8-chlor-7-fluor-12,12a-dihydro-9-oxo-9H,11H-azeto[2,1-c]imidazo[1,5-a][1,4]benzodiazepin-1-carboxylat,
Cyclohexyl (S)-8-fluor-11,12,13,13a-tetrahydro-9-oxo-9H-imidazo[1,5-a]pyrrolo[2,1-c][1,4]benzodiazepin-1-carboxylat,
Cyclopropylmethyl (S)-8-fluor-11,12,13,13a-tetrahydro-9-oxo-9H-imidazo[1,5-a]pyrrolo[2,1-c][1,4]benzodiazepin-1-carboxylat,
Cyclohexyl (S)-8-fluor-12,12a-dihydro-9-oxo-9H,11H-azeto[2,1-c]imidazo[1,5-a][1,4]benzodiazepin-1-carboxylat und
Cyclopropylmethyl (S)-8-fluor-12,12a-dihydro-9-oxo-9H,11H-azeto[2,1-c]imidazo[1,5-a][1,4]benzodiazepin-1-carboxylat.

Die Imidazobenzodiazepine der obigen Formel I und ihre pharmazeutisch annehmbaren Säureadditionssalze können erfindungsgemäss hergestellt werden, indem man

a) einen Carbonsäureester der allgemeinen Formel

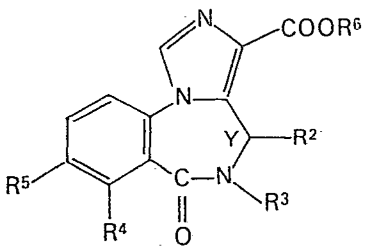

II

worin $R^6$ niederes Alkyl oder die Gruppe $-(A)_n-R^1$ bedeutet und A, n, $R^1$, $R^2$, $R^3$, $R^4$ und $R^5$ obige Bedeutung besitzen, mit einem den gewünschten Rest $-(A)_n-R^1$ liefernden Alkohol der allgemeinen Formel

$HO-(A)_n-R^1$ III

worin A, n und $R^1$ obige Bedeutung besitzen, umestert, oder

b) eine Carbonsäure der allgemeinen Formel

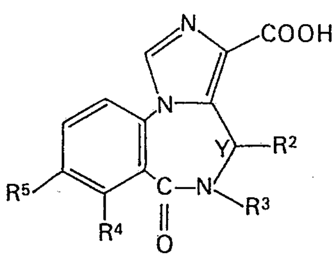

IV

worin $R^2$, $R^3$, $R^4$ und $R^5$ obige Bedeutung besitzen, mit einem den Rest $-(A)_n-R^1$ liefernden Mittel verestert, oder

c) eine Verbindung der allgemeinen Formel

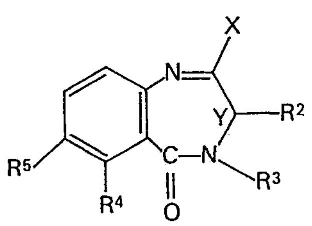

V

worin X eine Abgangsgruppe bedeutet und $R^2$, $R^3$, $R^4$ und $R^5$ obige Bedeutung besitzen, in Gegenwart einer Base mit einem Isocyanessigester der allgemeinen Formel

$CN-CH_2-COO-(A)_n-R^1$ VI

worin A, n und $R^1$ obige Bedeutung besitzen, umsetzt, oder

d) in einer Verbindung der Formel I, worin einer der Reste $R^4$ und $R^5$ Halogen und der andere Wasserstoff, Trifluormethyl, Amino, Nitro, Cyano oder niederes Alkyl bedeutet und A, n, $R^1$, $R^2$ und $R^3$ obige Bedeutung besitzen, das Halogenatom durch die Cyanogruppe ersetzt, oder

e) in einer Verbindung der Formel I, worin einer der Reste $R^4$ und $R^5$ Amino und der andere Wasserstoff, Halogen, Trifluormethyl, Nitro, Cyano oder niederes Alkyl bedeutet und A, n, $R^1$, $R^2$ und $R^3$ obige Bedeutung besitzen, die Aminogruppe durch ein Wasserstoff- oder Halogenatom oder durch eine Cyano- oder Nitrogruppe ersetzt, oder

f) eine Verbindung der Formel I, worin einer der Reste $R^4$ und $R^5$ Amino und der andere Wasserstoff bedeutet und A, n, $R^1$, $R^2$ und $R^3$ obige Bedeutung besitzen, in α-Stellung zur Aminogruppe halogeniert, oder

g) in einer Verbindung der Formel I, worin einer der Reste $R^4$ und $R^5$ Nitro und der andere Wasserstoff bedeutet und A, n, $R^1$, $R^2$ und $R^3$ obige Bedeutung besitzen, die Nitrogruppe zur Aminogruppe reduziert, und

h) erwünschtenfalls eine erhaltene Verbindung der Formel I in ein pharmazeutisch annehmbares Säureadditionssalz überführt.

Gemäss Verfahrensvariante a) können die Verbindungen der Formel I durch Umesterung von Verbindungen der Formel II mit einem Alkohol der Formel III hergestellt werden, d.h. dass in Verbindungen der Formel II der Rest $R^6$ gegen die gewünschte Gruppe $-(A)_n-R^1$ ausgetauscht wird.

Diese Umesterung erfolgt in an sich bekannter Weise durch Umsetzen einer Verbindung der Formel II mit einem Alkohol der Formel III in einem Temperaturbereich von etwa Raumtemperatur bis 150°C. Die Umesterung kann beispielsweise in Gegenwart einer Base durchgeführt werden, wobei sich im vorliegenden Fall insbesondere Kaliumcyanid oder ähnliche schwache Basen eignen. Als Base eignet sich aber auch das Natrium- oder Kaliumsalz eines Alkohols der Formel III. Die gewünschte Reaktion kann jedoch auch in Gegenwart eines Tetraalkyl-orthotitanats, wie Tetraäthylorthotitanat, durchgeführt werden, wobei in Gegenwart solcher Reagenzien auch tert.-Butylester umgeestert werden können. Als Lösungsmittel dient vorzugsweise der Alkohol der Formel III.

Gemäss Verfahrensvariante b) können die Verbindungen der Formel I hergestellt werden, indem man eine Carbonsäure der Formel IV mit einem den Rest $-(A)_n-R^1$ liefernden Mittel verestert. Diese Veresterung kann nach an sich bekannten und jedem Fachmann geläufigen Methoden durchgeführt werden. Man kann beispielsweise eine Carbonsäure der Formel IV mit einem geeigneten Reagens, z.B. mit Thionylchlorid, Phosphoroxychlorid, Oxalylchlorid oder dergleichen, in das entsprechende Carbonsäurechlorid überführen, und dieses in Gegenwart eines säurebindenden Mittels mit einem Alkohol der Formel III umsetzen. Als säurebindende Mittel eignen sich in erster Linie tertiäre Amine, wie Triäthylamin, Pyridin, Chinuclidin oder dergleichen. Unter Umständen kann die Anwesenheit einer katalytischen Menge an 4-Dimethylaminopyridin oder eines ähnlich reaktiven Amins vorteilhaft sein. Diese Veresterung kann in zwei getrennten Stufen, d.h. Bildung des reaktiven Carbonsäurederivates und Umsetzen desselben mit dem Alkohol der Formel III, oder in einem sogenannten Eintopfverfahren, was bevorzugt wird, durchgeführt werden. Als Lösungsmittel eignen sich z.B.

halogenierte Kohlenwasserstoffe, wie Methylenchlorid, Chloroform, 1,2-Dichloräthan und dergleichen, Äther, wie Diäthyläther, tert.-Butylmethyläther, Tetrahydrofuran und dergleichen, aprotisch polare Lösungsmittel, wie Acetonitril, Dimethylformamid etc. Die Reaktionstemperatur liegt zweckmässigerweise in einem Bereich von etwa -10°C bis Siedetemperatur des Reaktionsgemisches.

Es ist aber auch möglich, ein wie oben erhaltenes Carbonsäurechlorid oder ein Carbonsäureimidazolid, das durch Reaktion der freien Carbonsäure der Formel IV mit N,N'-Carbonyldiimidazol leicht zugänglich ist, mit einem Natrium- oder Kaliumsalz eines Alkohols der Formel III umzusetzen. Als Lösungsmittel eignen sich insbesondere Äther, wie Tetrahydrofuran und Dioxan, aprotisch polare Lösungsmittel, wie Dimethylformamid, und dergleichen. Man arbeitet dabei, je nach verwendetem Lösungsmittel, in einem Bereich von etwa 0° bis etwa 100°C, vorzugsweise jedoch bei Raumtemperatur.

Gemäss Verfahrensvariante c) können die Verbindungen der Formel I aus Verbindungen der Formel V und Isocyanessigestern der Formel VI hergestellt werden. Die in Formel V mit X bezeichnete Abgangsgruppe ist beispielsweise eine leicht abspaltbare Phosphinylgruppe, z.B. eine Gruppe der Formel

$$-\overset{\overset{O}{\|}}{O P}(OR^7)_2 \quad \text{oder} \quad -\overset{\overset{O}{\|}}{O P}(NR^8R^9)_2$$

worin $R^7$ niederes Alkyl und $R^8$ und $R^9$ je niederes Alkyl, Allyl, Phenyl oder substituiertes Phenyl oder zusammen mit dem Stickstoffatom einen unsubstituierten oder substituierten heterocyclischen Ring mit 3-8 Gliedern (wie Morpholin) bedeuten, ein Halogenatom, eine Alkylthiogruppe, eine Aralkylthiogruppe, eine N-Nitrosoalkylaminogruppe, eine Alkoxygruppe, eine Mercaptogruppe und dergleichen (wenn X eine Mercaptogruppe bedeutet, so handelt es sich bei der entsprechenden Verbindung der Formel V um die Iminothiol-Form des entsprechenden Thiolactams). Die Umsetzung der Verbindungen der Formeln V und VI erfolgt in einem inerten Lösungsmittel, wie Dimethylformamid, Hexamethylphosphorsäuretriamid, Dimethylsulfoxid, Tetrahydrofuran oder irgend einem anderen geeigneten organischen Lösungsmittel und in Gegenwart einer Base, die genügend stark basisch ist, um aus dem Isocyanessigester der Formel VI das Anion zu bilden. Als Basen eignen sich Alkalimetallalkoxide, wie Natriummethoxid oder Kalium-tert.-butoxid, Alkalimetallhydride, wie Natriumhydrid, Alkalimetallamide, wie Lithiumamid oder Lithiumdiisopropylamid, tertiäre Amine, wie Triäthylamin, und dergleichen. Die Reaktionstemperatur liegt zweckmässigerweise zwischen etwa -40°C und etwa Raumtemperatur.

Gemäss Verfahrensvariante d) können Verbindungen der Formel I, worin einer der Reste $R^4$ und $R^5$ Cyano und der andere Wasserstoff, Trifluormethyl, Amino, Nitro, Cyano oder niederes Alkyl bedeutet, hergestellt werden, indem man in einer Verbindung der Formel I, worin einer der Reste $R^4$ und $R^5$ Halogen bedeutet und der andere die vorstehend erwähnte Bedeutung besitzt, das Halogenatom durch die Cyanogruppe ersetzt. Dabei setzt man vorzugsweise eine entsprechende Brom- oder Jodverbindung ein. Die Reaktion kann beispielsweise so durchgeführt werden, dass man die entsprechende Brom- oder Jodverbindung in einem inerten organischen Lösungsmittel mit Kupfer(I)cyanid umsetzt. Geeignete Lösungsmittel sind beispielsweise Dimethylformamid und dergleichen. Die Reaktionstemperatur liegt zweckmässigerweise in einem Bereich von etwa Raumtemperatur bis Siedetemperatur der Reaktionsmischung.

Gemäss Verfahrensvariante e) kann man in einer Verbindung der Formel I, worin einer der Reste $R^4$ und $R^5$ Amino und der andere Wasserstoff, Halogen, Trifluormethyl, Nitro, Cyano oder niederes Alkyl bedeutet, die Aminogruppe durch ein Wasserstoff- oder Halogenatom oder eine Cyano- oder Nitrogruppe ersetzen. Für den Ersatz durch ein Halogenatom oder eine Cyano- oder Nitrogruppe kann man so vorgehen, dass man die Aminoverbindung der Formel I in ein entsprechendes Diazoniumsalz überführt und dieses, gegebenenfalls ohne vorgängige Isolierung, mit einem Nitrit, wie Natriumnitrit, oder mit einem Halogenid, z.B. mit einem Chlorid oder Bromid, oder einem Cyanid in Gegenwart eines Kupfer(I)salzes umsetzt. Für die Herstellung der entsprechenden Jodide ist die Anwesenheit eines Kupfer(I)salzes nicht erforderlich. Entsprechende Fluoride werden zweckmässigerweise über die entsprechenden Diazonium-tetrafluoroborate hergestellt, beispielsweise durch Bestrahlen mit UV-Licht. Diese Reaktionen werden in wässrigen Lösungen bei Temperaturen von etwa -10°C bis etwa Raumtemperatur durchgeführt.

Der Ersatz einer Aminogruppe in einer Verbindung der Formel I durch die Nitrogruppe kann aber auch dadurch bewerkstelligt werden, dass man eine Aminoverbindung der Formel I oxidiert. Als Oxidationsmittel eignen sich beispielsweise Persäuren, wie Peressigsäure, Trifluorperessigsäure, m-Chlorperbenzoesäure und Perbenzoesäure, und dergleichen. Als Lösungsmittel kommen, je nach eingesetztem Oxidationsmittel, Carbonsäuren, wie Essigsäure etc., halogenierte Kohlenwasserstoffe, wie Methylenchlorid, Chloroform, 1,2-Dichloräthan etc., oder dergleichen in Frage. In der Regel arbeitet man bei Temperaturen von etwa 0°C bis etwa Raumtemperatur.

Der Ersatz der Aminogruppe durch ein Wasserstoffatom kann beispielsweise durch Reduktion eines entsprechenden Diazoniumsalzes bewerkstelligt werden, beispielsweise durch Erhitzen in einem cyclischen Äther, wie Tetrahydrofuran oder Dioxan, oder in Dimethylformamid; man arbeitet dabei vorzugsweise bei der Siedetemperatur der Reaktionsmischung. In einer besonders bevorzugten Ausführungsform setzt man jedoch ein Amin der Formel I in einem cyclischen Äther,

wie Tetrahydrofuran oder Dioxan, mit t-Butylnitrit um, wobei man dabei vorzugsweise bei der Siedetemperatur des Reaktionsgemisches arbeitet.

Gemäss Verfahrensvariante f) kann man eine Verbindung der Formel I, worin einer der Reste $R^4$ und $R^5$ Amino und der andere Wasserstoff bedeutet, in α-Stellung zur Aminogruppe halogenieren. Geeignete Halogenierungsmittel sind beispielsweise N-Chlorsuccinimid, N-Bromsuccinimid, N-Chloracetamid, N-Bromacetamid und dergleichen. Als Lösungsmittel verwendet man zweckmässigerweise inerte organische Lösungsmittel, beispielsweise halogenierte Kohlenwasserstoffe, wie Methylenchlorid, 1,2-Dichloräthan, Chloroform und dergleichen, Dimethylformamid, Dimethylacetamid, Acetonitril, Äther, wie Diäthyläther, Tetrahydrofuran, Dioxan und dergleichen, usw. Die Halogenierung kann in einem Temperaturbereich von etwa 0°C bis Siedetemperatur der Reaktionsmischung durchgeführt werden, wobei ein Bereich von etwa Raumtemperatur bis etwa 100°C bevorzugt wird.

Gemäss Verfahrensvariante g) können Verbindungen der Formel I, worin einer der Reste $R^4$ und $R^5$ Amino und der andere Wasserstoff bedeutet, durch Reduktion einer entsprechenden Nitroverbindung hergestellt werden. Zweckmässigerweise verwendet man Reduktionsmittel, wie Zinn(II)chlorid, Zinn, Zink und dergleichen, in saurem, wässrigem Milieu, beispielsweise in wässriger Salzsäure, konzentrierter Salzsäure oder dergleichen, in einem Temperaturbereich von etwa 0°C bis etwa Raumtemperatur.

Gemäss Verfahrensvariante h) können Verbindungen der Formel I erfindungsgemäss in pharmazeutisch annehmbare Säureadditionssalze übergeführt werden. Die Herstellung von solchen pharmazeutisch annehmbaren Säureadditionssalzen erfolgt nach allgemein üblichen Methoden. Es kommen sowohl Salze mit anorganischen als auch Salze mit organischen Säuren in Betracht, beispielsweise Hydrochloride, Hydrobromide, Sulfate, Methansulfonate, p-Toluolsulfonate, Oxalate und dergleichen.

Die als Ausgangsstoffe verwendeten Verbindungen der Formel II, worin $R^6$ niederes Alkyl bedeutet, sind bekannt oder können in Analogie zu den bekannten Vertretern dieser Stoffklasse hergestellt werden. Sie können beispielsweise in Analogie zur weiter oben beschriebenen Verfahrensvariante c) durch Reaktion einer Verbindung der Formel V mit einem Isocyanessigester der allgemeinen Formel

$CN-CH_2-COOR^{61}$ VII

worin $R^{61}$ niederes Alkyl bedeutet, in Gegenwart einer Base leicht hergestellt werden.

Die als Ausgangsstoffe verwendeten Verbindungen der Formel IV sind durch Hydrolyse der Estergruppe in Verbindungen der Formel II, worin $R^6$ niederes Alkyl bedeutet, nach an sich bekannten und jedem Fachmann geläufigen Methoden leicht zugänglich. Auch diese Verbindungen gehören einer an sich bekannten Stoffklasse an.

Die Ausgangsstoffe der Formel V gehören ebenfalls einer an sich bekannten Stoffklasse an und können ausgehend von Verbindungen der allgemeinen Formel

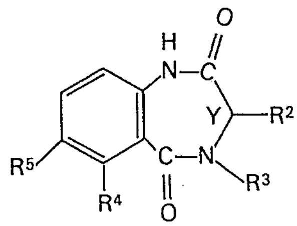

VIII

worin $R^2$, $R^3$, $R^4$ und $R^5$ obige Bedeutung besitzen, hergestellt werden, und zwar nach an sich bekannten Methoden, vgl. z.B. die Belgischen Patentschriften No. 802 233, 833 249 und 865 653, die U.S.-Patentschrift No. 3 681 341 und J. Org. Chemistry 29, 231 (1964).

Verschiedene der weiter unten folgenden Beispiele enthalten detaillierte Angaben betreffend die Herstellung von Verbindungen der Formeln II, IV und V.

Die Verbindungen der Formel VIII ihrerseits sind ebenfalls bekannt oder nach an sich bekannten Methoden leicht herstellbar. Sie können beispielsweise durch Umsetzen eines entsprechenden Carbonsäureanhydrids der allgemeinen Formel

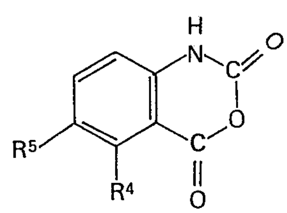

IX

worin $R^4$ und $R^5$ obige Bedeutung besitzen, mit einer Aminosäure der allgemeinen Formel

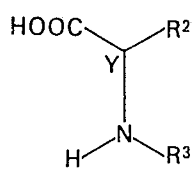

X

worin $R^2$ und $R^3$ obige Bedeutung besitzen, hergestellt werden.

Verbindungen der Formel VIII, worin einer der Reste $R^4$ und $R^5$ Halogen und der andere Wasserstoff, Trifluormethyl, Amino, Nitro, Cyano oder niederes Alkyl bedeutet, können durch Behandeln mit Kupfer(I)cyanid in entsprechende Cyanoverbindungen übergeführt werden. Eine weitere Möglichkeit zur Abwandlung von Verbindungen der Formel VIII besteht darin, dass man eine solche Verbindung, worin einer der Reste $R^4$ und $R^5$ Amino und der andere Wasserstoff bedeutet, in α-Stellung zur Aminogruppe halogeniert. Weiterhin kann man in einer Verbindung der Formel VIII, worin einer der Reste $R^4$ und $R^5$ Amino und der andere Wasserstoff, Halogen, Trifluormethyl,

Nitro, Cyano oder niederes Alkyl bedeutet, die Aminogruppe beispielsweise durch Reduktion eines entsprechenden Diazoniumsalzes abspalten, oder die Aminogruppe über ein entsprechendes Diazoniumsalz durch ein Halogenatom oder die Cyano- oder Nitrogruppe ersetzen, oder die Aminogruppe zur Nitrogruppe oxidieren. Schliesslich kann man auch eine Verbindung der Formel VIII, worin $R^4$ und $R^5$ Wasserstoff bedeuten, nitrieren, wobei man eine entsprechende Verbindung der Formel VIII erhält, worin $R^5$ Nitro und $R^4$ Wasserstoff bedeuten, oder eine entsprechende Verbindung, worin einer der Reste $R^4$ und $R^5$ Nitro bedeutet, zur entsprechenden Aminoverbindung reduzieren.

Wie eingangs erwähnt sind die Verbindungen der Formel I neu; sie besitzen äusserst wertvolle pharmakodynamische Eigenschaften und weisen nur eine geringe Toxizität auf. Sie besitzen als gemeinsames Merkmal eine ausgeprägte Affinität zu den zentralen Benzodiazepin-Rezeptoren und haben entweder ausgeprägte anxiolytische, antikonvulsive, muskelrelaxierende und sedativ-hypnotische Eigenschaften, und/oder sie antagonisieren selektiv einige oder alle Wirkungen, welche tranquilisierend wirksame 1,4-Benzodiazepine oder andere Substanzen über die zentralen Benzodiazepin-Rezeptoren entfalten, partiell oder vollständig.

Die Affinität von Verbindungen der allgemeinen Formel I zu den zentralen Benzodiazepin-Rezeptoren wurde nach der in Life Science 20, 2101–2110 (1977) und Science 198, 849–851 (1977) beschriebenen Methode festgestellt. Nach dieser Methode ermittelt man die Hemmung der Bindung von tritiiertem Diazepam an die spezifischen Benzodiazepin-Rezeptoren im cerebralen Cortex durch die jeweiligen Testsubstanzen. Man bezeichnet als $IC_{50}$ («50% inhibiting concentration») diejenige Konzentration der jeweiligen Testsubstanz, welche eine 50proz. Hemmung der spezifischen Bindung des tritiierten Diazepams an die spezifischen Benzodiazepin-Rezeptoren im cerebralen Cortex bewirkt.

Die zentralen Eigenschaften der erfindungsgemässen Verbindungen der Formel I können beispielsweise im nachfolgend beschriebenen und für die Erfassung antikonvulsiver Eigenschaften allgemein anerkannten Antipentetrazoltest nachgewiesen werden.

In diesem Tierversuch verabreicht man 60–80 g schweren, weiblichen Ratten die zu testende Verbindung oral und 30 Minuten später 120 mg/kg i.p. Pentetrazol, das in ungeschützten Versuchstieren 1 bis 4 Minuten nach der Injektion Emprosthotonus und tonische Streckungen der vorderen und/oder hinteren Gliedmassen bewirkt. Man verwendet zehn Versuchstiere pro Dosis Testsubstanz. Nach Auszählen der geschützten Versuchstiere ermittelt man die $ED_{50}$ nach der Probit-Methode. Die $ED_{50}$ ist diejenige Dosis, welche 50% der Versuchstiere vor den durch Pentetrazol bewirkten krampfartigen Anfällen schützt.

Die antagonistischen Eigenschaften der Verbindungen der Formel I, d.h. ihr Vermögen, Wirkungen, welche tranquilisierend wirksame 1,4-Benzodiazepine oder andere Substanzen über die zentralen Benzodiazepin-Rezeptoren entfalten, zu antagonisieren, können beispielsweise im nachfolgend beschriebenen, bekannten Traktions-Test nachgewiesen werden. In diesem Tierversuch wird das Vermögen der Testsubstanzen ermittelt, die durch höhere Dosen von Diazepam hervorgerufene sedative, muskelrelaxierende und die motorische Koordination störende Wirkung zu antagonisieren.

Man hebt 17–29 g schwere Mäuse beim Schwanz und lässt sie mit den Vorderpfoten einen horizontal gespannten Draht (Länge 15 cm, Höhe 20 cm, ∅ 1 mm) ergreifen, worauf man die Versuchstiere loslässt. Unbehandelte Kontrolltiere sind imstande, innerhalb von 3 Sekunden mit beiden Vorderpfoten und mindestens einer Hinterpfote den Draht zu ergreifen. Verabreicht man den Versuchstieren 3 mg/kg i.p. Diazepam, so verlieren sie ihre oben erwähnten Fähigkeiten, d.h. sie sind nicht mehr imstand, innerhalb von 3 Sekunden mit beiden Vorderpfoten und mindestens einer Hinterpfote den horizontal gespannten Draht zu ergreifen. 15 Minuten nach der Verabreichung von Diazepam erhalten die Versuchstiere die zu testende Verbindung oral, worauf man diejenigen Tiere auszählt, welche die oben erwähnten Fähigkeiten zurückerlangt haben. Als $ED_{50}$ bezeichnet man diejenige Dosis, welche in 50% der Versuchstiere die Wirkung des Diazepams antagonisiert, d.h. diese aufhebt.

In der nachfolgenden Tabelle werden die Resultate dargestellt, welche mit repräsentativen Vertretern der durch die allgemeine Formel I definierten Verbindungsklasse in den vorstehend geschilderten Versuchen erhalten worden sind. Ausserdem enthält die Tabelle Angaben über die akute Toxizität einiger dieser Verbindungen ($DL_{50}$ in mg/kg bei einmaliger oraler Verabreichung an Ratten).

| Verbindung der Formel I, worin | | | | | Konfiguration | $IC_{50}$ in $\mu M/l$ | Antipentetrazol-Test, $ED_{50}$ in mg/kg p.o. | Traktions-Test, $ED_{50}$ in mg/kg p.o. | Toxizität, $DL_{50}$ in mg/kg p.o. |
|---|---|---|---|---|---|---|---|---|---|
| $-(A)_n-R^1$ | $R^2$ | $R^3$ | $R^4$ | $R^5$ | | | | | |
| $-CH_2-$ 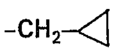 | | $-CH_2-CH_2-CH_2-$ | Cl | H | (S) | 2,3 | 0,66 | 0,8 | 1000–2000 |
| $-CH_2-$ 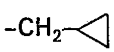 | | $-CH_2-CH_2-$ | Cl | H | (S) | 1,1 | 0,27 | $<30$ | – |
| 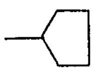 | | $-CH_2-CH_2-CH_2-$ | Cl | H | (S) | 2,2 | 2,9 | 0,5 | – |
| $-CH_2-$ 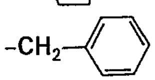 | | $-CH_2-CH_2-CH_2-$ | Cl | H | (S) | 1,5 | $>100$ | 0,09 | – |
| $-CH_2-C\equiv CH$ | | $-CH_2-CH_2-CH_2-$ | Cl | H | (S) | 2,1 | $>100$ | 0,005 | – |
| 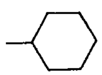 | | $-CH_2-CH_2-CH_2-$ | Br | H | (S) | 2,7 | 7,0 | $<30$ | $>5000$ |
| $-CH_2-$ 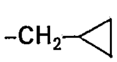 | | $-CH_2-CH_2-CH_2-$ | Br | H | (S) | 2,6 | 0,63 | $<30$ | 625–1250 |
| 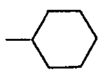 | | $-CH_2-CH_2-CH_2-$ | J | H | (S) | 2,1 | 2,2 | $>30$ | $>5000$ |

Eine selektive antagonistische Komponente, wie sie bei vielen der Verbindungen der Formel I nachgewiesen werden kann, ist von grosser therapeutischer Bedeutung, indem sie die Nutzung erwünschter Eigenschaften (z.B. anxiolytische oder antikonvulsive Wirkung) der erfindungsgemässen Stoffe erlaubt, unter Zurückdrängung der im bestimmten Anwendungsfall unerwünschten, zusätzlichen Eigenschaften (z.B. sedierende, muskelrelaxierende und die motorische Koordination störende Wirkungen).

In EP-A-27 214 sind u.a. entsprechende Methyl-, Äthyl-, Isopropyl- und 2-Hydroxyäthylester beschrieben, welche die zentral dämpfenden, muskelrelaxierenden, ataktischen, blutdrucksenkenden und atemdepressiven Eigenschaften von tranquilisierend wirksamen 1,4-Benzodiazepinen zu antagonisieren vermögen.

Die Verbindungen der Formel I und ihre pharmazeutisch annehmbaren Säureadditionssalze können als Heilmittel, z.B. in Form pharmazeutischer Präparate, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, oder parenteral, z.B. in Form von Injektionslösungen, erfolgen.

Zur Herstellung von pharmazeutischen Präparaten können die erfindungsgemässen Produkte mit pharmazeutisch inerten, anorganischen oder organischen Trägern verarbeitet werden. Als solche Träger kann man für Tabletten, Lacktabletten, Dragées und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Öle, Wachse, Fette, halbfeste und flüssige Polyole und dergleichen; je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Saccharose, Invertzucker, Glukose und dergleichen. Für Injektionslösungen eignen sich als Träger beispielsweise Wasser, Alkohole, Polyole, Glyzerin, pflanzliche Öle und dergleichen. Für Suppositorien eignen sich als Träger beispielsweise natürliche oder gehärtete Öle, Wachse, Fette, halbflüssige oder flüssige Polyole und dergleichen.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze, zur Veränderung des osmotischen Druckes, Puffer, Überzugsmittel oder Antioxidantien enthalten. Sie können auch noch andere therapeutisch wertvolle Stoffe enthalten.

Wie eingangs erwähnt, sind Arzneimittel, enthaltend eine Verbindung der Formel I oder ein pharmazeutisch annehmbares Säureadditionssalz davon, ebenfalls Gegenstand der vorliegenden Erfindung, weiterhin auch ein Verfahren zur Herstellung solcher Arzneimittel, welches dadurch gekennzeichnet ist, dass man eine oder mehrere Verbindungen der Formel I oder pharmazeutisch annehmbare Säureadditionssalze davon und gegebenenfalls einen oder mehrere andere therapeutisch wertvolle Stoffe in eine galenische Darreichungsform bringt.

Wie eingangs erwähnt, können die Verbindungen der Formel I und ihre pharmazeutisch annehmbaren Säureadditionssalze bei der Bekämpfung bzw. Verhütung von Krankheiten verwendet werden, und zwar insbesondere bei der Bekämpfung von Konvulsionen und Angstzuständen und/oder bei der partiellen oder vollständigen Antagonisierung einiger oder aller Wirkungen, welche tranquilisierend wirksame 1,4-Benzodiazepine oder andere Substanzen über die zentralen Benzodiazepin-Rezeptoren entfalten. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 0,1 mg bis 100 mg angemessen sein.

Die nachfolgenden Beispiele sollen die vorliegende Erfindung näher erläutern, ihren Umfang jedoch in keiner Weise beschränken. Sämtliche Temperaturen sind in Celsiusgraden angegeben.

Beispiel 1

Man rührt eine Mischung aus 10,37 g (0,03 Mol) Äthyl (S)-8-chlor-11,12,13,13a-tetrahydro-9-oxo-9H-imidazo[1,5-a]pyrrolo[2,1-c][1,4]benzodiazepin-1-carboxylat, 35 ml Hydroxymethyl-cyclopropan und 0,5 g Kaliumcyanid während 12 Stunden bei 125°, wobei man während dieser Zeit durch Anlegen eines schwachen Vakuums dreimal je 2–3 ml des Lösungsmittels abdestilliert. Anschliessend dampft man im Vakuum ein, verteilt den Rückstand zwischen Chloroform und Wasser, wäscht die Chloroformlösung nacheinander mit Wasser und gesättigter Kochsalzlösung, trocknet sie über Magnesiumsulfat und dampft ein. Der erhaltene Rückstand wird aus Essigester umkristallisiert, wobei man Cyclopropylmethyl (S)-8-chlor-11,12,13,13a-tetrahydro-9-oxo-9H-imidazo[1,5-a]pyrrolo[2,1-c][1,4]benzodiazepin-1-carboxylat vom Schmelzpunkt 195–196° erhält.

Beispiel 2

a) 11,3 g (0,057 Mol) 6-Chlorisatosäureanhydrid und 5,78 g (0,057 Mol) L-Azetidincarbonsäure werden in 50 ml Dimethylsulfoxid während 2 Stunden auf 125° erhitzt. Anschliessend dampft man im Hochvakuum zur Trockene ein und erhitzt den erhaltenen Rückstand während 2 Stunden auf 150°. Durch Chromatographieren an Kieselgel unter Eluieren mit Essigester erhält man (S)-5-Chlor-1,10a-dihydroazeto[2,1-c][1,4]benzodiazepin-4,10(2H,9H)-dion vom Schmelzpunkt 225–228°.

b) Eine Suspension von 1,30 g (29,8 mMol) Natriumhydrid (55proz. Öldispersion) in 40 ml trockenem Dimethylformamid wird bei −15° mit

6,38 g (27 mMol) (S)-5-Chlor-1,10a-dihydroazeto[2,1-c][1,4]benzodiazepin-4,10(2H,9H)-dion versetzt und während 0,5 Stunden bei dieser Temperatur gerührt. Anschliessend kühlt man auf −35° ab und versetzt tropfenweise mit 4,8 ml (29,8 mMol) Diäthylchlorphosphat.

Inzwischen wird eine Lösung von 3,55 g (32,4 mMol) Kalium-tert.-butylat in 14 ml trockenem Dimethylformamid in einem Aceton/Trockeneisbad abgekühlt, mit 4,1 ml (32,4 mMol) Isocyanessigsäure-äthylester versetzt und bei −10° dem obigen Reaktionsgemisch zugetropft. Man entfernt das Kühlbad, rührt noch während ca. 15 Minuten, neutralisiert mit Eisessig, giesst auf 100 ml Wasser und extrahiert dreimal mit Chloroform. Die vereinigten Chloroformauszüge werden dreimal mit Wasser gewaschen, über Magnesiumsulfat getrocknet und eingedampft. Das erhaltene Rohprodukt wird an Kieselgel chromatographiert und anschliessend aus Essigester umkristallisiert. Man erhält Äthyl (S)-8-chlor-12,12a-dihydro-9-oxo-9H,11H-azeto[2,1-c]imidazo[1,5-a][1,4]benzodiazepin-1-carboxylat vom Schmelzpunkt 184–185°.

c) Man rührt eine Mischung aus 8,0 g (24 mMol) Äthyl (S)-8-chlor-12,12a-dihydro-9-oxo-9H,11H-azeto[2,1-c]imidazo[1,5-a][1,4]benzodiazepin-1-carboxylat, 0,5 g (7,7 mMol) Kaliumcyanid und 60 ml Hydroxymethyl-cyclopropan 5 Stunden bei 130°, wobei man während dieser Zeit durch Anlegen eines schwachen Vakuums viermal jeweils etwas Lösungsmittel abdestilliert. Anschliessend dampft man im Vakuum ein, verteilt den Rückstand zwischen Wasser und Methylenchlorid und extrahiert die wässrige Phase zweimal mit Methylenchlorid. Die vereinigten organischen Auszüge werden dreimal mit gesättigter Natriumchloridlösung gewaschen, über Magnesiumsulfat getrocknet und eingedampft. Man kristallisiert das Rohprodukt aus Essigester und erhält Cyclopropylmethyl (S)-8-chlor-12,12a-dihydro-9-oxo-9H,11H-azeto[2,1-c]imidazo[1,5-a][1,4]benzodiazepin-1-carboxylat vom Zersetzungspunkt 183–185° (partiell racemisiert).

Durch Chromatographieren der Mutterlauge an Kieselgel unter Eluieren mit Essigester erhält man eine zweite Portion des gewünschten Stoffes von gleicher Reinheit.

Beispiel 3

a) Man erhitzt 9,2 g (0,038 Mol) 6-Brom-isatosäureanhydrid und 4,6 g (0,040 Mol) L-Prolin in 55 ml Dimethylsulfoxid während 1 Stunde auf 70°, entfernt das Lösungsmittel im Hochvakuum und erhitzt das erhaltene Öl während 15 Minuten auf 170°. Das Rohprodukt wird durch Chromatographieren an Kieselgel gereinigt, wobei man als Elutionsmittel ein Gemisch aus Chloroform und Methanol (20:1) verwendet. Man erhält (S)-6-Brom-1,2,3,11a-tetrahydro-5H-pyrrolo[2,1-c][1,4]benzodiazepin-5,11(10H)-dion, das nach Umkristallisieren aus Chloroform/Hexan bei 221–224° schmilzt.

b) Man versetzt eine Lösung von 9,94 g (33,7 mMol) (S)-6-Brom-1,2,3,11a-tetrahydro-5H-pyrrolo[2,1-c][1,4]benzodiazepin-5,11(10H)-dion in 30 ml trockenem Dimethylformamid bei −20 bis −10° unter Rühren mit 1,62 g (37 mMol) Natriumhydrid (55proz. Öldispersion), rührt noch während 1,25 Stunden in obigem Temperaturbereich und tropft dann bei −40° 5,5 ml (37 mMol) Diäthylchlorphosphat dazu.

Inzwischen löst man 4,15 g (37 mMol) Kalium-tert.-butylat in 10 ml trockenem Dimethylformamid, kühlt in einem Aceton/Trockeneisbad ab, versetzt mit 5,22 g (37 mMol) Isocyanessigsäure-tert.-butylester und gibt die erhaltene Lösung bei −15° tropfenweise zum obigen Reaktionsgemisch. Man lässt auf 10° erwärmen, neutralisiert mit 2,1 ml Eisessig, giesst auf 150 ml Wasser und extrahiert dreimal mit Methylenchlorid. Die Methylenchloridlösung wird zweimal mit Wasser gewaschen, über Magnesiumsulfat getrocknet und eingedampft. Das erhaltene Rohprodukt chromatographiert man unter Eluieren mit Essigester an Kieselgel. Durch Umkristallisieren aus Essigester erhält man tert.-Butyl (S)-8-brom-11,12,13,13a-tetrahydro-9-oxo-9H-imidazo[1,5-a]pyrrolo[2,1-c][1,4]benzodiazepin-1-carboxylat vom Schmelzpunkt 206–208°.

c) Man rührt eine Mischung aus 10 g (24 mMol) tert.-Butyl (S)-8-brom-11,12,13,13a-tetrahydro-9-oxo-9H-imidazo[1,5-a]pyrrolo[2,1-c][1,4]benzodiazepin-1-carboxylat, 30 g (300 mMol) Cyclohexanol und 1,80 g (8 mMol) Tetraäthyl-orthotitanat über Nacht bei 125°, dampft die erhaltene Lösung zur Trockne ein, nimmt den Rückstand in Chloroform auf und rührt eine halbe Stunde mit 40 ml einer gesättigten Kaliumfluoridlösung. Man filtriert die entstandene Emulsion durch Kieselgur, trennt die organische Phase ab, wäscht sie mit Wasser, trocknet über Magnesiumsulfat und dampft ein. Durch Umkristallisieren des Rückstandes aus Essigester erhält man Cyclohexyl (S)-8-brom-11,12,13,13a-tetrahydro-9-oxo-9H-imidazo[1,5-a]pyrrolo[2,1-c][1,4]benzodiazepin-1-carboxylat vom Schmelzpunkt 167–168°.

Beispiel 4

a) Man versetzt eine Suspension von 91 g (308 mMol) (S)-6-Brom-1,2,3,11a-tetrahydro-5H-pyrrolo[2,1-c][1,4]benzodiazepin-5,11(10H)-dion in 300 ml trockenem Dimethylformamid unter Rühren bei 0° mit 38 g (339 mMol) Kalium-tert.-butylat, rührt während 0,5 Stunden bei 10–20° und tropft anschliessend bei −20° 58 g (339 mMol) Diäthylchlorphosphat dazu.

Inzwischen löst man 38 g (339 mMol) Kalium-tert.-butylat in 100 ml trockenem Dimethylformamid, kühlt in einem Aceton/Trockeneisbad ab, versetzt mit 38,34 g (339 mMol) Isocyanessigsäureäthylester und tropft die so erhaltene Lösung bei −15° zum obigen Reaktionsgemisch. Man lässt auf Raumtemperatur erwärmen, giesst auf 1500 ml Wasser und rührt während 1 Stunde. Anschliessend extrahiert man dreimal mit Methylenchlorid, wäscht die organische Phase zweimal mit Wasser, trocknet sie über Magnesiumsulfat und destilliert das Lösungsmittel ab. Das Rohprodukt chromatographiert man unter Eluieren mit Essigester an Kieselgel und kristallisiert dann aus Essigester um. Man erhält Äthyl (S)-8-brom-11,12,13,13a-tetrahydro-9-oxo-9H-imidazo[1,5-a] pyrrolo[2,1-c][1,4]benzodiazepin-1-carboxylat vom Schmelzpunkt 191–192°.

b) Man rührt eine Mischung aus 9,50 g (24,3 mMol) Äthyl (S)-8-brom-11,12,13,13a-tetrahydro-9-oxo-9H-imidazo[1,5-a]pyrrolo[2,1-c][1,4]benzodiazepin-1-carboxylat, 60 ml (560 mMol) Cyclohexanol und 300 mg pulverisiertem Kaliumcyanid während 24 Stunden bei 130°, wobei man von Zeit zu Zeit ein wenig Cyclohexanol abdestilliert. Anschliessend dampft man die erhaltene Lösung zur Trockne ein, nimmt den Rückstand in Chloroform auf, wäscht mit Wasser und trocknet über Magnesiumsulfat. Nach Entfernen des Lösungsmittels wird der Rückstand unter Eluieren mit Essigester an einer Kieselgelsäule chromatographiert und anschliessend aus Essigester umkristallisiert. Man erhält Cyclohexyl (S)-8-brom-11,12,13,13a-tetrahydro-9-oxo-9H-imidazo[1,5-a]pyrrolo[2,1-c][1,4]benzodiazepin-1-carboxylat vom Schmelzpunkt 167–168°.

Beispiel 5

Man rührt eine Mischung aus 12 g (31 mMol) Äthyl (S)-8-brom-11,12,13,13a-tetrahydro-9-oxo-9H-imidazo[1,5-a]pyrrolo[2,1-c][1,4]benzodiazepin-1-carboxylat, 60 ml (750 mMol) Hydroxymethyl-cyclopropan und 500 mg pulverisiertem Kaliumcyanid während 1,5 Stunden bei 135°, wobei man gleichzeitig über einen Hickmann-Aufsatz und eine 15 cm-Vigreux-Kolonne den Alkohol abdestilliert. Man dampft das Reaktionsgemisch zur Trockne ein, nimmt den Rückstand in Chloroform auf, wäscht mit Wasser, trocknet über Magnesiumsulfat und dampft ein. Durch Umkristallisieren aus Essigester erhält man Cyclopropylmethyl (S)-8-brom-11,12,13,13a-tetrahydro-9-oxo-9H-imidazo[1,5-a]pyrrolo[2,1-c][1,4]benzodiazepin-1-carboxylat vom Schmelzpunkt 135–136°.

Beispiel 6

Man rührt eine Mischung aus 10 g (24 mMol) tert.-Butyl (S)-8-brom-11,12,13,13a-tetrahydro-9-oxo-9H-imidazo[1,5-a]pyrrolo[2,1-c][1,4]benzodiazepin-1-carboxylat, 31 g (430 mMol) Hydroxymethyl-cyclopropan und 1,80 g (8 mMol) Tetraäthyl-orthotitanat über Nacht bei 125°, dampft die erhaltene Lösung zur Trockne ein, nimmt den Rückstand in Chloroform auf und rührt eine halbe Stunde mit 40 ml einer gesättigten Kaliumfluoridlösung. Man filtriert die entstandene Emulsion durch Kieselgur, trennt die organische Phase ab, wäscht sie mit Wasser, trocknet über Magnesiumsulfat und dampft ein. Durch Umkristallisieren aus Essigester und Hexan erhält man Cyclopropylmethyl (S)-8-brom-11,12,13,13a-tetrahydro-9-oxo-9H-imidazo[1,5-a]pyrrolo[2,1-c][1,4]benzodiazepin-1-carboxylat vom Schmelzpunkt 135–136°.

Beispiel 7

a) Man rührt 70 g (242 mMol) 6-Jodisatosäureanhydrid und 24,46 g (242 mMol) L-Azetidin-2-carbonsäure in 140 ml Dimethylsulfoxid während 2 Stunden bei 105°, entfernt das Dimethylsulfoxid im Vakuum und erhitzt den Rückstand während 2,5 Stunden im Hochvakuum auf 140°. Durch Chromatographieren an Kieselgel unter Eluieren mit Essigester und anschliessendes Umkristallisieren aus Essigester erhält man (S)-1,10a-Dihydro-5-jod-azeto[2,1-c][1,4]benzodiazepin-4,10(2H, 9H)-dion vom Schmelzpunkt 186–187°.

b) Man versetzt 23,6 g (72 mMol) (S)-1,10a-Dihydro-5-jod-azeto[2,1-c][1,4]benzodiazepin-4,10 (2H,9H)-dion in 70 ml trockenem Dimethylformamid mit 2,04 g (85 mMol) Natriumhydrid (55proz. Öldispersion mit n-Hexan gewaschen), rührt während 1 Stunde bei −20°, versetzt dann bei dieser Temperatur tropfenweise mit 13,8 g Diäthylchlorphosphat und rührt noch 0,5 Stunden bei −20°.

Inzwischen löst man 9,53 g (85 mMol) Kalium-tert.-butylat in 20 ml trockenem Dimethylformamid, kühlt im Aceton/Trockeneisbad ab, versetzt mit 11,9 g (85 mMol) Isocyanessigsäure-tert.-butylester und tropft die so erhaltene Lösung bei −10 bis −20° zum obigen Reaktionsgemisch. Man lässt auf Raumtemperatur erwärmen, giesst auf ca. 700 ml Wasser und rührt während 1 Stunde. Man extrahiert dann dreimal mit Methylenchlorid, wäscht zweimal mit Wasser, trocknet über Magnesiumsulfat und destilliert das Lösungsmittel ab. Durch Chromatographieren an Kieselgel unter Eluieren mit Essigester und anschliessendes Umkristallisieren aus Essigester erhält man tert.-Butyl (S)-12,12a-dihydro-8-jod-9-oxo-9H,11H-azeto[2,1-c]imidazo[1,5-a][1,4] benzodiazepin-1-carboxylat vom Schmelzpunkt 249–250°.

c) Man rührt 3,0 g (5,5 mMol) tert.-Butyl (S)-12,12a-dihydro-8-jod-9-oxo-9H,11H-azeto[2,1-c]imidazo[1,5-a][1,4]benzodiazepin-1-carboxylat, 20 ml (250 mMol) Hydroxymethyl-cyclopropan und 0,7 g (3 mMol) Tetraäthyl-orthotitanat über Nacht bei 120°, dampft die Lösung zur Trockne ein, nimmt den Rückstand in Chloroform auf und rührt während einer halben Stunde mit 30 ml einer gesättigten Kaliumfluoridlösung. Man filtriert die entstandene Emulsion durch Kieselgur, trennt die organische Phase ab, wäscht sie mit Wasser, trocknet über Magnesiumsulfat und dampft ein. Durch Umkristallisieren aus Essigester erhält man Cyclopropylmethyl (S)-12,12a-dihydro-8-jod-9-oxo-9H,11H-azeto[2,1-c]imidazo[1,5-a][1,4]benzodiazepin-1-carboxylat vom Schmelzpunkt 190–191°.

Beispiel 8

a) Man erwärmt 14,6 g (0,050 Mol) 6-Jod-isatosäureanhydrid und 6,6 g (0,058 Mol) L-Prolin in 50 ml Dimethylsulfoxid während 30 Minuten auf 70°, entfernt das Lösungsmittel im Hochvakuum und erhitzt das erhaltene Öl während 15 Minuten auf 170°. Das Rohprodukt wird durch Chromatographieren an Kieselgel gereinigt, wobei man als Elutionsmittel Methylenchlorid und ein Gemisch aus Methylenchlorid und Essigester verwendet.

Man erhält (S)-1,2,3,11a-Tetrahydro-6-jod-5H-pyrrolo[2,1-c][1,4]benzodiazepin-5,11(10H)-dion, das nach Umkristallisieren aus Methanol bei 212–215° schmilzt.

b) Man versetzt eine Lösung von 10,0 g (29,2 mMol) (S)-1,2,3,11a-Tetrahydro-6-jod-5H-pyrrolo[2,1-c][1,4]benzodiazepin-5,11(10H)-dion in 30 ml trockenem Dimethylformamid bei −20° unter Rühren mit 1,4 g (32,1 mMol) Natriumhydrid (55proz. Öldispersion), rührt noch während 1 Stunde bei obiger Temperatur und tropft anschliessend bei −45° 4,8 ml (32,1 mMol) Diäthylchlorphosphat dazu.

Inzwischen löst man 3,6 g (32,1 mMol) Kalium-tert.-butylat in 8 ml trockenem Dimethylformamid, kühlt in einem Aceton/Trockeneisbad ab, versetzt mit 4,5 g (32,1 mMol) Isocyanessigsäure-tert.-butylester und tropft die erhaltene Lösung bei −20° zum obigen Reaktionsgemisch. Man rührt noch während 15 Minuten bei −20°, neutralisiert mit 1,9 ml Eisessig, giesst auf 150 ml Wasser und extrahiert dreimal mit Methylenchlorid. Die organischen Auszüge werden zweimal mit Wasser gewaschen, über Magnesiumsulfat getrocknet und eingedampft. Das erhaltene Rohprodukt chromatographiert man unter Eluieren mit Essigester, das 30% n-Hexan enthält, an Kieselgel und kristallisiert anschliessend aus Essigester um. Man erhält tert.-Butyl (S)-11,12,13,13a-tetrahydro-8-jod-9-oxo-9H-imidazo[1,5-a]pyrrolo[2,1-c][1,4]benzodiazepin-1-carboxylat vom Schmelzpunkt 241–242°.

c) Man rührt 5 g (11 mMol) tert.-Butyl (S)-11,12,13,13a-tetrahydro-8-jod-9-oxo-9H-imidazo[1,5-a]pyrrolo[2,1-c][1,4]benzodiazepin-1-carboxylat, 30 g (300 mMol) Cyclohexanol und 1 g (4 mMol) Tetraäthyl-orthotitanat über Nacht bei 125°, dampft die Lösung zur Trockne ein und nimmt den Rückstand in Chloroform auf. Man rührt dann eine halbe Stunde mit 40 ml einer gesättigten Kaliumfluoridlösung, filtriert die entstandene Emulsion durch Kieselgur, trennt die organische Phase ab, wäscht sie mit Wasser, trocknet über Magnesiumsulfat und dampft ein. Durch Kristallisieren aus Essigester erhält man Cyclohexyl (S)-11,12,13,13a-tetrahydro-8-jod-9-oxo-9H-imidazo[1,5-a]pyrrolo[2,1-c][1,4]benzodiazepin-1-carboxylat vom Schmelzpunkt 181–182°.

Beispiel 9

Man rührt 12 g (26,6 mMol) tert.-Butyl (S)-12,12a-dihydro-8-jod-9-oxo-9H,11H-azeto[2,1-c]imidazo[1,5-a][1,4]benzodiazepin-1-carboxylat, 70 ml (660 mMol) Cyclohexanol und 2 g (8 mMol) Tetraäthyl-orthotitanat über Nacht bei 120°, dampft die Lösung zur Trockene ein und nimmt den Rückstand in Chloroform auf. Man rührt dann eine halbe Stunde mit 120 ml einer gesättigten Kaliumfluoridlösung, filtriert die entstandene Emulsion durch Kieselgur, trennt die organische Phase ab, wäscht sie mit Wasser, trocknet über Magnesiumsulfat und dampft ein. Durch Kristallisieren aus Essigester und Hexan erhält man Cyclohexyl (S)-12,12a-dihydro-8-jod-9-oxo-9H,11H-azeto[2,1-c]imidazo[1,5-a][1,4]benzodiazepin-1-carboxylat vom Schmelzpunkt 124–125°.

Beispiel 10

Man rührt 4,65 g (10 mMol) tert.-Butyl (S)-11,12,13,13a-tetrahydro-8-jod-9-oxo-9H-imidazo[1,5-a]pyrrolo[2,1-c][1,4]benzodiazepin-1-carboxylat, 24 g (330 mMol) Hydroxymethyl-cyclopropan und 3 g (13 mMol) Tetraäthyl-orthotitanat über Nacht bei 115°; dampft die Lösung zur Trockene ein und nimmt den Rückstand in Chloroform auf. Man rührt eine halbe Stunde mit 40 ml einer gesättigten Kaliumfluoridlösung, filtriert die entstandene Emulsion durch Kieselgur, trennt die organische Phase ab, wäscht sie mit Wasser, trocknet über Magnesiumsulfat und dampft ein. Durch Kristallisieren aus Essigester und Hexan erhält man Cyclopropylmethyl (S)-11,12,13,13a-tetrahydro-8-jod-9-oxo-9H-imidazo-[1,5-a]pyrrolo[2,1-c][1,4]benzodiazepin-1-carboxylat vom Schmelzpunkt 164–165°.

Beispiel 11

a) Man rührt ein Gemisch aus 4,8 g (24,3 mMol) 6-Chlor-isatosäureanhydrid, 2,83 g (25 mMol) (L)-3,4-Dehydroprolin und 20 ml Dimethylsulfoxid während 1,25 Stunden bei 100°, giesst anschliessend auf 200 ml Wasser und extrahiert dreimal mit Essigester. Die Essigesterlösung wird einmal mit Wasser gewaschen, über Magnesiumsulfat getrocknet, filtriert und eingedampft. Man chromatographiert den erhaltenen Rückstand an Kieselgel, kristallisiert anschliessend aus Essigester und erhält (S)-6-Chlor-3,11a-dihydro-5H-pyrrolo[2,1-c][1,4]benzodiazepin-5,11(10H)-dion vom Schmelzpunkt 254–256°.

b) Man versetzt unter Rühren eine Suspension von 1,9 g (44,6 mMol) Natriumhydrid (55proz. Öldispersion) in 80 ml trockenem Dimethylformamid bei −10° mit 10,0 g (40,2 mMol) (S)-6-Chlor-3,11a-dihydro-5H-pyrrolo[2,1-c][1,4]benzodiazepin-5,11(10H)-dion, rührt während 1 Stunde und tropft anschliessend bei −35° 7,7 ml (44,6 mMol) Diäthylchlorphosphat dazu.

Inzwischen löst man 4,9 g (44,4 mMol) Kalium-tert.-butylat in 15 ml trockenem Dimethylformamid, kühlt in einem Aceton/Trockeneisbad ab, versetzt mit 6,31 g (44,6 mMol) Isocyanessigsäure-tert.-butylester und gibt die erhaltene Lösung bei −15° tropfenweise zum obigen Reaktionsgemisch. Man lässt auf 15° erwärmen, neutralisiert mit 2,5 ml Eisessig, giesst auf 150 ml Wasser und extrahiert dreimal mit Methylenchlorid. Die organischen Auszüge werden zweimal mit Wasser gewaschen, über Magnesiumsulfat getrocknet und eingedampft. Das erhaltene Rohprodukt chromatographiert man unter Eluieren mit Essigester an Kieselgel. Nach Umkristallisieren aus Essigester/n-Hexan erhält man tert.-Butyl 8-chlor-11,13a-dihydro-9-oxo-9H-imidazo[1,5-a]pyrrolo[2,1-c][1,4]benzodiazepin-1-carboxylat vom Schmelzpunkt 227–229°.

c) Man rührt 2,0 g (5,4 mMol) tert.-Butyl 8-chlor-11,13a-dihydro-9-oxo9H-imidazo[1,5-a]

pyrrolo[2,1-c][1,4]benzodiazepin-1-carboxylat, 0,5 g (2,1 mMol) Tetraäthyl-orthotitanat und 25 ml Hydroxymethyl-cyclopropan während 23 Stunden bei 130°, wobei man dreimal je ca. 2 ml Lösungsmittel im Vakuum abdestilliert. Anschliessend dampft man im Vakuum ein, versetzt den Rückstand mit Wasser und Chloroform, rührt während 30 Minuten, filtriert über Kieselgur und trennt die organische Phase ab. Man extrahiert die wässrige Phase zweimal mit Chloroform, wäscht die organischen Auszüge einmal mit gesättigter Natriumchloridlösung, trocknet über Magnesiumsulfat und dampft ein. Das Rohprodukt chromatographiert man unter Eluieren mit Essigester an Kieselgel. Nach Umkristallisieren aus Essigester/n-Hexan erhält man Cyclopropylmethyl 8-chlor-11,13a-dihydro-9-oxo-9H-imidazo-[1,5-a]pyrrolo[2,1-c][1,4]benzodiazepin-1-carboxylat vom Schmelzpunkt 189–190°.

Beispiel 12

Man rührt 4,51 g (10 mMol) tert.-Butyl (S)-12,12a-dihydro-8-jod-9-oxo-9H,11H-azeto [2,1-c]imidazo[1,5-a][1,4]benzodiazepin-1-carboxylat, 10,3 g (120 mMol) Hydroxymethyl-cyclobutan und 0,8 g (3 mMol) Tetraäthyl-orthotitanat 2,5 Stunden bei 130°, dampft die Lösung zur Trockene ein und nimmt den Rückstand in Chloroform auf. Man rührt eine halbe Stunde mit 40 ml einer gesättigten Kaliumfluoridlösung, filtriert die entstandene Emulsion durch Kieselgur, trennt die organische Phase ab, wäscht sie mit Wasser, trocknet über Magnesiumsulfat und Kristallisieren aus Essigester erhält man Cyclobutylmethyl (S)-12,12a-dihydro-8-jod-9-oxo-9H,11H-azeto[2,1-c]imidazo[1,5-a][1,4]benzodiazepin-1-carboxylat vom Schmelzpunkt 169–170°.

Beispiel 13

Man rührt 3,5 g (7,7 mMol) tert.-Butyl (S)-12,12a-dihydro-8-jod-9-oxo-9H,11H-azeto [2,1-c]imidazo[1,5-a][1,4]benzodiazepin-1-carboxylat, 10 g (116 mMol) Cyclopentanol und 0,8 g (3 mMol) Tetraäthyl-orthotitanat über Nacht bei 125°, dampft die erhaltene Lösung ein und nimmt den Rückstand in Chloroform auf. Man rührt eine halbe Stunde mit 40 ml einer gesättigten Kaliumfluoridlösung, filtriert die entstandene Emulsion durch Kieselgur, trennt die organische Phase ab, wäscht sie mit Wasser, trocknet über Magnesiumsulfat und dampft ein. Durch eine rasche Filtration durch ca. 200 g Kieselgel unter Eluieren mit Essigester und anschliessendes Kristallisieren aus Essigester erhält man Cyclopentyl (S)-12,12a-dihydro-8-jod-9-oxo-9H,11H-azeto[2,1-c] imidazo[1,5-a][1,4]benzodiazepin-1-carboxylat vom Schmelzpunkt 184–185°.

Beispiel 14

a) Man rührt 25 g (103 mMol) 6-Bromisatosäureanhydrid und 10,44 g (103 mMol) L-Azetidin-2-carbonsäure in 100 ml Dimethylsulfoxid während 25 Minuten bei 95°, entfernt das Dimethylsulfoxid im Vakuum und erhitzt den Rückstand während 3 Stunden im Hochvakuum auf 150°. Durch Chromatographieren des erhaltenen Materials an Kieselgel unter Eluieren mit Essigester und anschliessendes Umkristallisieren aus Essigester erhält man (S)-5-Brom-1,10a-dihydro-azeto[2,1-c][1,4]benzodiazepin-4,10(2H,9H)-dion vom Schmelzpunkt 182–183°.

b) Man versetzt eine Suspension von 19 g (67,3 mMol) (S)-5-Brom-1,10a-dihydro-azeto[2,1-c][1,4]benzodiazepin-4,10(2H,9H)-dion in 60 ml trockenem Dimethylformamid unter Rühren bei −20° mit 1,75 g (73 mMol) Natriumhydrid (55proz. Öldispersion, mit n-Hexan gewaschen), rührt während 20 Minuten bei −20°, versetzt dann mit 12,6 g (73 mMol) Diäthylchlorphosphat und rührt noch 0,5 Stunden bei dieser Temperatur.

Inzwischen löst man 8,2 g (73 mMol) Kalium-tert.-butylat in 20 ml trockenem Dimethylformamid, kühlt in einem Aceton/Trockeneisbad ab, versetzt mit 10,3 g (73 mMol) Isocyanessigsäure-tert.-butylester und tropft die so erhaltene Lösung zum obigen Reaktionsgemisch. Man lässt auf Raumtemperatur erwärmen, giesst auf ca. 500 ml Wasser und rührt noch 1 Stunde. Man extrahiert anschliessend dreimal mit Methylenchlorid, wäscht die organische Phase zweimal mit Wasser, trocknet über Magnesiumsulfat und dampft ein. Durch Chromatographieren an Kieselgel unter Eluieren mit Essigester und anschliessendes Kristallisieren aus Essigester erhält man tert.-Butyl (S)-8-brom-12,12a-dihydro-9-oxo-9H,11H-azeto[2,1-c]imidazo[1,5-a][1,4] benzodiazepin-1-carboxylat vom Schmelzpunkt 240–241°.

c) Man rührt 8 g (2 mMol) tert.-Butyl (S)-8-brom-12,12a-dihydro-9-oxo-9H,11H-azeto[2,1-c]imidazo[1,5-a][1,4]benzodiazepin-1-carboxylat, 25 g (250 mMol) Cyclohexanol und 1,8 g (8 mMol) Tetraäthyl-orthotitanat über Nacht bei 120°, entfernt das überschüssige Cyclohexanol im Wasserstrahlvakuum und nimmt den Rückstand in Chloroform auf. Man rührt eine halbe Stunde mit 40 ml eines 1:1-Gemisches aus konzentrierter Salzsäure und Wasser, trennt die organische Phase ab, wäscht sie einmal mit 1N-Salzsäure und einmal mit gesättigter Natriumbicarbonatlösung, trocknet über Magnesiumsulfat und dampft ein. Durch Umkristallisieren aus Äthanol erhält man Cyclohexyl (S)-8-brom-12,12a-dihy dro-9-oxo-9H,11H-azeto[2,1-c]imidazo[1,5-a][1,4] benzodiazepin-1-carboxylat vom Schmelzpunkt 203–204°.

Beispiel 15

Man rührt 8 g (20 mMol) tert.-Butyl (S)-8-brom-12,12a-dihydro-9-oxo-9H,11H-azeto[2,1-c]imidazo[1,5-c][1,4]benzodiazepin-1-carboxylat, 25 g (300 mMol) Hydroxymethyl-cyclopropan und 1,8 g (8 mMol) Tetraäthyl-orthotitanat über Nacht bei 120°, dampft die Lösung zur Trockene ein und nimmt den Rückstand in Chloroform auf. Man rührt während einer halben Stunde mit 40 ml einer gesättigten Kaliumfluoridlösung, filtriert die entstandene Emulsion durch Kieselgur, trennt die organische Phase ab, wäscht sie mit Wasser, trocknet über Magnesiumsulfat und dampft ein.

Durch Umkristallisieren aus Äthanol erhält man Cyclopropylmethyl (S)-8-brom-12,12a-dihydro-9-oxo-9H,11H-azeto[2,1-c]imidazo[1,5-a][1,4]benzodiazepin-1-carboxylat vom Schmelzpunkt 212–213°.

Beispiel 16

a) 11,3 g (0,057 Mol) 6-Chlorisatosäureanhydrid und 5,78 g (0,057 Mol) L-Azetidin-2-carbonsäure werden in 50 ml Dimethylsulfoxid während 2 Stunden auf 125° erhitzt. Anschliessend dampft man im Hochvakuum zur Trockene ein und erhitzt den erhaltenen Rückstand während 2 Stunden auf 150°. Durch Chromatographieren an Kieselgel unter Eluieren mit Essigester erhält man (S)-5-Chlor-1,10a-dihydroazeto[2,1-c][1,4]benzodiazepin-4,10(2H,9H)-dion vom Schmelzpunkt 225–228°.

b) Man versetzt eine Suspension von 0,47 g (10,8 mMol) Natriumhydrid (55proz. Öldispersion) in 10 ml trockenem Dimethylformamid bei −15° und unter Rühren mit 2,12 g (9,0 mMol) (S)-5-Chlor-1,10a-dihydroazeto[2,1-c][1,4]benzodiazepin-4,10(2H,9H)-dion, rührt noch während 1 Stunde und tropft anschliessend bei −35° 1,8 ml (10,8 mMol) Diäthylchlorphosphat dazu.

Inzwischen löst man 1,18 g (10,8 mMol) Kalium-tert.-butylat in 8 ml trockenem Dimethylformamid, kühlt in einem Aceton/Trockeneisbad ab, versetzt mit 1,52 g (10,8 mMol) Isocyanessigsäure-tert.-butylester und tropft die erhaltene Lösung bei −15° zum obigen Reaktionsgemisch. Man lässt auf 10° erwärmen, neutralisiert mit 0,6 ml Eisessig, giesst auf 80 ml Wasser und extrahiert dreimal mit Chloroform. Die Chloroformlösung wird zweimal mit Wasser gewaschen, über Magnesiumsulfat getrocknet und eingedampft. Das erhaltene Rohprodukt chromatographiert man unter Eluieren mit Essigester an Kieselgel, kristallisiert anschliessend aus Essigester um und erhält tert.-Butyl (S)-8-chlor-12,12a-dihydro-9-oxo-9H,11H-azeto[2,1-c]imidazo[1,5-a][1,4]benzodiazepin-1-carboxylat vom Schmelzpunkt 235–236°.

c) Man rührt 12,0 g (33,4 mMol) tert.-Butyl (S)-8-chlor-12,12a-dihydro-9-oxo-9H,11H-azeto[2,1-c]imidazo[1,5-a][1,4]benzodiazepin-1-carboxylat, 3,4 g (13,6 mMol) Tetraäthyl-orthotitanat und 75 ml Cyclohexanol während 20 Stunden bei 125°, wobei man zweimal ein wenig Lösungsmittel im Vakuum abdestilliert. Anschliessend dampft man im Vakuum ein, löst den Rückstand in heissem Chloroform, versetzt mit 200 ml Wasser und rührt während 30 Minuten. Man filtriert dann über Kieselgur, trennt die organische Phase ab, extrahiert die wässrige Phase zweimal mit Chloroform, trocknet die organischen Auszüge über Magnesiumsulfat und dampft sie ein. Nach Chromatographieren an Kieselgel unter Eluieren mit Essigester und anschliessendem Umkristallisieren aus Essigester erhält man Cyclohexyl (S)-8-chlor-12,12a-dihydro-9-oxo-9H,11H-azeto[2,1-c]imidazo[1,5-a][1,4]benzodiazepin-1-carboxylat vom Schmelzpunkt 223–224°.

Beispiel 17

Man rührt 12 g (35 mMol) Äthyl (S)-8-chlor-11,12,13,13a-tetrahydro-9-oxo-9H-imidazo[1,5-a]-pyrrolo[2,1-c][1,4]benzodiazepin-1-carboxylat und 1 g pulverisiertes Kaliumcyanid in 120 ml Cyclohexanol während 18 Stunden bei 130°, wobei man im Verlauf der Reaktion dreimal je ca. 10 ml Cyclohexanol abdestilliert. Anschliessend dampft man das Reaktionsgemisch ein, nimmt den Rückstand in Chloroform auf, wäscht mit Wasser, trocknet über Magnesiumsulfat und dampft ein. Durch Säulenchromatographie an Kieselgel erhält man Cyclohexyl (S)-8-chlor-11,12,13,13a-tetrahydro-9-oxo-9H-imidazo[1,5-a]pyrrolo[2,1-c]-[1,4]benzodiazepin-1-carboxylat vom Schmelzpunkt 166–167°.

Beispiel 18

a) Man versetzt 15 g (43,4 mMol) Äthyl-(S)-8-chlor-11,12,13,13a-tetrahydro-9-oxo-9H-imidazo[1,5-a]pyrrolo[2,1-c][1,4]benzodiazepin-1-carboxylat und 1,85 g (46,3 mMol) Natriumhydroxid mit 60 ml Äthanol und 10 ml Wasser, erhitzt dann während 45 Minuten unter Rückfluss zum Sieden, destilliert anschliessend das Äthanol im Vakuum ab, versetzt schliesslich mit 46,5 ml 1N-Salzsäure und lässt während 2 Stunden im Eisbad stehen. Das ausgefallene Material wird abgenutscht, mit Wasser gewaschen und bis zur Gewichtskonstanz getrocknet. Man erhält (S)-8-Chlor-11,12,13,13a-tetrahydro-9-oxo-9H-imidazo[1,5-a]pyrrolo[2,1-c][1,4]benzodiazepin-1-carbonsäure mit einem Zersetzungspunkt von 265°.

b) Man rührt 9,54 g (30 mMol) (S)-8-Chlor-11,12,13,13a-tetrahydro-9-oxo-9H-imidazo[1,5-a]pyrrolo[2,1-c][1,4]benzodiazepin-1-carbonsäure und 6,32 g (39 mMol) N,N′-Carbonyldiimidazol in 50 ml trockenem Dimethylformamid während 1 Stunde bei Raumtemperatur und während 1 Stunde bei 50°. Anschliessend giesst man auf ca. 300 ml Wasser, nutscht das ausgefallene Material ab, wäscht es mit Wasser und trocknet es bis zur Gewichtskonstanz. Man erhält (S)-1-[(8-Chlor-11,12,13,13a-tetrahydro-9-oxo-9H-imidazo[1,5-a]pyrrolo[2,1-c][1,4]benzodiazepin-1-yl)carbonyl]imidazol vom Schmelzpunkt 240–241,5°.

c) Man rührt eine Mischung aus 601 mg (6 mMol) Cyclohexanol, 10 ml Dimethylformamid und 288 mg (6 mMol) Natriumhydrid (55proz. Öldispersion) während einer halben Stunde bei Raumtemperatur, versetzt die erhaltene Lösung mit 2,20 g (6 mMol) (S)-1-[(8-Chlor-11,12,13,13a-tetrahydro-9-oxo-9H-imidazo[1,5-a]pyrrolo[2,1-c][1,4]benzodiazepin-1-yl)carbonyl]imidazol, rührt noch eine halbe Stunde bei Raumtemperatur und giesst dann das Reaktionsgemisch auf ca. 70 ml Wasser. Das ausgefallene Material wird abgenutscht, mit Wasser gewaschen und getrocknet. Durch Umkristallisieren aus Essigester erhält man Cyclohexyl (S)-8-chlor-11,12,13,13a-tetrahydro-9-oxo-9H-imidazo[1,5-a]pyrrolo[2,1-c][1,4]benzodiazepin-1-carboxylat vom Schmelzpunkt 166–167°.

Beispiel 19

Man rührt 3,45 g (10 mMol) Äthyl (S)-8-chlor-11,12,13,13a-tetrahydro-9-oxo-9H-imidazo[1,5-a]pyrrolo[2,1-c][1,4]benzodiazepin-1-carboxylat und 70 mg pulverisiertes Kaliumcyanid in 10 ml Cyclopentanol über Nacht bei Siedetemperatur, entfernt das überschüssige Cyclopentanol im Vakuum, nimmt den Rückstand in Chloroform auf, filtriert und dampft ein. Durch Umkristallisieren aus Essigester/Hexan erhält man Cyclopentyl (S)-8-chlor-11,12,13,13a-tetrahydro-9-oxo-9H-imidazo[1,5-a]pyrrolo[2,1-c][1,4]benzodiazepin-1-carboxylat vom Schmelzpunkt 210,5–212°.

Beispiel 20

Man erhitzt 10,35 g (0,03 mMol) Äthyl (S)-8-chlor-11,12,13,13a-tetrahydro-9-oxo-9H-imidazo[1,5-a]pyrrolo[2,1-c][1,4]benzodiazepin-1-carboxylat, 25 ml Hydroxymethylcyclobutan und 0,5 g pulverisiertes Kaliumcyanid während 2 Stunden auf 165°, wobei man das gebildete Äthanol laufend abdestilliert. Anschliessend dampft man im Vakuum ein, nimmt den Rückstand in Chloroform auf, wäscht die Chloroformlösung dreimal mit Wasser, trocknet über Magnesiumsulfat und dampft ein. Durch Umkristallisieren des erhaltenen Materials aus Essigester erhält man Cyclobutylmethyl (S)-8-chlor-11,12,13,13a-tetrahydro-9-oxo-9H-imidazo[1,5-a]pyrrolo[2,1-c][1,4]benzodiazepin-1-carboxylat vom Schmelzpunkt 204–205°.

Beispiel 21

Man rührt eine (5 mMol) (S)-1-[(8-Chlor-11,12,13,13a-tetrahydro-9-oxo-9H-imidazo[1,5-a]pyrrolo[2,1-c][1,4]benzodiazepin-1-yl)carbonyl]imidazol, 0,56 g (6 mMol) Phenol, 0,83 g (6 mMol) pulverisiertem Kaliumcarbonat und 20 ml trockenem Dimethylformamid während 26 Stunden bei Raumtemperatur, giesst dann auf 100 ml Wasser und extrahiert dreimal mit Chloroform. Man wäscht die Chloroformlösung zweimal mit Wasser und dreimal mit gesättigter Natriumchloridlösung, trocknet über Magnesiumsulfat und dampft ein. Das Rohprodukt wird durch Chromatographieren an Kieselgel unter Eluieren mit Essigester und Umkristallisieren aus Essigester gereinigt. Man erhält Phenyl (S)-8-chlor-11,12,13,13a-tetrahydro-9-oxo-9H-imidazo[1,5-a]pyrrolo[2,1-c][1,4]benzodiazepin-1-carboxylat vom Schmelzpunkt 211–212°.

Beispiel 22

a) Man versetzt eine Lösung von 7,03 g (30 mMol) (S)-7-Fluor-1,2,3,11a-tetrahydro-5H-pyrrolo[2,1-c][1,4]benzodiazepin-5,11(10H)-dion in 50 ml trockenem Dimethylformamid unter Rühren bei −25° mit 1,51 g (34,5 mMol) Natriumhydrid (55proz. Öldispersion), rührt noch während 50 Minuten bei −20 bis −10° und tropft anschliessend bei −40° 9,1 ml (34,5 mMol) Diäthylchlorphosphat dazu.

Inzwischen löst man 3,86 g (34,5 mMol) Kalium-tert.-butylat in 10 ml trockenem Dimethylformamid, kühlt in einem Aceton/Trockeneisbad ab, versetzt mit 4,86 g (34,5 mMol) Isocyanessigsäure-tert.-butylester und tropft die so erhaltene Lösung bei −15° zum obigen Reaktionsgemisch. Man lässt auf 5° erwärmen, neutralisiert mit 3,9 ml Eisessig, giesst auf 250 ml Wasser und rührt noch während 0,5 Stunden. Man extrahiert dann dreimal mit Methylenchlorid, wäscht die organische Lösung zweimal mit Wasser, trocknet über Magnesiumsulfat und destilliert das Lösungsmittel ab. Das erhaltene Rohprodukt chromatographiert man unter Eluieren mit Essigester an Kieselgel. Durch Kristallisation aus Äthanol erhält man tert.-Butyl (S)-7-fluor-11,12,13,13a-tetrahydro-9-oxo-9H-imidazo[1,5-a]pyrrolo[2,1-c][1,4]benzodiazepin-1-carboxylat vom Schmelzpunkt 154–155°.

b) Man rührt 11,85 g (33,2 mMol) tert.-Butyl (S)-7-fluor-11,12,13,13a-tetrahydro-9-oxo-9H-imidazo[1,5-a]pyrrolo[2,1-c][1,4]benzodiazepin-1-carboxylat, 30 g (400 mMol) Hydroxymethyl-cyclopropan und 6 ml Tetraäthyl-orthotitanat über Nacht bei 115°, worauf man das Reaktionsgemisch eindampft und den Rückstand in Chloroform löst. Man rührt die Lösung eine halbe Stunde mit ca. 40 ml einer gesättigten Kaliumfluoridlösung, filtriert das Gemisch durch Kieselgur und trennt die organische Phase ab. Diese wird mit Wasser gewaschen, über Magnesiumsulfat getrocknet und eingedampft. Durch Chromatographieren an einer Kieselgelsäule und anschliessendes Kristallisieren aus Essigester erhält man Cyclopropylmethyl (S)-7-fluor-11,12,13,13a-tetrahydro-9-oxo-9H-imidazo[1,5-a]pyrrolo[2,1-c][1,4]benzodiazepin-1-carboxylat vom Schmelzpunkt 140–142°.

Beispiel 23

a) Man erhitzt 13,0 g (128,4 mMol) L-Azetidin-2-carbonsäure und 22,7 g (128,4 mMol) 6-Methylisatosäureanhydrid in 150 ml Dimethylsulfoxid während 3 Stunden auf 95°, dampft im Hochvakuum zur Trockne ein und erhitzt den erhaltenen Rückstand während 2,25 Stunden auf 140°. Durch Kristallisieren aus Essigester erhält man (S)-1,10a-Dihydro-5-methyl-2H-azeto[2,1-c][1,4]benzodiazepin-4,10(9H)-dion vom Schmelzpunkt 159–160°.

b) Man versetzt eine Lösung von 13,8 g (63,8 mMol) (S)-1,10a-Dihydro-5-methyl-2H-azeto[2,1-c][1,4]benzodiazepin-4,10(9H)-dion in 55 ml trockenem Dimethylformamid bei −20 bis −10° unter Rühren mit 3,06 g (70,2 mMol) Natriumhydrid (55proz. Öldispersion), rührt noch während 40 Minuten bei obiger Temperatur und tropft anschliessend bei −35° 10,5 ml (70,2 mMol) Diäthylchlorphosphat dazu.

Inzwischen löst man 7,88 g (70,2 mMol) Kalium-tert.-butylat in 12 ml trockenem Dimethylformamid, kühlt in einem Aceton/Trockeneisbad ab, versetzt mit 11 g (70,2 mMol) ca. 90proz. Isocyanessigsäure-tert.-butylester und tropft die erhaltene Lösung bei −20 bis −15° zum obigen Reaktionsgemisch. Man lässt auf 0° erwärmen, neutralisiert mit 4 ml Eisessig, giesst auf 300 ml Wasser und extrahiert dreimal mit Methylenchlorid. Die

Methylenchloridlösung wird zweimal mit Wasser gewaschen, über Magnesiumsulfat getrocknet und eingedampft. Das erhaltene Rohprodukt chromatographiert man unter Eluieren mit Essigester, das 50% n-Hexan enthält, an Kieselgel. Nach Umkristallisieren aus Essigester/n-Hexan erhält man tert.-Butyl (S)-12,12a-dihydro-8-methyl-9-oxo-9H,11H-azeto[2,1-c]imidazo[1,5-a][1,4]benzodiazepin-1-carboxylat vom Schmelzpunkt 191–192°.

c) Man erhitzt 3,39 g (10 mMol) tert.-Butyl (S)-12,12a-dihydro-8-methyl-9-oxo-9H,11H-azeto-[2,1-c]imidazo[1,5-a][1,4]benzodiazepin-1-carboxylat, 50 ml Cyclohexanol und 1 g Tetraäthyl-orthotitanat während 16 Stunden auf 130°, wobei man nach 2 und 4 Stunden jeweils etwas Lösungsmittel abdestilliert. Anschliessend dampft man im Vakuum ein, löst den Rückstand in Chloroform und giesst auf Wasser. Nach Filtrieren durch Kieselgur trennt man die Chloroformphase ab, trocknet sie über Magnesiumsulfat und dampft ein. Durch Kristallisieren aus Essigester erhält man Cyclohexyl (S)-12,12a-dihydro-8-methyl-9-oxo-9H,11H-azeto[2,1-c]imidazo[1,5-a][1,4]benzodiazepin-1-carboxylat vom Schmelzpunkt 199–200°.

Beispiel 24

Man rührt 5 g (14,5 mMol) Äthyl (S)-8-chlor-11,12,13,13a-tetrahydro-9-oxo-9H-imidazo[1,5-a]pyrrolo[2,1-c][1,4]benzodiazepin-1-carboxylat, 70 mg pulverisiertes Kaliumcyanid und 20 ml Benzylalkohol während 1 Stunde bei 110°, dampft zur Trockene ein und nimmt den Rückstand in Chloroform auf. Man wäscht die Lösung mit Wasser, trocknet über Magnesiumsulfat und dampft ein. Durch Umkristallisieren aus Äthanol und Äther erhält man Benzyl (S)-8-chlor-11,12,13,13a-tetrahydro-9-oxo-9H-imidazo[1,5-a]pyrrolo[2,1-c][1,4]benzodiazepin-1-carboxylat vom Schmelzpunkt 141–142°.

Beispiel 25

Man versetzt 1,2 g (12 mMol) 1-Methyl-cyclopentanol in 25 ml trockenem Dimethylformamid mit 0,5 g (10,3 mMol) Natriumhydrid (55proz. Öldispersion) und gibt zur erhaltenen Lösung bei −20° portionenweise 3,79 g (10,3 mMol) (S)-1-[(8-Chlor-11,12,13,13a-tetrahydro-9-oxo-9H-imidazo[1,5-a]pyrrolo[2,1-c][1,4]benzodiazepin-1-yl)carbonyl]imidazol. Man rührt 1 Stunde bei Raumtemperatur, giesst auf ca. 250 ml Wasser, nutscht das ausgefallene Material ab, wäscht es mit Wasser und trocknet. Man erhält 1-Methyl-cyclopentyl 8-chlor-11,12,13,13a-tetrahydro-9-oxo-9H-imidazo[1,5-a]pyrrolo[2,1-c][1,4]benzodiazepin-1-carboxylat vom Schmelzpunkt 190–191°.

Beispiel 26

Man rührt 3,19 g (10 mMol) Äthyl 7-chlor-5,6-dihydro-5-methyl-6-oxo-4H-imidazo[1,5-a][1,4]benzodiazepin-3-carboxylat, 70 mg pulverisiertes Kaliumcyanid und 10 ml Cyclopentanol über Nacht bei 120°, dampft anschliessend ein und chromatographiert den Rückstand unter Eluieren mit Essigester an Kieselgel. Durch Umkristallisieren aus Essigester erhält man Cyclopentyl 7-chlor-5,6-dihydro-5-methyl-6-oxo-4H-imidazo[1,5-a][1,4]benzodiazepin-3-carboxylat vom Schmelzpunkt 162–163°.

Beispiel 27

Man erhitzt eine Mischung aus 6,92 g (0,02 Mol) Äthyl (S)-8-chlor-11,12,13,13a-tetrahydro-9-oxo-9H-imidazo[1,5-a]pyrrolo[2,1-c][1,4]benzodiazepin-1-carboxylat, 100 ml Propargylalkohol und 0,4 g Kaliumcyanid während 4 Stunden unter Rückfluss zum Sieden, destilliert dann 75 ml des Lösungsmittels ab, gibt 75 ml Propargylalkohol zum Reaktionsgemisch und erhitzt während weiteren 16 Stunden unter Rückfluss zum Sieden. Man engt ein, gibt 25 ml Wasser dazu, extrahiert dreimal mit je 25 ml Chloroform, trocknet die Chloroformlösung über Magnesiumsulfat und dampft ein. Den Rückstand chromatographiert man unter Eluieren mit Essigester an Kieselgel. Durch Umkristallisieren aus Essigester erhält man 2-Propinyl (S)-8-chlor-11,12,13,13a-tetrahydro-9-oxo-9H-imidazo[1,5-a]pyrrolo[2,1-c][1,4]benzodiazepin-1-carboxylat vom Schmelzpunkt 213–214°.

Beispiel 28

Man rührt eine Mischung aus 6,9 g (20 mMol) Äthyl (S)-8-chlor-11,12,13,13a-tetrahydro-9-oxo-9H-imidazo[1,5-a]pyrrolo[2,1-c][1,4]benzodiazepin-1-carboxylat, 0,6 g (9,2 mMol) Kaliumcyanid und 70 ml Hydroxymethyl-cyclohexan während 16 Stunden bei 130°, wobei im Verlauf der Reaktion zweimal je etwas Lösungsmittel im Vakuum abdestilliert wird. Anschliessend dampft man im Hochvakuum ein, verteilt den Rückstand zwischen Wasser und Chloroform, wäscht die Chloroformlösung zweimal mit gesättigter Natriumchloridlösung und trocknet sie über Magnesiumsulfat. Nach dem Eindampfen der Chloroformlösung kristallisiert man den Rückstand aus Essigester/n-Hexan und erhält Cyclohexylmethyl (S)-8-chlor-11,12,13,13a-tetrahydro-9-oxo-9H-imidazo[1,5-a]pyrrolo[2,1-c][1,4]benzodiazepin-1-carboxylat vom Schmelzpunkt 123–124°.

Beispiel 29

Man rührt eine Mischung aus 6,9 g (20 mMol) Äthyl (S)-8-chlor-11,12,13,13a-tetrahydro-9-oxo-9H-imidazo[1,5-a]pyrrolo[2,1-c][1,4]benzodiazepin-1-carboxylat, 0,6 g (9,2 mMol) Kaliumcyanid und 30 ml Cyclopentanmethanol während 26 Stunden bei 130°, wobei man zweimal je ein wenig Lösungsmittel im Vakuum abdestilliert. Anschliessend dampft man im Hochvakuum ein, verteilt den Rückstand zwischen Wasser und Chloroform und extrahiert die wässrige Phase noch einmal mit Chloroform. Man wäscht die organischen Auszüge dreimal mit gesättigter Natriumchloridlösung, trocknet über Magnesiumsulfat und dampft ein. Man nimmt den Rückstand in siedendem Essigester auf, filtriert und dampft ein. Nach Kristallisieren des verbleibenden Öls aus Essigester/n-Hexan erhält man Cyclopentylmethyl (S)-8-chlor-11,12,13,13a-tetrahydro-9-oxo-9H-imidazo[1,5-a]pyrrolo[2,1-c][1,4]benzodiazepin-1-carboxylat vom Schmelzpunkt 112–114°.

Beispiel 30

Man rührt eine Mischung aus 3,45 g (10 mMol) Äthyl (S)-8-chlor-11,12,13,13a-tetrahydro-9-oxo-9H-imidazo[1,5-a]pyrrolo[2,1-c][1,4]benzodiazepin-1-carboxylat, 11,4 g (100 mMol) trans-2-Methylcyclohexanol und 100 mg pulverisiertem Kaliumcyanid während 60 Stunden bei 130°, worauf man die erhaltene Lösung zur Trockene eindampft und den Rückstand an einer Kieselgelsäule chromatographiert. Durch Umkristallisieren aus Essigester erhält man trans-2-Methylcyclohexyl (S)-8-chlor-11,12,13,13a-tetrahydro-9-oxo-9H-imidazo[1,5-a]pyrrolo[2,1-c][1,4]benzodiazepin-1-carboxylat vom Schmelzpunkt 213–215° (1:1-Gemisch der beiden Diastereoisomeren).

Beispiel 31

Man rührt eine Mischung aus 3,45 g (10 mMol) Äthyl (S)-8-chlor-11,12,13,13a-tetrahydro-9-oxo-9H-imidazo[1,5-a]pyrrolo[2,1-c][1,4]benzodiazepin-1-carboxylat, 100 mg pulverisiertem Kaliumcyanid und 10 g (100 mMol)) Tetrahydro-2H-pyran-4-ol während 20 Stunden bei 140°, worauf man das überschüssige Tetrahydro-2H-pyran-4-ol abdestilliert und den Rückstand in Chloroform aufnimmt. Man wäscht mit Wasser, trocknet über Magnesiumsulfat und dampft ein. Durch Kristallisieren aus Essigester erhält man Tetrahydro-2H-pyran-4-yl (S)-8-chlor-11,12,13,13a-tetrahydro-9-oxo-9H-imidazo[1,5-a]pyrrolo[2,1-c][1,4]-benzodiazepin-1-carboxylat vom Schmelzpunkt 226–227°.

Beispiel 32

Man rührt eine Mischung aus 20,0 g (62,5 mMol) Äthyl 7-chlor-5,6-dihydro-5-methyl-6-oxo-4H-imidazo[1,5-a][1,4]benzodiazepin-3-carboxylat, 1,0 g (15,4 mMol) Kaliumcyanid und 100 ml Propargylalkohol während 40 Stunden bei 130°, wobei man nach 16 Stunden ca. 50 ml Lösungsmittel im Vakuum abdestilliert und 50 ml Propargylalkohol dazugibt. Anschliessend dampft man im Vakuum zur Trockene ein, nimmt den Rückstand in Chloroform auf, wäscht die Chloroformlösung einmal mit Wasser und einmal mit gesättigter Natriumchloridlösung und trocknet sie über Magnesiumsulfat. Man nutscht vom Magnesiumsulfat ab und filtriert die Chloroformlösung durch Kieselgel, wobei man anschliessend mit Essigester eluiert. Nach dem Eindampfen der Eluate kristallisiert man zweimal aus Chloroform/Toluol und einmal aus Äthanol um und erhält 2-Propinyl 7-chlor-5,6-dihydro-5-methyl-6-oxo-4H-imidazo[1,5-a][1,4]benzodiazepin-3-carboxylat vom Schmelzpunkt 198–200°.

Beispiel 33

Man rührt eine Mischung aus 20,0 g (65,9 mMol) Äthyl 8-fluor-5,6-dihydro-5-methyl-6-oxo-4H-imidazo[1,5-a][1,4]benzodiazepin-3-carboxylat, 1,0 g (15,4 mMol) Kaliumcyanid und 100 ml Propargylalkohol während 20 Stunden bei 125° und während 1 Stunde bei 135°, dampft im Vakuum zur Trockene ein und nimmt den Rückstand in Chloroform auf. Man wäscht die Chloroformlösung zweimal mit Wasser, trocknet über Magnesiumsulfat und dampft ein. Den Rückstand chromatographiert man unter Eluieren mit Essigester an Kieselgel. Durch Umkristallisieren des erhaltenen Materials aus Chloroform/Toluol erhält man 2-Propinyl 8-fluor-5,6-dihydro-5-methyl-6-oxo-4H-imidazo[1,5-a][1,4]benzodiazepin-3-carboxylat vom Schmelzpunkt 220–222°.

Beispiel 34

Man erhitzt eine Mischung aus 5,0 g (15,6 mMol) Äthyl 7-chlor-5,6-dihydro-5-methyl-6-oxo-4H-imidazo[1,5-a][1,4]benzodiazepin-3-carboxylat, 20 ml Hydroxymethyl-cyclopropan und 0,5 g pulverisiertem Kaliumcyanid während 18 Stunden auf 130°, wobei man nach 1,3 und 6 Stunden jeweils etwa 1 ml Lösungsmittel abdestilliert. Man dampft anschliessend im Vakuum ein und verteilt den Rückstand zwischen Chloroform und Wasser. Die Chloroformphase wird dreimal mit Wasser gewaschen, über Magnesiumsulfat getrocknet und eingedampft. Das erhaltene Material wird unter Eluieren mit Essigester an Kieselgel chromatographiert und anschliessend aus Essigester kristallisiert. Man erhält Cyclopropylmethyl 7-chlor-5,6-dihydro-5-methyl-6-oxo-4H-imidazo[1,5-a][1,4]benzodiazepin-3-carboxylat vom Schmelzpunkt 194–195°.

Beispiel 35

a) Ausgehend von 3,4-Dichloranilin erhält man nach der Sandmeyer'schen Isatinsynthese [T. Sandmeyer, Helv. 2, 234 (1919)] 4,5-Dichlorisatin. Die Auftrennung der Isomeren nach P. W. Sadler, J. Org. Chemistry 21, 169 (1956), liefert nach Umkristallisieren reines 4,5-Dichlorisatin vom Schmelzpunkt 227–230°.

b) Man suspendiert 49 g (0,226 Mol) 4,5-Dichlorisatin in 200 ml Essigsäure und 200 ml Essigsäureanhydrid, versetzt über einen Zeitraum von 50 Minuten zwischen 80 und 90° mit 37,65 g (0,376 Mol) Chromtrioxid und kühlt anschliessend auf 5° ab. Das ausgefallene Material wird abgenutscht, mit Wasser gewaschen und getrocknet. Man erhält 5,6-Dichlorisatosäureanhydrid vom Schmelzpunkt 280–283°.

c) Man erhitzt eine Mischung aus 22 g (94,8 mMol) 5,6-Dichlorisatosäureanhydrid, 10,85 g (94,8 mMol) L-Prolin und 100 ml Dimethylsulfoxid während 0,5 Stunden auf 90°, dampft anschliessend im Hochvakuum zur Trockene ein und erhitzt den Rückstand während 1 Stunde auf 150°. Durch Chromatographieren an Kieselgel unter Eluieren mit Essigester und anschliessendes Kristallisieren aus Essigester und Hexan erhält man (S)-6,7-Dichlor-1,2,3,11a-tetrahydro-5H-pyrrolo-[2,1-c][1,4]benzodiazepin-5,11(10H)-dion vom Schmelzpunkt 203–205°.

d) Man versetzt eine Suspension von 21 g (73,6 mMol) (S)-6,7-Dichlor-1,2,3,11a-tetrahydro-5H-pyrrolo[2,1-c][1,4]benzodiazepin-5,11(10H)-dion in 60 ml trockenem Dimethylformamid unter Rühren bei −30° mit 3,20 g (85 mMol) Natriumhydrid (55 proz. Öldispersion), rührt während 0,5 Stunden bei −20° und tropft anschliessend 14,67 g (85 mMol) Diäthylchlorphosphat dazu.

Inzwischen löst man 9,53 g (85 mMol) Kalium-tert.-butylat in 30 ml trockenem Dimethylformamid, kühlt in einem Aceton/Trockeneisbad ab, versetzt mit 12 g (85 mMol) Isocyanessigsäure-tert.-butylester und tropft die so erhaltene Lösung bei −15° zum obigen Reaktionsgemisch. Man lässt auf Raumtemperatur erwärmen, giesst auf 400 ml Wasser und rührt noch während 1 Stunde. Man extrahiert dreimal mit Methylenchlorid, wäscht die organische Phase zweimal mit Wasser, trocknet über Magnesiumsulfat und destilliert das Lösungsmittel ab. Das erhaltene Rohprodukt chromatographiert man unter Eluieren mit Essigester an Kieselgel, kristallisiert anschliessend aus Essigester um und erhält tert.-Butyl (S)-7,8-dichlor-11,12,13,13a-tetrahydro-9-oxo-9H-imidazo[1,5-a]pyrrolo[2,1-c][1,4]benzodiazepin-3-carboxylat vom Schmelzpunkt 213–214°.

e) Man rührt eine Mischung aus 4,0 g (7,3 mMol) tert.-Butyl (S)-7,8-dichlor-11,12,13,13a-tetrahydro-9-oxo-9H-imidazo[1,5-a]pyrrolo[2,1-c][1,4]benzodiazepin-1-carboxylat, 10 ml (126 mMol) Hydroxymethyl-cyclopropan und 1 g (3 mMol) Tetraäthyl-orthotitanat über Nacht bei 125°, dampft das Reaktionsgemisch dann ein und nimmt den Rückstand in Chloroform auf. Man rührt die Lösung während einer halben Stunde mit 30 ml einer gesättigten Kaliumfluoridlösung und filtriert das Gemisch durch Kieselgur. Die organische Phase wird abgetrennt, mit Wasser gewaschen, über Magnesiumsulfat getrocknet und eingedampft. Durch Kristallisieren aus Essigester erhält man Cyclopropylmethyl (S)-7,8-dichlor-11,12,13,13a-tetrahydro-9-oxo-9H-imidazo[1,5-a]pyrrolo[2,1-c][1,4]benzodiazepin-1-carboxylat vom Schmelzpunkt 185–186°.

Beispiel 36

Man erhitzt 6,92 g (20 mMol) Äthyl (S)-7-chlor-11,12,13,13a-tetrahydro-9-oxo-9H-imidazo[1,5-a]pyrrolo[2,1-c][1,4]benzodiazepin-1-carboxylat, 20 g Hydroxymethyl-cyclopropan und 1 g Tetraäthyl-orthotitanat während 16 Stunden auf 130°, dampft im Vakuum ein, nimmt den Rückstand in Chloroform auf und giesst diese Lösung auf etwa 20proz. Salzsäure. Man rührt während 20 Minuten, trennt die Chloroformphase ab, wäscht sie nacheinander mit 1N-Salzsäure und gesättigter Natriumbicarbonatlösung, trocknet über Magnesiumsulfat und dampft ein. Durch Kristallisieren aus Essigester erhält man Cyclopropylmethyl (S)-7-chlor-11,12,13,13a-tetrahydro-9-oxo-9H-imidazo[1,5-a]pyrrolo[2,1-c][1,4]benzodiazepin-1-carboxylat vom Schmelzpunkt 146–148°.

Beispiel 37

Man rührt eine Mischung von 17,29 g (50 mMol) Äthyl (S)-8-chlor-11,12,13,13a-tetrahydro-9-oxo-9H-imidazo[1,5-a]pyrrolo[2,1-c][1,4]benzodiazepin-1-carboxylat, 1,0 g (15,4 mMol) Kaliumcyanid und 50 ml 3-Methyl-cyclohexanol (Gemisch der cis- und trans-Isomeren) während etwa 25 Stunden bei 140°, wobei man nach 5 und 16 Stunden je 20 ml Lösungsmittel abdestilliert und erneut 25 ml 3-Methyl-cyclohexanol dazugibt. Anschliessend dampft man im Vakuum ein, nimmt den Rückstand in 300 ml Chloroform auf, wäscht die Chloroformlösung einmal mit Wasser, trocknet über Magnesiumsulfat und dampft ein. Den Rückstand chromatographiert man unter Eluieren mit Essigester/n-Hexan (1:1) an Kieselgel und kristallisiert anschliessend die beiden erhaltenen Substanzen aus Essigester/n-Hexan um.

Man erhält (rac)-trans-3-Methylcyclohexyl (S)-8-chlor-11,12,13,13a-tetrahydro-9-oxo-9H-imidazo[1,5-a]pyrrolo[2,1-c][1,4]benzodiazepin-1-carboxylat als (1:1)-Gemisch der beiden Diastereomeren vom Schmelzpunkt 130–142° und mit dem grösseren Rf-Wert und (rac)-cis-3-Methylcyclohexyl (S)-8-chlor-11,12,13,13a-tetrahydro-9-oxo-9H-imidazo[1,5-a]pyrrolo[2,1-c][1,4]benzodiazepin-1-carboxylat als (1:1)-Gemisch der beiden Diastereomeren vom Schmelzpunkt 142–160° und mit dem kleineren Rf-Wert.

Beispiel 38

Man rührt eine Mischung aus 3,45 g (10 mMol) Äthyl (S)-8-chlor-11,12,13,13a-tetrahydro-9-oxo-9H-imidazo[1,5-a]pyrrolo[2,1-c][1,4]benzodiazepin-1-carboxylat, 0,8 g (3 mMol) Tetraäthyl-orthotitanat und 10 g (102 mMol) 2-Cyclohexen-1-ol über Nacht bei 120°, dampft zur Trockene ein und nimmt den Rückstand in Chloroform auf. Man rührt diese Lösung mit ca. 30 ml gesättigter Kaliumfluoridlösung, filtriert durch Kieselgur, wäscht die organische Phase nach dem Abtrennen mit Wasser, trocknet über Magnesiumsulfat und dampft ein. Durch Chromatographieren an Kieselgel unter Eluieren mit Essigester und anschliessendes Umkristallisieren aus Essigester und Hexan erhält man (R,S)-2-Cyclohexen-1-yl (S)-8-chlor-11,12,13,13a-tetrahydro-9-oxo-9H-imidazo[1,5-a]pyrrolo[2,1-c][1,4]benzodiazepin-1-carboxylat vom Schmelzpunkt 179–180°.

Beispiel 39

Man erhitzt eine Mischung aus 10,35 g (30 mMol) Äthyl (S)-8-chlor-11,12,13,13a-tetrahydro-9-oxo-9H-imidazo[1,5-a]pyrrolo[2,1-c][1,4]benzodiazepin-1-carboxylat, 50 g Cycloheptanol und 2 g Tetraäthyl-orthotitanat während 8 stunden auf 130°, dampft im Vakuum ein, nimmt den Rückstand in Chloroform auf und giesst diese Lösung auf etwa 20proz. Salzsäure. Man rührt während 15 Minuten, trennt die Chloroformphase ab, wäscht diese nacheinander mit 2N-Salzsäure und gesättigter Natriumbicarbonatlösung, trocknet sie über Magnesiumsulfat und dampft ein. Durch Kristallisieren aus Essigester/Hexan erhält man Cycloheptyl (S)-8-chlor-11,12,13,13a-tetrahydro-9-oxo-9H-imidazo[1,5-a]pyrrolo[2,1-c][1,4]benzodiazepin-1-carboxylat vom Schmelzpunkt 179–180°.

Beispiel 40

Man erhitzt eine Mischung aus 10,35 g (0,03 mMol) Äthyl (S)-8-chlor-11,12,13,13a-tetrahydro-9-oxo-9H-imidazo[1,5-a]pyrrolo[2,1-c][1,4]benzodiazepin-1-carboxylat, 50 g Cyclooctanol und 2 g Tetraäthyl-orthotitanat während 8 Stunden auf 130°, wobei man zweimal je etwas Lösungsmittel im Vakuum abdestilliert. Anschliessend dampft man im Vakuum ein, nimmt den Rückstand in Chloroform auf und giesst diese Lösung auf etwa 20proz. Salzsäure. Man rührt während 20 Minuten, trennt die Chloroformphase ab, wäscht diese nacheinander mit 1N-Salzsäure und gesättigter Natriumbicarbonatlösung, trocknet sie über Magnesiumsulfat und dampft ein. Durch Chromatographieren an einer Kieselgelsäule unter Eluieren mit Essigester und anschliessendes Kristallisieren aus Essigester/Hexan erhält man Cyclooctyl (S)-8-chlor-11,12,13,13a-tetrahydro-9-oxo-9H-imidazo[1,5-a]pyrrolo[2,1-c][1,4]benzodiazepin-1-carboxylat vom Schmelzpunkt 183–184°.

Beispiel 41

Man erhitzt eine Mischung aus 6,93 g (0,02 Mol) Äthyl (S)-8-chlor-11,12,13,13a-tetrahydro-9-oxo-9H-imidazo[1,5-a]pyrrolo[2,1-c][1,4]benzodiazepin-1-carboxylat, 1,4 g Tetraäthyl-orthotitanat und 24 g 2-Cyclohexyl-äthanol während 2,5 Stunden auf 140°, dampft im Vakuum ein, nimmt den Rückstand in Chloroform auf und giesst diese Lösung auf etwa 20proz. Salzsäure. Man rührt während 15 Minuten, trennt die Chloroformphase ab, wäscht nacheinander mit 2N-Salzsäure und gesättigter Natriumbicarbonatlösung, trocknet über Magnesiumsulfat und dampft ein. Nach Chromatographieren an einer Kieselgelsäule unter Eluieren mit Essigester und anschliessendem Kristallisieren aus Essigester/Hexan erhält man 2-Cyclohexyläthyl (S)-8-chlor-11,12,13,13a-tetrahydro-9-oxo-9H-imidazo[1,5-a]pyrrolo[2,1-c][1,4]benzodiazepin-1-carboxylat vom Schmelzpunkt 131–132°.

Beispiel 42

Man rührt eine Mischung aus 8,72 g (27 mMol) Äthyl 7-chlor-5,6-dihydro-5-methyl-6-oxo-4H-imidazo[1,5-a][1,4]benzodiazepin-3-carboxylat, 2 g (9 mMol) Tetraäthyl-orthotitanat und 27 g (270 mMol) Cyclohexanol über Nacht bei 125°, dampft zur Trockene ein, nimmt den Rückstand in Chloroform auf, wäscht nacheinander mit 40 ml 5N-Salzsäure und gesättigter Natriumbicarbonatlösung, trocknet über Magnesiumsulfat und dampft ein. Durch Umkristallisieren aus Alkohol und Äther erhält man Cyclohexyl 7-chlor-5,6-dihydro-5-methyl-6-oxo-4H-imidazo[1,5-a][1,4]benzodiazepin-3-carboxylat vom Schmelzpunkt 208–209°.

Beispiel 43

Man rührt eine Mischung aus 3,45 g (10 mMol) Äthyl (S)-8-chlor-11,12,13,13a-tetrahydro-9-oxo-9H-imidazo[1,5-a]pyrrolo[2,1-c][1,4]benzodiazepin-1-carboxylat, 100 mg pulverisiertem Kaliumcyanid und 14,20 g (100 mMol) 2-Chlorbenzylalkohol während 90 Stunden bei 130°, verdünnt das Reaktionsgemisch mit ca. 30 ml Methylenchlorid und chromatographiert unter Eluieren mit Essigester an ca. 300 g Kieselgel. Durch Umkristallisieren aus Essigester erhält man o-Chlorbenzyl (S)-8-chlor-11,12,13,13a-tetrahydro-9-oxo-9H-imidazo[1,5-a]pyrrolo[2,1-c][1,4]benzodiazepin-1-carboxylat vom Schmelzpunkt 192–193°.

Beispiel 44

Man rührt eine Mischung aus 3,45 g (10 mMol) Äthyl (S)-8-chlor-11,12,13,13a-tetrahydro-9-oxo-9H-imidazo[1,5-a]pyrrolo[2,1-c][1,4]benzodiazepin-1-carboxylat, 100 mg pulverisiertem Kaliumcyanid und 14,2 g 3-Chlorbenzylalkohol während 48 Stunden bei 130°, verdünnt das Reaktionsgemisch mit ca. 20 ml Methylenchlorid und chromatographiert unter Eluieren mit Essigester an ca. 300 g Kieselgel. Durch Kristallisieren aus Essigester und Hexan erhält man m-Chlorbenzyl (S)-8-chlor-11,12,13,13a-tetrahydro-9-oxo-9H-imidazo[1,5-a]pyrrolo[2,1-c][1,4]benzodiazepin-1-carboxylat vom Schmelzpunkt 114–116°.

Beispiel 45

Man rührt eine Mischung aus 3,45 g (10 mMol) Äthyl (S)-8-chlor-11,12,13,13a-tetrahydro-9-oxo-9H-imidazo[1,5-a]pyrrolo[2,1-c][1,4]benzodiazepin-1-carboxylat, 100 mg pulverisiertem Kaliumcyanid und 9,90 g 4-Chlorbenzylalkohol während 48 Stunden bei 130°, verdünnt das Reaktionsgemisch mit ca. 20 ml Methylenchlorid und chromatographiert unter Eluieren mit Essigester an ca. 300 g Kieselgel. Durch Umkristallisieren aus Essigester erhält man p-Chlorbenzyl (S)-8-chlor-11,12,13,13a-tetrahydro-9-oxo-9H-imidazo[1,5-a]pyrrolo[2,1-c][1,4]benzodiazepin-1-carboxylat vom Schmelzpunkt 183–184°.

Beispiel 46

Man rührt eine Mischung aus 3,45 g (10 mMol) Äthyl (S)-8-chlor-11,12,13,13a-tetrahydro-9-oxo-9H-imidazo[1,5-a]pyrrolo[2,1-c][1,4]benzodiazepin-1-carboxylat, 100 mg (1,5 mMol) Kaliumcyanid und 20 ml 2-Methoxybenzylalkohol während 36 Stunden bei 110°, wobei man von Zeit zu Zeit das entstandene Äthanol im Vakuum entfernt. Anschliessend destilliert man das Lösungsmittel im Hochvakuum ab und chromatographiert den Rückstand unter Eluieren mit Essigester an Kieselgel. Durch Umkristallisieren aus Essigester/n-Hexan erhält man o-Methoxybenzyl (S)-8-chlor-11,12,13,13a-tetrahydro-9-oxo-9H-imidazo[1,5-a]pyrrolo[2,1-c][1,4]benzodiazepin-1-carboxylat vom Schmelzpunkt 178–179°.

Beispiel 47

Man rührt eine Mischung aus 3,45 g (10 mMol) Äthyl (S)-8-chlor-11,12,13,13a-tetrahydro-9-oxo-9H-imidazo[1,5-a]pyrrolo[2,1-c][1,4]benzodiazepin-1-carboxylat, 100 mg (1,5 mMol) Kaliumcyanid und 20 ml 3-Methoxybenzylalkohol während 36 Stunden bei 110°, wobei man von Zeit zu Zeit das entstandene Äthanol im Vakuum entfernt.

Anschliessend destilliert man das Lösungsmittel im Hochvakuum ab, nimmt den Rückstand in Chloroform auf, wäscht die Chloroformlösung dreimal mit Wasser, trocknet über Magnesiumsulfat und dampft ein. Das Rohprodukt chromatographiert man unter Eluieren mit Essigester an Kieselgel. Durch Kristallisieren aus Essigester/n-Hexan erhält man m-Methoxybenzyl (S)-8-chlor-11,12,13,13a-tetrahydro-9-oxo-9H-imidazo[1,5-a] pyrrolo[2,1-c][1,4]benzodiazepin-1-carboxylat vom Schmelzpunkt 131–133°.

Beispiel 48

Man rührt eine Mischung aus 3,45 g (10 mMol) Äthyl (S)-8-chlor-11,12,13,13a-tetrahydro-9-oxo-9H-imidazo[1,5-a]pyrrolo[2,1-c][1,4]benzodiazepin-1-carboxylat, 100 mg (1,5 mMol) Kaliumcyanid und 20 ml 4-Methoxybenzylalkohol während 3,5 Tagen bei 110°, wobei man von Zeit zu Zeit das entstandene Äthanol im Vakuum entfernt. Anschliessend destilliert man das Lösungsmittel im Hochvakuum ab, nimmt den Rückstand in Methylenchlorid auf, wäscht die Methylenchloridlösung dreimal mit Wasser, trocknet über Magnesiumsulfat und dampft ein. Das Rohprodukt chromatographiert man unter Eluieren mit Essigester an Kieselgel. Durch Umkristallisieren aus Essigester erhält man p-Methoxybenzyl (S)-8-chlor-11,12,13,13a-tetrahydro-9-oxo-9H-imidazo[1,5-a] pyrrolo[2,1-c][1,4]benzodiazepin-1-carboxylat vom Schmelzpunkt 185–186°.

Beispiel 49

Man rührt eine Mischung aus 3,59 g (10 mMol) tert.-Butyl (S)-8-chlor12,12a-dihydro-9-oxo-9H,11H-azeto[2,1-c]imidazo[1,5-a][1,4]benzodiazepin-1-carboxylat, 1,5 g (6 mMol) Tetraäthyl-orthotitanat und 30 ml Benzylalkohol während 6 Stunden bei 130°, wobei man zweimal je etwas Lösungsmittel im Vakuum abdestilliert. Anschliessend dampft man im Hochvakuum ein, nimmt den Rückstand in 40 ml Chloroform auf, versetzt mit 40 ml konz. Salzsäure/Wasser (1:1) und rührt, bis beide Phasen klar sind. Man wäscht die Chloroformlösung nacheinander mit 20 ml 1N-Salzsäure und gesättigter Natriumbicarbonatlösung, trocknet über Magnesiumsulfat und dampft zur Trockene ein. Den Rückstand kristallisiert man aus Essigester um und erhält Benzyl (S)-8-chlor-12,12a-dihydro-9-oxo-9H,11H-azeto [2,1-c]imidazo[1,5-c][1,4]benzodiazepin-1-carboxylat vom Schmelzpunkt 180–182°.

Durch Chromatographieren der Mutterlauge an Kieselgel unter Eluieren mit Essigester und anschliessendes Umkristallisieren aus Essigester erhält man eine zweite Portion des gewünschten Stoffes von gleicher Reinheit.

Beispiel 50

a) Man erhitzt 10,6 g (50,9 mMol) 6-Nitro-isatosäureanhydrid und 6,1 g (50,9 mMol) L-Prolin in 70 ml Dimethylsulfoxid während 45 Minuten auf 90°, dampft anschliessend im Hochvakuum ein und erhitzt den erhaltenen Rückstand während 4 Stunden auf 140°. Man nimmt das kristalline Rohprodukt in 100 ml siedendem Äthanol auf, lässt über Nacht in der Kälte stehen, nutscht das erhaltene Material unter Waschen mit kaltem Äthanol ab und trocknet es bis zur Gewichtskonstanz. Man erhält (S)-1,2,3,11a-Tetrahydro-6-nitro-5H-pyrrolo[2,1-c][1,4]benzodiazepin-5,11(10H)-dion mit einem Zersetzungspunkt von 235–237°.

b) 57,3 g (219,3 mMol) (S)-1,2,3-Tetrahydro-6-nitro-5H-pyrrolo[2,1-c][1,4]benzodiazepin-5,11 (10H)-dion werden in 1,2 l Methanol mit 3 g 10proz. Palladiumkohle bei Raumtemperatur und Normaldruck hydriert. Nach beendeter Wasserstoffaufnahme wird zum Sieden erwärmt und vom Katalysator abgenutscht, wonach man das Filtrat eindampft. Durch Umkristallisieren aus Methanol erhält man (S)-6-Amino-1,2,3,11a-tetrahydro-5H-pyrrolo[2,1-c][1,4]benzodiazepin-5,11(10H)-dion vom Schmelzpunkt 246–248°.

c) Man versetzt eine Suspension von 26,8 g (115,9 mMol) (S)-6-Amino-1,2,3,11a-tetrahydro-5H-pyrrolo[2,1-c][1,4]benzodiazepin-5,11(10H)-dion in 80 ml trockenem Dimethylformamid bei −20 bis −10° unter Rühren mit 5,56 g (127,4 mMol) Natriumhydrid (55proz. Öldispersion), rührt noch während 1 Stunde in obigem Temperaturbereich und tropft dann bei −45 19 ml (127,4 mMol) Diäthylchlorphosphat dazu.

Inzwischen löst man 16,5 g (127,4 mMol) Kalium-tert.-butylatin23 mltrockenemDimethylformamid, kühlt in einem Aceton/Trockeneisbad ab, versetzt mit 18 g (127,4 mMol) Isocyanessigsäure-tert.-butylester und gibt die erhaltene Lösung bei −20° tropfenweise zum obigen Reaktionsgemisch. Man lässt auf 5° erwärmen, neutralisiert mit 7,3 ml Eisessig, giesst auf 500 ml Wasser und extrahiert dreimal mit Methylenchlorid. Man wäscht die Methylenchloridlösung einmal mit Wasser und einmal mit gesättigter Natriumchloridlösung, trocknet über Magnesiumsulfat und dampft ein. Das Rohprodukt wird aus Essigester/Diäthyläther kristallisiert. Durch Umkristallisieren aus Essigester/n-Hexan erhält man tert.-Butyl (S)-8-amino-11,12,13,13a-tetrahydro-9-oxo-9H-imidazo[1,5-a]pyrrolo[2,1-c][1,4]benzodiazepin-1-carboxylat mit einem Zersetzungspunkt von 223–224°.

d) Man rührt eine Mischung aus 10,0 g (28,2 mMol) tert.-Butyl (S)-8-amino-11,12,13,13a-tetrahydro-9-oxo-9H-imidazo[1,5-a]pyrrolo [2,1-c][1,4]benzodiazepin-1-carboxylat, 2,6 g (11,1 mMol) Tetraäthyl-orthotitanat und 60 ml Cyclohexanol während 24 Stunden bei 130°, wobei man dreimal je etwas Lösungsmittel im Vakuum abdestilliert. Anschliessend dampft man im Vakuum ein, nimmt den Rückstand in 60 ml Chloroform auf, versetzt mit Wasser, rührt noch während 1 Stunde, filtriert über Kieselgur und trennt dann die organische Phase ab. Man trocknet diese über Magnesiumsulfat und dampft ein. Den Rückstand chromatographiert man unter Eluieren mit Essigester an Kieselgel und kristallisiert anschliessend aus Essigester um. Man erhält Cyclohexyl (S)-8-amino-11,12,13,13a-tetrahydro-9-oxo-9H-imidazo[1,5-a]pyrrolo[2,1-c][1,4]benzodiazepin-1-carboxylat vom Schmelzpunkt 211–212°.

Beispiel 51

Man rührt eine Mischung aus 3,0 g (8,2 mMol) (S)-1-[(8-chlor-11,12,13,13a-tetrahydro-9-oxo-9H-imidazo[1,5-a]pyrrolo[2,1-c][1,4]benzodiazepin-1-yl)carbonyl]imidazol, 1,59 g (11,5 mMol) pulverisiertem Kaliumcarbonat, 1,43 g (11,5 mMol) Hydrochinon-monomethyläther und 20 ml trockenem Dimethylformamid während 16 Stunden bei Raumtemperatur, giesst dann auf 60 ml Wasser und extrahiert dreimal mit Methylenchlorid. Man wäscht die organischen Auszüge zweimal mit gesättigter Natriumchloridlösung, trocknet über Magnesiumsulfat und dampft ein. Nach Chromatographieren unter Eluieren mit Essigester an Kieselgel und anschliessendem Umkristallisieren aus Essigester erhält man p-Methoxyphenyl (S)-8-chlor-11,12,13,13a-tetrahydro-9-oxo-9H-imidazo[1,5-a]pyrrolo[2,1-c][1,4]benzodiazepin-1-carboxylat vom Schmelzpunkt 210–211°.

Beispiel 52

Man rührt eine Mischung aus 3,0 g (8,2 mMol) 1-‹[(S)-8-Chlor-11,12,13,13a-tetrahydro-9-oxo-9H-imidazo[1,5-a]pyrrolo[2,1-c][1,4]benzodiazepin-1-yl]carbonyl›imidazol, 1,59 g (11,5 mMol) pulverisiertem Kaliumcarbonat, 1,48 g (11,5 mMol) 4-Chlorphenol und 20 ml trockenem Dimethylformamid während 21 Stunden bei Raumtemperatur, giesst dann auf 60 ml Wasser und extrahiert viermal mit Methylenchlorid. Man wäscht die organischen Auszüge zweimal mit gesättigter Natriumchloridlösung, trocknet über Magnesiumsulfat und dampft ein. Nach Chromatographieren an Kieselgel unter Eluieren mit Essigester und anschliessendem Umkristallisieren aus Essigester erhält man p-Chlorphenyl (S)-8-chlor-11,12,13,13a-tetrahydro-9-oxo-9H-imidazo [1,5-a] pyrrolo[2,1-c][1,4]benzodiazepin-1-carboxylat vom Schmelzpunkt 241–242°.

Beispiel 53

Man rührt eine Mischung aus 3,0 g (8,2 mMol) 1-‹[(S)-8-Chlor-11,12,13,13a-tetrahydro-9-oxo-9H-imidazo[1,5-a]pyrrolo[2,1-c][1,4]benzodiazepin-1-yl]carbonyl›imidazol, 1,59 g (11,5 mMol) pulverisiertem Kaliumcarbonat, 1,3 ml (11,5 mMol) 2-Chlorphenol und 20 ml trockenem Dimethylformamid während 5 Tagen bei Raumtemperatur, giesst dann auf 60 ml Wasser und extrahiert dreimal mit Methylenchlorid. Man wäscht die organischen Auszüge zweimal mit gesättigter Natriumchloridlösung, trocknet über Magnesiumsulfat und dampft ein. Das Rohprodukt chromatographiert man an Kieselgel, wobei man nacheinander mit Essigester/n-Hexan (1:1), Essigester/n-Hexan (3:2) und Essigester/n-Hexan (7:3) eluiert. Nach Umkristallisieren aus Essigester erhält man o-Chlorphenyl (S)-8-chlor-11,12,13,13a-tetrahydro-9-oxo-9H-imidazo[1,5-a]pyrrolo[2,1-c][1,4]benzodiazepin-1-carboxylat vom Schmelzpunkt 214–215°.

Beispiel 54

Man rührt eine Mischung aus 3,0 g (8,2 mMol) 1-‹[(S)-8-Chlor-11,12,13,13a-tetrahydro-9-oxo-9H-imidazo[1,5-a]pyrrolo[2,1-c][1,4]benzodiazepin-1-yl]carbonyl›imidazol, 1,59 g (11,5 mMol) pulverisiertem Kaliumcarbonat, 1,48 g (11,5 mMol) 3-Chlorphenol und 20 ml trockenem Dimethylformamid während 5 Tagen bei Raumtemperatur, giesst dann auf 60 ml Wasser und extrahiert viermal mit Methylenchlorid. Man wäscht die organischen Auszüge einmal mit gesättigter Natriumchloridlösung, trocknet über Magnesiumsulfat und dampft ein. Den erhaltenen Rückstand chromatographiert man unter Eluieren mit Essigester an Kieselgel und kristallisiert dann aus Äthanol. Man erhält m-Chlorphenyl (S)-8-chlor-11,12,13,13a-tetrahydro-9-oxo-9H-imidazo[1,5-a]pyrrolo [2,1-c][1,4]benzodiazepin-1-carboxylat vom Schmelzpunkt 194–195°.

Beispiel 55

Man rührt eine Mischung aus 3,0 g (8,2 mMol) 1-‹(S)-8-Chlor-11,12,13,13a-tetrahydro-9-oxo-9H-imidazo[1,5-a]pyrrolo[2,1-c][1,4]benzodiazepin-1-yl]carbonyl›imidazol, 1,59 g (11,4 mMol) pulverisiertem Kaliumcarbonat, 1,48 g (11,5 mMol) Guajacol und 20 ml trockenem Dimethylformamid während 23 Stunden bei Raumtemperatur, giesst dann auf 60 ml Wasser und extrahiert dreimal mit Methylenchlorid. Man wäscht die organischen Auszüge zweimal mit gesättigter Natriumchloridlösung, trocknet über Magnesiumsulfat und dampft ein. Das Rohprodukt chromatographiert man unter Eluieren mit Essigester an Kieselgel. Nach Umkristallisieren aus Essigester erhält man o-Methoxyphenyl (S)-8-chlor-11,12,13,13a-tetrahydro-9-oxo-9H-imidazo[1,5-a]pyrrolo[2,1-c][1,4]benzodiazepin-1-carboxylat vom Schmelzpunkt 205–206°.

Beispiel 56

Man rührt eine Mischung aus 3,0 g (8,2 mMol) 1-‹[(S)-8-Chlor-11,12,13,13a-tetrahydro-9-oxo-9H-imidazo[1,5-a]pyrrolo[2,1-c][1,4]benzodiazepin-1-yl]carbonyl›imidazol, 1,59 g (11,5 mMol) pulverisiertem Kaliumcarbonat, 1,43 g (11,5 mMol) Resorcin-monomethyläther und 20 ml trockenem Dimethylformamid während 2 Tagen bei Raumtemperatur, giesst dann auf 60 ml Wasser und extrahiert dreimal mit Methylenchlorid. Man wäscht die organischen Auszüge einmal mit gesättigter Natriumchloridlösung, trocknet über Magnesiumsulfat und dampft ein. Den erhaltenen Rückstand chromatographiert man unter Eluieren mit Essigester/n-Hexan (3:1) an Kieselgel und kristallisiert anschliessend aus Essigester um. Man erhält m-Methoxyphenyl (S)-8-chlor-11,12,13,13a-tetrahydro-9-oxo-9H-imidazo[1,5-a]pyrrolo[2,1-c][1,4]benzodiazepin-1-carboxylat vom Schmelzpunkt 184–185°.

Beispiel 57

Man rührt eine Mischung aus 3,45 g (10 mMol) Äthyl (S)-8-chlor-11,12,13,13a-tetrahydro-9-oxo-9H-imidazo[1,5-a]pyrrolo[2,1-c][1,4]benzodiazepin-1-carboxylat, 100 mg pulverisiertem Kaliumcyanid und 11,4 g (100 mMol) cis-4-Methylcyclohexanol über Nacht bei 130°, entfernt das überschüssige cis-4-Methylhexanol im Vakuum und chromatographiert den Rückstand an Kieselgel. Durch Kristallisieren aus Essigester und Hexan erhält man cis-4-Methylcyclohexyl (S)-8-chlor-11,12,13,13a-tetrahydro-9-oxo-9H-imidazo[1,5-a] pyrrolo[2,1-c][1,4]benzodiazepin-1-carboxylat vom Schmelzpunkt 176–177°.

Beispiel 58

Man rührt eine Mischung aus 3,45 g (10 mMol) Äthyl (S)-8-chlor-11,12,13,13a-tetrahydro-9-oxo-9H-imidazo[1,5-a]pyrrolo[2,1-c][1,4]benzodiazepin-1-carboxylat, 11,40 g (100 mMol) cis-2-Methylcyclohexanol und 0,8 g (3,5 mMol) Tetraäthylorthotitanat während 48 Stunden bei 110°, dampft anschliessend zur Trockene ein, nimmt den Rückstand in Chloroform auf, rührt eine halbe Stunde mit 40 ml einer gesättigten Kaliumfluoridlösung und filtriert die entstandene Emulsion durch Kieselgur. Die Chloroform-Phase wird abgetrennt, mit Wasser gewaschen, über Magnesiumsulfat getrocknet und eingeengt. Durch Chromatographieren an einer Kieselgelsäule und anschliessendes Umkristallisieren aus Essigester und Hexan erhält man cis-2-Methylcyclohexyl (S)-8-chlor-11,12,13,13a-tetrahydro-9-oxo-9H-imidazo[1,5-a]pyrrolo[2,1-c][1,4]benzodiazepin-1-carboxylat als (1:1)-Gemisch der beiden Diastereoisomeren vom Schmelzpunkt 178–180°.

Beispiel 59

a) Man erhitzt eine Mischung aus 23,7 g (0,131 Mol) 5-Fluorisatosäureanhydrid, 13,23 g (0,131 Mol) L-Azetidin-2-carbonsäure und 150 ml Dimethylsulfoxid während 3 Stunden auf 90°, dampft anschliessend im Hochvakuum ein und erhitzt den Rückstand während 17 Stunden im Hochvakuum. Durch Umkristallisieren aus Methanol erhält man (S)-6-Fluor-1,10a-dihydroazeto[2,1-c][1,4]benzodiazepin-4,10(2H,9H)-dion vom Schmelzpunkt 216–217°.

b) Man versetzt eine Suspension von 5,5 g (125 mMol) Natriumhydrid (55proz. Öldispersion) in 100 ml trockenem Dimethylformamid bei −20 bis −10° unter Rühren mit 25 g (113,5 mMol) (S)-6-Fluor-1,10a-dihydroazeto[2,1-c][1,4]benzodiazepin-4,10(2H,9H)-dion, rührt noch während 45 Minuten bei obiger Temperatur und tropft anschliessend bei −40° 18,1 ml (125 mMol) Diäthylchlorphosphat dazu.

Inzwischen löst man 14,1 g (125 mMol) Kalium-tert.-butylat in 40 ml trockenem Dimethylformamid, kühlt in einem Aceton/Trockeneisbad ab, versetzt mit 20,9 g (125 mMol) Isocyanessigsäure-cyclohexylester und tropft die erhaltene Lösung bei −20 bis −15° zum obigen Reaktionsgemisch. Man lässt auf 20° erwärmen, neutralisiert dann mit 7,1 ml Eisessig, giesst auf 600 ml Eiswasser und extrahiert dreimal mit Methylenchlorid. Die organischen Auszüge werden nacheinander einmal mit Wasser und einmal mit gesättigter Natriumchloridlösung gewaschen, über Magnesiumsulfat getrocknet und eingedampft. Man chromatographiert den Rückstand unter Eluieren mit Essigester/n-Hexan (3:2) an Kieselgel. Durch Umkristallisieren aus Essigester erhält man Cyclohexyl (S)-7-fluor-12,12a-diyhdro-9-oxo-9H, 11H-azeto[2,1-c]imidazo[1,5-a][1,4]benzodiazepin-1-carboxylat vom Schmelzpunkt 231–232°.

Beispiel 60

Man rührt eine Mischung aus 3,45 g (10 mMol) Äthyl (S)-8-chlor-11,12,13,13a-tetrahydro-9-oxo-9H-imidazo[1,5-a]pyrrolo[2,1-c][1,4]benzodiazepin-1-carboxylat, 1 g (4,4 mMol) Tetraäthyl-orthotitanat und 11,4 g (100 mMol) trans-4-Methylcyclohexanol über Nacht bei 125°, dampft zur Trockene ein und nimmt den Rückstand in Methylenchlorid auf. Man wäscht nacheinander mit 5N-Salzsäure und mit gesättigter Natriumbicarbonatlösung, trocknet über Magnesiumsulfat und dampft ein. Durch Umkristallisieren aus Essigester und Hexan erhält man trans-4-Methylcyclohexyl (S)-8-chlor-11,12,13,13a-tetrahydro-9-oxo-9H-imidazo[1,5-a]pyrrolo[2,1-c][1,4]benzodiazepin-1-carboxylat vom Schmelzpunkt 220–221°.

Beispiel 61

Man rührt eine Mischung aus 3,0 g (7,2 mMol) tert.-Butyl (S)-8-brom-11,12,13,13a-tetrahydro-9-oxo-9H-imidazo[1,5-a]pyrrolo[2,1-c][1,4]benzodiazepin-1-carboxylat, 0,54 g (2,4 mMol) Tetraäthyl-orthotitanat und 6,17 g (72 mMol) Hydroxymethyl-cyclobutan während 2,5 Stunden bei 120°, dampft zur Trockene ein und nimmt den Rückstand in Methylenchlorid auf. Man wäscht nacheinander mit 5N-Salzsäure und gesättigter Natriumbicarbonatlösung, trocknet über Magnesiumsulfat und dampft ein. Durch Umkristallisieren aus Essigester erhält man Cyclobutylmethyl (S)-8-brom-11,12,13,13a-tetrahydro-9-oxo-9H-imidazo[1,5-a]pyrrolo[2,1-c][1,4]benzodiazepin-3-carboxylat vom Schmelzpunkt 170–172°.

Beispiel 62

Man rührt eine Mischung aus 3,45 g (10 mMol) Äthyl (S)-8-chlor-11,12,13,13a-tetrahydro-9-oxo-9H-imidazo[1,5-a]pyrrolo[2,1-c][1,4]benzodiazepin-1-carboxylat, 0,8 g (3 mMol) Tetraäthyl-orthotitanat und 8,61 g (100 mMol) 1-Cyclopropyl-äthanol über Nacht bei 115°, dampft anschliessend zur Trockene ein, nimmt den Rückstand in Chloroform auf und chromatographiert an Kieselgel unter Eluieren mit Essigester. Durch Umkristallisieren aus Essigester und Hexan erhält man (R,S)-1-Cyclopropyläthyl (S)-8-chlor-11,12,13,13a-tetrahydro-9-oxo-9H-imidazo[1,5-a]pyrrolo[2,1-c][1,4]benzodiazepin-1-carboxylat vom Schmelzpunkt 157–158°.

Beispiel 63

Man rührt eine Mischung aus 4,32 g (13,9 mMol) Äthyl (S)-11,12,13,13a-tetrahydro-9-oxo-9H-imidazo[1,5-a]pyrrolo[2,1-c][1,4]benzodiazepin-1-carboxylat, 1,52 g (6,7 mMol) Tetraäthyl-orthotitanat und 30 g (300 mMol) Cyclohexanol über Nacht bei 120°, dampft anschliessend zur Trockene ein, nimmt den Rückstand in Methylenchlorid auf, wäscht die Lösung nacheinander mit 5N-Salzsäure und gesättigter Natriumbicarbonatlösung, trocknet sie über Magnesiumsulfat und dampft ein. Durch Umkristallisieren aus Essigester erhält man Cyclohexyl (S)-11,12,13,13a-tetrahydro-9-oxo-9H-imidazo[1,5-a]pyrrolo[2,1-c][1,4]benzodiazepin-1-carboxylat vom Schmelzpunkt 212–213°.

Beispiel 64

Man rührt eine Mischung aus 3,19 g (10 mMol) Äthyl 7-chlor-5,6-dihydro-5-methyl-6-oxo-4H-imidazo[1,5-a][1,4]benzodiazepin-3-carboxylat, 0,8 g (3 mMol) Tetraäthyl-orthotitanat und 7 g (81 mMol) Hydroxymethyl-cyclobutan während 5 Stunden bei 125°, dampft anschliessend zur Trokkene ein und nimmt den Rückstand in Methylenchlorid auf. Man wäscht die organische Lösung nacheinander mit 5N-Salzsäure und mit gesättigter Natriumbicarbonatlösung, trocknet über Magnesiumsulfat und dampft ein. Durch Umkristallisieren aus Essigester erhält man Cyclobutylmethyl 7-chlor-5,6-dihydro-5-methyl-6-oxo-4H-imidazo[1,5-a][1,4]benzodiazepin-3-carboxylat vom Schmelzpunkt 181–182°.

Beispiel 65

a) Man versetzt eine Suspension von 4,43 g (101,4 mMol) Natriumhydrid (55proz. Öldispersion) in 70 ml trockenem Dimethylformamid bei −20 bis −30° unter Rühren mit 20,3 g (92,2 mMol) (S)-6-Fluor-1,10a-dihydroazeto[2,1-c][1,4]benzodiazepin-4,10(2H,9H)-dion, rührt noch während 45 Minuten bei obiger Temperatur und tropft anschliessend bei −35 bis −40° 15,1 ml (101,4 mMol) Diäthylchlorphosphat dazu.

Inzwischen löst man 11,76 g (101,4 mMol) Kalium-tert.-butylat in 30 ml trockenem Dimethylformamid, kühlt in einem Aceton/Trockeneisbad ab, gibt 14,3 g (101,4 mMol) Isocyanessigsäure-tert.-butylester tropfenweise dazu und tropft die erhaltene Lösung bei −20 bis −15° zum obigen Reaktionsgemisch. Man lässt auf 5° erwärmen, neutralisiert mit 5,8 ml Eisessig, giesst auf 300 ml Wasser und extrahiert viermal mit Methylenchlorid. Die organischen Auszüge werden zweimal mit gesättigter Natriumchloridlösung gewaschen, über Magnesiumsulfat getrocknet und eingedampft. Man chromatographiert den Rückstand unter Eluieren mit Essigester/n-Hexan (3:2) an Kieselgel. Nach Kristallisieren des erhaltenen Öls erhält man tert.-Butyl (S)-7-fluor-12,12a-dihydro-9-oxo-9H,11H-azeto[2,1-c]imidazo[1,5-a][1,4]benzodiazepin-1-carboxylat vom Schmelzpunkt 148–149°.

b) Man rührt eine Mischung aus 2,95 g (8,6 mMol) tert.-Butyl (S)-7-fluor-12,12a-dihydro-9-oxo-9H,11H-azeto[2,1-c]imidazo[1,5-a][1,4]benzodiazepin-1-carboxylat, 10 g Hydroxymethylcyclopropan und 1,6 g (6,8 mMol) Tetraäthyl-orthotitanat während 24 Stunden bei 120°, wobei man nach einer Stunde etwa 3 ml Lösungsmittel im Vakuum abdestilliert. Anschliessend dampft man in Vakuum ein, löst den Rückstand in 40 ml Chloroform, versetzt mit 30 ml Wasser und rührt während 1,25 Stunden. Man filtriert durch Kieselgur, wobei man mit Chloroform wäscht, trocknet die Chloroformphase über Magnesiumsulfat und dampft ein. Das Rohprodukt chromatographiert man unter Eluieren mit Essigester an Kieselgel. Nach Umkristallisieren aus Essigestern/n-Hexan erhält man Cyclopropylmethyl (S)-7-fluor-12,12a-dihydro-9-oxo-9H,11H-azeto[2,1-c]imidazo[1,5-a][1,4]benzodiazepin-1-carboxylat vom Schmelzpunkt 171–172°.

Beispiel 66

Man löst 1,84 g (7,4 mMol) Kupfersulfat.5$H_2O$ bei 50–60° in 7 ml Wasser, tropft nacheinander eine Lösung von 0,464 g (3,7 mMol) wasserfreiem Natriumsulfit in 3 ml Wasser und 0,54 g (11 mMol) Natriumcyanid in 2,5 ml Wasser dazu und rührt noch während 10 Minuten bei obiger Temperatur. Anschliessend kühlt man in einem Eisbad ab, nutscht den entstandenen Niederschlag unter Waschen mit Wasser ab und nimmt ihn in einer Lösung von 0,97 g (19,8 mMol) Natriumcyanid in 6 ml Wasser auf.

Inzwischen löst man 2,1 g (5,5 mMol) Cyclohexyl (S)-8-amino-11,12,13,13a-tetrahydro-9-oxo-9H-imidazo[1,5-a]pyrrolo[2,1-c][1,4]benzodiazepin-1-carboxylat in einem Gemisch aus 2,8 ml konzentrierter Salzsäure und 5,6 ml Wasser und tropft bei 0 bis −3° eine Lösung von 0,39 g (5,6 mMol) Natriumnitrit in 2,5 ml Wasser dazu. Die erhaltene Diazoniumsalzlösung tropft man bei 0 bis −3° zur obigen Kupfer(I)cyanidlösung und rührt noch während 1,5 Stunden ohne Kühlung. Anschliessend stellt man mit 2N-Natronlauge alkalisch, versetzt mit etwa 40 ml Essigester, nutscht ab, wäscht die Essigesterphase nacheinander einmal mit 2N-Natronlauge, einmal mit Wasser und einmal mit gesättigter Natriumchloridlösung, trocknet über Magnesiumsulfat und dampft ein. Den Rückstand chromatographiert man unter Eluieren mit Chloroform, das 0,6% Methanol enthält, an Kieselgel. Nach Umkristallisieren aus Essigester erhält man Cyclohexyl (S)-8-cyano-11,12,13,13a-tetrahydro-9-oxo-9H-imidazo[1,5-a]pyrrolo[2,1-c][1,4]benzodiazepin-1-carboxylat vom Schmelzpunkt 246–247°.

Beispiel 67

Man löst 5,29 g (27,6 mMol) etwa 90proz. m-Chlorperbenzoesäure in 55 ml Methylenchlorid, kühlt auf 0° ab, versetzt die erhaltene Suspension portionenweise mit 3,5 g (9,2 mMol) Cyclohexyl (S)-8-amino-11,12,13,13a-tetrahydro-9-oxo-9H-imidazo[1,5-a]pyrrolo[2,1-c][1,4]benzodiazepin-1-carboxylat und rührt noch während 2 Stunden ohne Kühlung. Man giesst anschliessend auf etwa 70 ml Eiswasser, stellt mit gesättigter Natriumbicarbonatlösung alkalisch und trennt die Methylenchloridlösung ab. Man wäscht die Methylenchloridlösung nacheinander dreimal mit gesättigter Natriumbicarbonatlösung und zweimal mit gesättigter Natriumchloridlösung, trocknet über Magnesiumsulfat und dampft ein. Den

erhaltenen Rückstand chromatographiert man unter Eluierenmit Essigester an Kieselgel. Durch Umkristallisieren aus Essigester/n-Hexan erhält man Cyclohexyl (S)-11,12,13,13a-tetrahydro-8-nitro-9-oxo-9H-imidazo[1,5-a]pyrrolo[2,1-c][1,4] benzodiazepin-1-carboxylat mit einem Zersetzungspunkt von 204°.

Beispiel 68

Man rührt eine Mischung aus 6,22 g (20 mMol) Äthyl (S)-11,12,13,13a-tetrahydro-9-oxo-9H-imidazo[1,5-a]pyrrolo[2,1-c][1,4]benzodiazepin-1-carboxylat, 1,52 g (6,7 mMol) Tetraäthyl-orthotitanat und 10 g (194 mMol) Hydroxymethyl-cyclopropan über Nacht bei 115°, dampft zur Trockene ein, nimmt den Rückstand in Methylenchlorid auf und rührt während 0,5 Stunden mit etwa 50 ml Wasser. Man filtriert die erhaltene Emulsion durch Kieselgur, wäscht die organische Phase mit gesättigter Natriumbicarbonatlösung, trocknet über Magnesiumsulfat und dampft ein. Durch Umkristallisieren des Rückstandes aus Essigester erhält man Cyclopropylmethyl (S)-11,12,13,13a-tetrahydro-9-oxo-9H-imidazo[1,5-a]pyrrolo[2,1-c] [1,4]benzodiazepin-1-carboxylat vom Schmelzpunkt 189–190°.

Beispiel 69

Man rührt eine Mischung aus 9,4 g (26,1 mMol) tert.-Butyl (S)-8-chlor-12,12a-dihydro-9-oxo-9H,11H-azeto[2,1-c]imidazo[1,5-a][1,4]benzodiazepin-1-carboxylat, 30 g (416 mMol) Hydroxymethyl-cyclopropan und 1,4 g (6 mMol) Tetraäthylorthotitanat während 24 Stunden bei 120°, wobei man dreimal je etwas Lösungsmittel im Vakuum abdestilliert. Anschliessend dampft man im Vakuum ein, nimmt den Rückstand in 60 ml Chloroform auf, versetzt mit 40 ml Wasser und rührt während 1,5 Stunden. Dann filtriert man unter Waschen mit Chloroform durch Kieselgur, trocknet die Chloroformphase über Magnesiumsulfat und dampft sie ein. Den Rückstand chromatographiert man unter Eluieren von Essigester an Kieselgel. Nach Umkristallisieren aus Essigester erhält man Cyclopropylmethyl (S)-8-chlor-12,12a-dihydro-9-oxo-9H,11H-azeto[2,1-c]imidazo[1,5-a] [1,4]benzodiazepin-1-carboxylat vom Schmelzpunkt 193–194°.

Beispiel 70

Man rührt eine Mischung aus 5 g (16,5 mMol) Äthyl 8-fluor-5,6-dihydro-5-methyl-6-oxo-4H-imidazo[1,5-a][1,4]benzodiazepin-3-carboxylat, 1,8 g (8 mMol) Tetraäthyl-orthotitanat und 16,5 g (230 mMol) Hydroxymethyl-cyclopropan über Nacht bei 110°, dampft zur Trockene ein, nimmt den Rückstand in Methylenchlorid auf und rührt die erhaltene Lösung während 0,5 Stunden mit 50 ml einer gesättigten Kaliumfluoridlösung. Man filtriert die erhaltene Emulsion durch Kieselgur, wäscht die abgetrennte organische Phase mit Wasser, trocknet sie über Magnesiumsulfat und dampft ein. Durch Kristallisieren aus Essigester erhält man Cyclopropylmethyl 8-fluor-5,6-dihydro-5-methyl-6-oxo-4H-imidazo[1,5-a][1,4]benzodiazepin-3-carboxylat vom Schmelzpunkt 156–157°.

Beispiel 71

Man erhitzt eine Mischung aus 6,9 g (0,02 Mol) Äthyl (S)-8-chlor-11,12,13,13a-tetrahydro-9-oxo-9H-imidazo[1,5-a]pyrrolo[2,1-c][1,4]benzodiazepin-1-carboxylat, 100 ml Allylalkohol und 0,5 g Kaliumcyanid während 4 Stunden unter Rückfluss zum Sieden, engt dann auf die Hälfte ein, gibt 50 ml Allylalkohol dazu und erhitzt weitere 10 Stunden unter Rückfluss zum Sieden. Man dampft ein, löst den Rückstand in Chloroform, wäscht mit Wasser, trocknet die Chloroformphase über Magnesiumsulfat und dampft ein. Man chromatographiert den Rückstand an Kieselgel unter Eluieren mit Essigester und kristallisiert dann aus Essigester/Hexan. Man erhält Allyl (S)-8-chlor-11,12,13,13a-tetrahydro-9-oxo-9H-imidazo[1,5-a]pyrrolo[2,1-c][1,4]benzodiazepin-1-carboxylat vom Schmelzpunkt 122–123°.

Beispiel 72

a) Man erhitzt 11,55 g (0,05 Mol) 6-(Trifluormethyl)-isatosäureanhydrid und 5,75 g (0,05 Mol) L-Prolin in 100 ml Dimethylsulfoxid während 1 Stunde auf 70°, enternt das Lösungsmittel im Hochvakuum und erhitzt das erhaltene Öl während 15 Minuten auf 170°. Das Rohprodukt wird durch Chromatographieren an Kieselgel gereinigt, wobei man als Elutionsmittel Methylenchlorid und Gemische aus Methylenchlorid und Essigester (5%, 10%, 15%) verwendet. Nach Umkristallisieren des Rohproduktes aus Essigester/Diäthyläther erhält man reines (S)-1,2,3,11a-Tetrahydro-6-(trifluormethyl)-5H-pyrrolo[2,1-c] [1,4] benzodiazepin-5,11(10H)-dion vom Schmelzpunkt 176–178°.

b) Man versetzt eine Lösung von 9,15 g (32,2 mMol) (S)-1,2,3,11a-Tetrahydro-6-(trifluormethyl)-5H-pyrrolo[2,1-c][1,4]benzodiazepin-5, 11(10H)-dion in 30 ml trockenem Dimethylformamid bei −20 bis −10° unter Rühren mit 1,54 g (35,4 mMol) Natriumhydrid (55proz. Öldispersion), rührt noch während 1 Stunde in obigem Temperaturbereich und tropft dann bei −40° 5,3 ml (35,4 mMol) Diäthylchlorphosphat dazu.

Inzwischen löst man 3,97 g (35,4 mMol) Kalium-tert.-butylat in 9 ml trockenem Dimethylformamid, kühlt in einem Aceton/Trockeneisbad ab- und versetzt mit 4,99 g (35,4 mMol) Isocyanessigsäure-tert.-butylester. Die erhaltene Lösung tropft man bei −20° zum obigen Reaktionsgemisch. Man lässt auf 10° erwärmen, neutralisiert mit 2,0 ml Eisessig, giesst auf 150 ml Wasser und extrahiert dreimal mit Methylenchlorid. Man wäscht die Methylenchloridlösung einmal mit Wasser, trocknet über Magnesiumsulfat, dampft ein und chromatographiert das erhaltene Rohprodukt unter Eluieren mit Essigester an Kieselgel. Die anschliessende Kristallisation aus Diäthyläther liefert tert.-Butyl (S)-11,12,13,13a-tetrahydro-9-oxo-8-(trifluormethyl)-9H-imidazo[1,5-a] pyrrolo[2,1-c][1,4]benzodiazepin-1-carboxylat vom Schmelzpunkt 201–203°.

c) Man rührt eine Mischung aus 2,88 g (7,1 mMol) tert.-Butyl (S)-11,12,13,13a-tetrahydro-9-oxo-8-(trifluormethyl)-9H-imidazo[1,5-a] pyrrolo[2,1-c][1,4]benzodiazepin-1-carboxylat, 1,0 g (4,2 mMol) Tetraäthyl-orthotitanat und 15 g Cyclohexanol während 22 Stunden bei 120°, wobei man viermal je etwa 3 ml Lösungsmittel im Vakuum abdestilliert. Anschliessend dampft man im Vakuum ein, nimmt den Rückstand in 40 ml Chloroform auf, versetzt mit 25 ml Wasser und rührt während 1,25 Stunden. Danach nutscht man durch Kieselgur, trennt die organische Phase ab, extrahiert die wässrige Phase einmal mit Chloroform, trocknet die vereinigten Chloroformauszüge über Magnesiumsulfat und dampft ein. Durch Kristallisieren des zurückbleibenden Öls aus Essigester/n-Hexan erhält man Cyclohexyl (S)-11,12,13,13a-tetrahydro-9-oxo-8-(trifluormethyl)-9H-imidazo[1,5-a]pyrrolo[2,1-c][1,4]benzodiazepin-1-carboxylat vom Schmelzpunkt 193–194°.

Beispiel 73

a) Ausgehend von 3-Chlor-4-fluor-anilin erhält man nach der Sandmeyer'schen Isatinsynthese [T. Sandmeyer, Helv. 2, 234 (1919)] 4-Chlor-5-fluor-isatin. Die Auftrennung der Isomeren nach P. W. Sadler, J. org. Chemistry 21, 169 (1956) liefert nach Umkristallisieren reines 4-Chlor-5-fluor-isatin vom Schmelzpunkt 249–251°.

b) Man suspendiert 7,8 g (0,039 mMol) 4-Chlor-5-fluor-isatin in 50 ml 100proz. Essigsäure, versetzt mit 0,25 ml konzentrierter Schwefelsäure und tropft dann bei 30° 4,4 ml (0,043 Mol) 30 proz. Wasserstoffperoxid dazu. Das Reaktionsgemisch wird anschliessend während 2,5 Stunden auf 70° erhitzt, dann auf 10° abgekühlt und filtriert. Das Rohprodukt wird aus Aceton/Hexan umkristallisiert, wobei man 6-Chlor-5-fluor-isatosäureanhydrid vom Schmelzpunkt 275–278° (Zersetzung) erhält.

c) Man erhitzt 3,3 g (0,015 Mol) 6-Chlor-5-fluor-isatosäureanhydrid und 2 g (0,017 Mol) L-Prolin in 7,5 ml Dimethylformamid während 2 Stunden auf 120°, versetzt nach dem Abkühlen mit 12 ml dest. Wasser und filtriert die ausgefallenen braunen Kristalle ab. Durch Umkristallisieren des Rohproduktes aus Aceton/Hexan erhält man (S)-6-Chlor-7-fluor-1,2,3,11a-tetrahydro-5H-pyrrolo[2,1-c][1,4]benzodiazepin-5,11(10H)-dion vom Schmelzpunkt 217–219°.

d) Man versetzt eine Lösung von 7,16 g (26,6 mMol) (S)-6-Chlor-7-fluor-1,2,3,11a-tetrahydro-5H-pyrrolo[2,1-c][1,4]benzodiazepin-5,11 (10H)-dion in 25 ml trockenem Dimethylformamid bei −20 bis −10° unter Rühren mit 1,27 g (29,26 mMol) Natriumhydrid (55proz. Öldispersion), rührt noch während 1,25 Stunden in obigem Temperaturbereich und tropft dann bei −40° 4,4 ml (29,26 mMol) Diäthylchlorphosphat dazu.

Inzwischen löst man 3,28 g (29,3 mMol) Kalium-tert.-butylat in 8 ml trockenem Dimethylformamid, kühlt in einem Aceton/Trockeneisbad ab, versetzt mit 4,13 g (29,3 mMol) Isocyanessigsäure-tert.-butylester und tropft die erhaltene Lösung bei −20 bis −10° zum obigen Reaktionsgemisch. Man lässt auf 10° erwärmen, neutralisiert mit 1,7 ml Eisessig, giesst auf 150 ml Wasser und extrahiert dreimal mit Methylenchlorid. Man wäscht die Methylenchloridlösung einmal mit Wasser, trocknet über Magnesiumsulfat, dampft ein und chromatographiert das erhaltene Rohprodukt unter Eluieren mit Essigester an Kieselgel. Durch Kristallisieren aus Diäthyläther erhält man tert.-Butyl (S)-8-chlor-7-fluor-11,12,13,13a-tetrahydro-9-oxo-9H-imidazo[1,5-a]pyrrolo[2,1-c][1,4]benzodiazepin-1-carboxylat vom Schmelzpunkt 211–212°.

e) Man rührt eine Mischung aus 4,0 g (10 mMol) tert.-Butyl (S)-8-chlor-7-fluor,11,12,13,13a-tetrahydro-9-oxo-9H-imidazo[1,5-a] pyrrolo[2,1-c][1,4]benzodiazepin-1-carboxylat, 0,8 g (3 mMol) Tetraäthyl-orthotitanat und 15 ml Cyclohexanol über Nacht bei 120°, dampft zur Trockene ein, nimmt den Rückstand in Methylenchlorid auf, wäscht die erhaltene Lösung nacheinander mit 40 ml 5N-Salzsäure, Wasser und gesättigter Natriumbicarbonatlösung, trocknet sie über Magnesiumsulfat und dampft ein. Man erhält Cyclohexyl (S)-8-chlor-7-fluor-11,12,13,13a-tetrahydro-9-oxo-9H-imidazo[1,5-a]pyrrolo[2,1-c][1,4]benzodiazepin-1-carboxylat vom Schmelzpunkt 163–166°.

Beispiel 74

Man rührt eine Mischung aus 3,6 g (10 mMol) tert.-Butyl (S)-8-chlor-12,12a-dihydro-9-oxo-9H, 11H-azeto[2,1-c]imidazo[1,5-a][1,4]benzodiazepin-1-carboxylat, 1,7 g (7,2 mMol) Tetraäthyl-orthotitanat und 20 ml 3-Methoxybenzylalkohol während 5 Stunden bei 120°, wobei man während dieser Zeit dreimal je etwas Lösungsmittel im Vakuum entfernt. Anschliessend destilliert man das überschüssige 3-Methoxybenzylalkohol im Hochvakuum ab, nimmt den Rückstand in 30 ml Chloroform auf, versetzt mit 25 ml Wasser, rührt während 1,25 Stunden und filtriert durch Kieselgur. Man trennt die organische Phase ab, extrahiert die wässrige Phase einmal mit Chloroform, trocknet die vereinigten Chloroformextrakte über Magnesiumsulfat und dampft ein. Das Rohprodukt chromatographiert man unter Eluieren mit Essigester an Kieselgel. Durch Umkristallisieren aus Essigester/n-Hexan erhält man m-Methoxybenzyl (S)-8-chlor-12,12a-dihydro-9-oxo-9H,11H-azeto[2,1-c]imidazo[1,5-a][1,4]benzodiazepin-1-carboxylat vom Schmelzpunkt 126–127°.

Beispiel 75

Man rührt eine Mischung aus 3,45 g (10 mMol) Äthyl (S)-8-chlor-11,12,13,13a-tetrahydro-9-oxo-9H-imidazo[1,5-a]pyrrolo[2,1-c][1,4]benzodiazepin-1-carboxylat, 0,17 g (0,72 mMol) Tetraäthyl-orthotitanat und 7,5 g 2-Cyclopropyläthanol während 14,5 Stunden bei 130°, wobei man nach 0,5 Stunden etwa 2 ml Lösungsmittel abdestilliert. Anschliessend dampft man im Vakuum zur Trockene ein, nimmt den Rückstand in Chloroform auf und chromatographiert unter Eluieren mit Essig ester an Kieselgel. Nach Kristallisieren des erhaltenen Öls aus Essigester/n-Hexan erhält man 2-Cyclopropyläthyl (S)-8-chlor-11,12,13,13a-tetrahydro-9-oxo-9H-imidazo[1,5-a]pyrrolo[2,1-c][1,4]benzodiazepin-1-carboxylat vom Schmelzpunkt 163–164°.

Beispiel 76

Man rührt eine Mischung aus 1,58 g (4,4 mMol) Cyclopropyl (S)-8-chlor-12,12a-dihydro-9-oxo-9H,11H-azeto[2,1-c]imidazo[1,5-a][1,4]benzodiazepin-1-carboxylat, 1,3 g (5,5 mMol) Tetraäthylorthotitanat und 15 g 1-Cyclopropyläthanol während 16 Stunden bei 120°. Danach dampft man im Vakuum zur Trockene ein, nimmt den Rückstand in Methylenchlorid auf und chromatographiert unter Eluieren mit Essigester an Kieselgel. Durch Umkristallisieren aus Essigester/n-Hexan erhält man (R,S)-1-Cyclopropyläthyl (S)-8-chlor-12,12a-dihydro-9-oxo-9H,11H-azeto[2,1-c]imidazo[1,5-a][1,4]benzodiazepin-1-carboxylat vom Schmelzpunkt 187–188° (Gemisch der beiden Diastereoisomeren).

Cyclohexyl (S)-8-brom-11,12,13,13a-tetrahydro-9-oxo-9H-imidazo[1,5-a]pyrrolo[2,1-c][1,4]benzodiazepin-1-carboxylat kann wie in den nachfolgenden Beispielen A bis C angegeben als Wirkstoff zur Herstellung von pharmazeutischen Präparaten verwendet werden:

Beispiel A

Es werden in üblicher Weise Tabletten folgender Zusammensetzung hergestellt:

| | mg/Tabletten |
|---|---|
| Wirkstoff | 5 |
| Milchzucker | 45 |
| Maisstärke | 15 |
| Mikrokristalline Cellulose | 34 |
| Magnesiumstearat | 1 |
| Tablettengewicht | 100 |

Beispiel B

Es werden Kapseln folgender Zusammensetzung hergestellt:

| | mg/Kapseln |
|---|---|
| Wirkstoff | 10 |
| Milchzucker | 155 |
| Maisstärke | 30 |
| Talk | 5 |
| Kapselfüllgewicht | 200 |

Der Wirkstoff, Milchzucker und Maisstärke werden zunächst in einem Mischer und dann in einer Zerkleinerungsmaschine vermengt. Man bringt das Gemisch wieder in den Mischer zurück, gibt den Talk zu und vermengt gründlich. Das Gemisch wird maschinell in Hartgelatinekapsel abgefüllt.

Beispiel C

Es werden Suppositorien folgender Zusammensetzung hergestellt:

| | mg/Supp. |
|---|---|
| Wirkstoff | 15 |
| Suppositorienmasse | 1285 |
| Total | 1300 |

Die Suppositorienmasse wird in einem Glas- oder Stahlgefäss geschmolzen, gründlich vermengt und auf 45° abgekühlt. Hierauf gibt man den fein pulverisierten Wirkstoff zu und rührt, bis er vollständig dispergiert ist. Man giesst die Mischung in Suppositorienformen geeigneter Grösse, lässt abkühlen, nimmt dann die Suppositorien aus den Formen und verpackt sie einzeln in Wachspapier oder Metallfolien.

Die nachfolgend aufgeführten Verbindungen der Formel I können anstelle von Cyclohexyl (S)-8-brom-11,12,13,13a-tetrahydro-9-oxo-9H-imidazo[1,5-a]pyrrolo[2,1-c][1,4]benzodiazepin-1-carboxylat wie in den obigen Beispielen A bis C angegeben als Wirkstoffe verwendet werden:

Cyclopropylmethyl (S)-8-chlor-12,12a-dihydro-9-oxo-9H,11H-azeto[2,1-c]imidazo[1,5-a][1,4]benzodiazepin-1-carboxylat,

Cyclohexyl (S)-8-chlor-7-fluor-11,12,13,13a-tetrahydro-9-oxo-9H-imidazo[1,5-a]pyrrolo[2,1-c][1,4]benzodiazepin-1-carboxylat,

Cyclopropylmethyl (S)-7-fluor-11,12,13,13a,tetrahydro-9-oxo-9H-imidazo[1,5-a]pyrrolo[2,1-c][1,4]benzodiazepin-1-carboxylat,

Cyclohexyl (S)-8-brom-11,12,13,13a-tetrahydro-9-oxo-9H-imidazo[1,5-a]pyrrolo[2,1-c][1,4]benzodiazepin-1-carboxylat,

(R,S)-1-Cyclopropyläthyl (S)-8-chlor-11,12,13,13a-tetrahydro-9-oxo-9H-imidazo[1,5-a]pyrrolo[2,1-c][1,4]benzodiazepin-1-carboxylat,

Cyclohexyl (S)-7-fluor-12,12a-dihydro-9-oxo-9H,11H-azeto[2,1-c]imidazo[1,5-a][1,4-]benzodiazepin-1-carboxylat,

(R,S)-2-Cyclohexen-1-yl (S)-8-chlor-11,12,13,13a-tetrahydro-9-oxo-9H-imidazo[1,5-a]pyrrolo[2,1-c][1,4]benzodiazepin-1-carboxylat,

(R,S)-1-Cyclopropyläthyl (S)-8-chlor-12,12a-dihydro-9-oxo-9H,11H-azeto[2,1-c]imidazo[1,5-a][1,4]benzodiazepin-1-carboxylat,

Cyclohexyl (S)-12,12a-dihydro-8-jod-9-oxo-9H,11H-azeto[2,1-c]imidazo[1,5-a][1,4]benzodiazepin-1-carboxylat,

Cyclopropylmethyl (S)-7-fluor-12,12a-dihydro-9-oxo-9H-imidazo[1,5-a]pyrrolo[2,1-c][1,4]benzodiazepin-1-carboxylat,

Cyclohexyl (S)-11,12,13,13a-tetrahydro-9-oxo-8-(trifluormethyl)-9H-imidazo[1,5-a]pyrrolo[2,1-c][1,4]benzodiazepin-1-carboxylat und

Cyclohexyl (S)-8-chlor-12,12a-dihydro-9-oxo-9H,11H-azeto[2,1-c]imidazo[1,5-a][1,4]benzodiazepin-1-carboxylat.

**Patentansprüche für die Vertragsstaaten: BE, CH, DE, FR, GB, IT, LI, LU, NL und SE**

1. Imidazobenzodiazepine der allgemeinen Formel

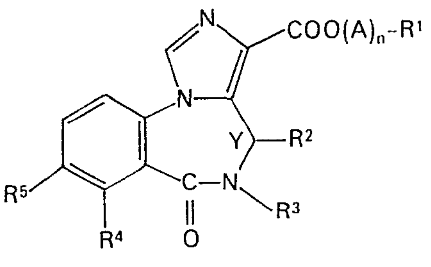

I

worin A niederes Alkylen, n die Zahl 0 oder 1, $R^1$ niederes Alkinyl, niederes Alkenyl, gegebenenfalls durch Halogen, niederes Alkyloxy, niederes Alkyl, Trifluormethyl oder Nitro substituiertes Phenyl, jeweils gegebenenfalls durch niederes Alkyl substituiertes $(C_{3-8})$-Cycloalkyl oder $(C_{5-8})$-Cycloalkenyl oder einen gegebenenfalls durch niederes Alkyl substituierten 5- oder 6gliedrigen gesättigten oder ungesättigten Heterocyclus, der als Ringglied ein Sauerstoff- oder Schwefelatom enthält, $R^4$ und $R^5$ je Wasserstoff, Halogen, Trifluormethyl, Cyano, Nitro, Amino oder niederes Alkyl und entweder $R^2$ Wasserstoff und $R^3$ niederes Alkyl oder $R^2$ und $R^3$ zusammen Dimethylen, Trimethylen oder Propenylen bedeuten, wobei die Verbindungen der Formel I, worin $R^2$ und $R^3$ zusammen Dimethylen, Trimethylen oder Propenylen bedeuten, bezüglich des mit $\gamma$ bezeichneten Kohlenstoffatoms die (S)- oder (R,S)-Konfiguration aufweisen und wobei die als «nieder» bezeichneten Reste höchstens 7 Kohlenstoffatome aufweisen und pharmazeutisch annehmbare Säureadditionssalze davon.

2. Verbindungen gemäss Anspruch 1, dadurch gekennzeichnet, dass n die Zahl 0 bedeutet oder dass n die Zahl 1 und A gegebenenfalls durch Alkyl, das höchstens 7 Kohlenstoffatome aufweist, substituiertes Methylen oder 1,2-Äthylen bedeuten.

3. Verbindungen gemäss Anspruch 1 oder 2, dadurch gekennzeichnet, dass $R^1$ Alkinyl, Phenyl oder jeweils gegebenenfalls durch Alkyl, das höchstens 7 Kohlenstoffatome aufweist, substituiertes $(C_{3-8})$-Cycloalkyl oder $(C_{5-8})$-Cycloalkenyl bedeutet.

4. Verbindungen gemäss Anspruch 3, dadurch gekennzeichnet, dass $R^1$ gegebenenfalls durch Alkyl, das höchstens 7 Kohlenstoffatome aufweist, substituiertes $(C_{3-6})$-Cycloalkyl bedeutet.

5. Verbindungen gemäss Anspruch 4, dadurch gekennzeichnet, dass die Gruppe $-(A)_n-R^1$ Cyclohexyl, 2-Cyclohexen-1-yl, Cyclopropylmethyl, 1-Cyclopropyläthyl oder 2-Cyclopropyläthyl bedeutet.

6. Verbindungen gemäss einem der Ansprüche 1 bis 5, dadurch gekennzeichnet, dass $R^2$ und $R^3$ zusammen Dimethylen, Trimethylen oder Propenylen bedeuten und die entsprechenden Verbindungen der Formel I bezüglich des mit $\gamma$ bezeichneten Kohlenstoffatoms die (S)-Konfiguration aufweisen.

7. Verbindungen gemäss einem der Ansprüche 1 bis 6, dadurch gekennzeichnet, dass $R^4$ Wasserstoff, Halogen, Trifluormethyl, Nitro, Cyano oder Alkyl, das höchstens 7 Kohlenstoffatome aufweist, bedeutet.

8. Verbindungen gemäss einem der Ansprüche 1 bis 7, dadurch gekennzeichnet, dass $R^5$ Wasserstoff oder Halogen bedeutet.

9. Cyclopropylmethyl (S)-8-chlor-12,12a-dihydro-9-oxo-9H,11H-azeto[2,1-c]imidazo[1,5-a][1,4]benzodiazepin-1-carboxylat.

10. Cyclohexyl (S)-8-brom-11,12,13,13a-tetrahydro-9-oxo-9H-imidazo[1,5-a]pyrrolo[2,1-c][1,4]benzodiazepin-1-carboxylat.

11. (R,S)-2-Cyclohexen-1-yl (S)-8-chlor-11,12,13,13a-tetrahydro-9-oxo-9H-imidazo[1,5-a]pyrrolo[2,1-c][1,4]benzodiazepin-1-carboxylat.

12. Cyclohexyl (S)-8-chlor-12,12a-dihydro-9-oxo-9H,11H-azeto[2,1-c]imidazo[1,5-a][1,4]benzodiazepin-1-carboxylat.

13. Cyclohexyl (S)-7-fluor-12,12a-dihydro-9-oxo-9H,11H-azeto[2,1-c]imidazo[1,5-a][1,4]benzodiazepin-1-carboxylat.

14. Cyclohexyl (S)-8-chlor-7-fluor-11,12,13,13a-tetrahydro-9-oxo-9H-imidazo[1,5-a]pyrrolo[2,1-c][1,4]benzodiazepin-1-carboxylat.

15. (R,S)-1-Cyclopropyläthyl (S)-8-chlor-12,12a-dihydro-9-oxo-9H,11H-azeto[2,1-c]imidazo[1,5-a][1,4]benzodiazepin-1-carboxylat.

16. Cyclohexyl (S)-12,12a-dihydro-8-jod-9-oxo-9H,11H-azeto[2,1-c]imidazo[1,5-a][1,4]benzodiazepin-1-carboxylat.

17. Cyclopropylmethyl (S)-7-fluor-11,12,13,13a-tetrahydro-9-oxo-9H-imidazo[1,5-a]pyrrolo[2,1-c][1,4]benzodiazepin-1-carboxylat.

18. (R,S)-1-Cyclopropyläthyl (S)-8-chlor-11,12,13,13a-tetrahydro-9-oxo-9H-imidazo[1,5-a]pyrrolo[2,1-c][1,4]benzodiazepin-1-carboxylat.

19. Cyclopropylmethyl (S)-7-fluor-12,12a-dihydro-9-oxo-9H,11H-azeto[2,1-c]imidazo[1,5-a][1,4]benzodiazepin-1-carboxylat.

20. Cyclohexyl (S)-11,12,13,13a-tetrahydro-9-oxo-8-(trifluormethyl)-9H-imidazo[1,5-a]pyrrolo[2,1-c][1,4]benzodiazepin-1-carboxylat.

21. Cyclohexyl (S)-8-chlor-11,12,13,13a-tetrahydro-9-oxo-9H-imidazo[1,5-a]pyrrolo[2,1-c][1,4]benzodiazepin-1-carboxylat, 2-Propinyl (S)-8-chlor-11,12,13,13a-tetrahydro-9-oxo-9H-imidazo[1,5-a]pyrrolo[2,1-c][1,4]benzodiazepin-1-carboxylat, Cyclopropylmethyl 8-chlor-11,13a-dihydro-9-oxo-9H-imidazo[1,5-a]pyrrolo[2,1-c][1,4]benzodiazepin-1-carboxylat, Cyclopropylmethyl (S)-8-chlor-11,12,13,13a-tetrahydro-9-oxo-9H-imidazo[1,5-a]pyrrolo[2,1-c][1,4]benzodiazepin-1-carboxylat, Cyclopropylmethyl (S)-8-brom-11,12,13,13a-tetrahydro-9-oxo-9H-imidazo[1,5-a]pyrrolo[2,1-c][1,4]benzodiazepin-1-carboxylat, Cyclopropylmethyl (S)-11,12,13,13a-tetrahydro-8-jod-9-oxo-9H-imidazo[1,5-a]pyrrolo[2,1-c][1,4]benzodiazepin-1-carboxylat, 2-Cyclopropyläthyl (S)-8-chlor-11,12,13,13a-tetrahydro-9-oxo-9H-imidazo[1,5-a]pyrrolo[2,1-c][1,4]benzodiazepin-1-carboxylat, Cyclohexyl (S)-12,12a-dihydro-8-methyl-9-oxo-9H,11H-azeto[2,1-c]imidazo[1,5-a][1,4]benzodiazepin-1-carboxylat, rac-cis-3-Methylcyclohexyl (S)-8-chlor-11,12,13,13a-tetrahydro-9-oxo-9H-imidazo[1,5-a]pyrrolo[2,1-c][1,4]benzodiazepin-1-carboxylat, Cyclopentyl (S)-12,12a-dihydro-8-jod-9-oxo-9H,11H-azeto[2,1-c]imidazo[1,5-a][1,4]benzodiazepin-1-carboxylat, Cycloheptyl (S)-8-chlor-11,12,13,13a-tetrahydro-9-oxo-9H-imidazo[1,5-a]pyrrolo[2,1-c][1,4]benzodiazepin-1-carboxylat, Benzyl (S)-8-chlor-12,12a-dihydro-9-oxo-9H,11H-azeto[2,1-c]imidazo[1,5-a][1,4]benzodiazepin-1-carboxylat, Cyclopropylmethyl (S)-12,12a-dihydro-8-jod-9-oxo-9H,11H-azeto[2,1-c]imidazo[1,5-a][1,4]benzodiazepin-1-carboxylat, Cyclohexyl (S)-8-brom-12,12a-dihydro-9-oxo-9H,11H-azeto[2,1-c]imidazo[1,5-a][1,4]benzodiazepin-1-carboxylat, Cyclopropylmethyl (S)-8-brom-12,12a-dihydro-9-oxo-9H,11H-azeto[2,1-c]imidazo[1,5-a][1,4]benzodiazepin-1-carboxylat, Cyclohexyl (S)-11,12,13,13a-tetrahydro-8-nitro-9-oxo-9H-imidazo[1,5-a]pyrrolo[2,1-c][1,4]benzodiazepin-1-carboxylat und Cyclohexyl (S)-8-cyano-11,12,13,13a-tetrahydro-9-oxo-9H-imidazo[1,5-a]pyrrolo[2,1-c][1,4]benzodiazepin-1-carboxylat.

22. Verbindungen gemäss einem der Ansprüche 1 bis 21 zur Anwendung als therapeutische Wirkstoffe.

23. Cyclopropylmethyl (S)-8-chlor-12,12a-dihydro-9-oxo-9H,11H-azeto[2,1-c]imidazo[1,5-a][1,4]benzodiazepin-1-carboxylat zur Anwendung als therapeutischer Wirkstoff.

24. Verfahren zur Herstellung von Verbindungen gemäss einem der Ansprüche 1 bis 21, dadurch gekennzeichnet, dass man

a) einen Carbonsäureester der allgemeinen Formel

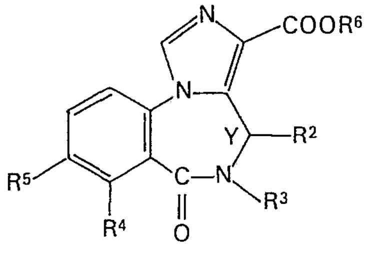

II

worin $R^6$ niederes Alkyl oder die Gruppe $-(A)_n-R^1$ bedeutet und A, n, $R^1$, $R^2$, $R^3$, $R^4$ und $R^5$ die in Anspruch 1 gegebene Bedeutung besitzen, mit einem den gewünschten Rest $-(A)_n-R^1$ liefernden Alkohol der allgemeinen Formel

$$HO-(A)_n-R^1 \quad III$$

worin A, n und $R^1$ die in Anspruch 1 gegebene Bedeutung besitzen, umestert, oder

b) eine Carbonsäure der allgemeinen Formel

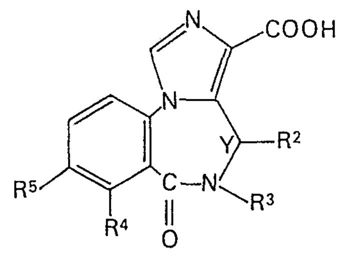

IV

worin $R^2$, $R^3$, $R^4$ und $R^5$ die in Anspruch 1 gegebene Bedeutung besitzen, mit einem den Rest $-(A)_n-R^1$ liefernden Mittel verestert, oder

c) eine Verbindung der allgemeinen Formel

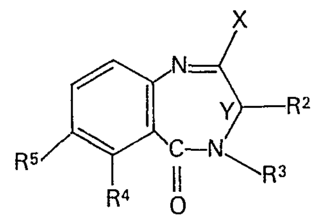

V

worin X eine Abgangsgruppe bedeutet und $R^2$, $R^3$, $R^4$ und $R^5$ die in Anspruch 1 gegebene Bedeutung besitzen, in Gegenwart einer Base mit einem Isocyanessigester der allgemeinen Formel

$$CN-CH_2-COO-(A)_n-R^1 \quad VI$$

worin A, n und $R^1$ die in Anspruch 1 gegebene Bedeutung besitzen, umsetzt, oder

d) in einer Verbindung der Formel I, worin einer der Reste $R^4$ und $R^5$ Halogen und der andere Wasserstoff, Trifluormethyl, Amino, Nitro, Cyano oder niederes Alkyl bedeutet und A, n, $R^1$, $R^2$ und $R^3$ die in Anspruch 1 gegebene Bedeutung besitzen, das Halogenatom durch die Cyanogruppe ersetzt, oder

e) in einer Verbindung der Formel I, worin einer der Reste $R^4$ und $R^5$ Amino und der andere Wasserstoff, Halogen, Trifluormethyl, Nitro, Cyano oder niederes Alkyl bedeutet und A, n, $R^1$, $R^2$ und $R^3$ die in Anspruch 1 gegebene Bedeutung besitzen, die Aminogruppe durch ein Wasserstoff- oder Halogenatom oder durch eine Cyano- oder Nitrogruppe ersetzt, oder

f) eine Verbindung der Formel I, worin einer der Reste $R^4$ und $R^5$ Amino und der andere Wasserstoff bedeutet und A, n, $R^1$, $R^2$ und $R^3$ die in Anspruch 1 gegebene Bedeutung besitzen, in α-Stellung zur Aminogruppe halogeniert, oder

g) in einer Verbindung der Formel I, worin einer der Reste $R^4$ und $R^5$ Nitro und der andere Wasserstoff bedeutet und A, n, $R^1$, $R^2$ und $R^3$ die in Anspruch 1 gegebene Bedeutung besitzen, die Nitrogruppe zur Aminogruppe reduziert, und

h) erwünschtenfalls eine erhaltene Verbindung der Formel I in ein pharmazeutisch annehmbares Säureadditionssalz überführt.

25. Arzneimittel, enthaltend eine Verbindung gemäss einem der Ansprüche 1 bis 21 und ein therapeutisch inertes Exzipiens.

26. Arzneimittel, enthaltend Cyclopropylmethyl (S)-8-chlor-12,12a-dihydro-9-oxo-9H,11H-azeto[2,1-c]imidazo[1,5-a][1,4]benzodiazepin-1-carboxylat.

**Patentansprüche für den Vertragsstaat: AT**

1. Verfahren zur Herstellung von Imidazobenzodiazepinen der allgemeinen Formel

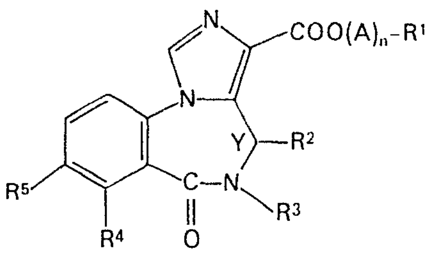

I

worin A niederes Alkylen, n die Zahl 0 oder 1, $R^1$ niederes Alkinyl, niederes Alkenyl, gegebenenfalls durch Halogen, niederes Alkyloxy, niederes Alkyl, Trifluormethyl oder Nitro substituiertes Phenyl, jeweils gegebenenfalls durch niederes Alkyl substituiertes $(C_{3-8})$-Cycloalkyl oder $(C_{5-8})$-Cycloalkenyl oder einen gegebenenfalls durch niederes Alkyl substituierten 5- oder 6gliedrigen gesättigten oder ungesättigten Heterocyclus, der als Ringglied ein Sauerstoff- oder Schwefelatom enthält, $R^4$ und $R^5$ je Wasserstoff, Halogen, Trifluormethyl, Cyano, Nitro, Amino oder niederes Alkyl und entweder $R^2$ Wasserstoff und $R^3$ niederes Alkyl oder $R^2$ und $R^3$ zusammen Dimethylen, Trimethylen oder Propenylen bedeuten, wobei die Verbindungen der Formel I, worin $R^2$ und $R^3$ zusammen Dimethylen, Trimethylen oder Propenylen bedeuten, bezüglich des mit $\gamma$ bezeichneten Kohlenstoffatoms die (S)- oder (R,S)-Konfiguration aufweisen und wobei die als «nieder» bezeichneten Reste höchstens 7 Kohlenstoffatome aufweisen, und pharmazeutisch annehmbaren Säureadditionssalzen davon, dadurch gekennzeichnet, dass man

a) einen Carbonsäureester der allgemeinen Formel

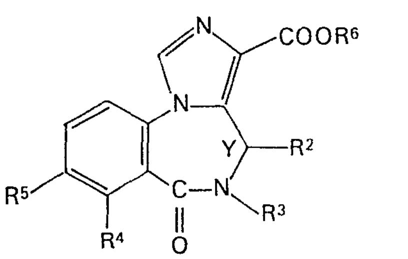

II

worin $R^6$ niederes Alkyl oder die Gruppe $-(A)_n-R^1$ bedeutet und A, n, $R^1$, $R^2$, $R^3$, $R^4$ und $R^5$ obige Bedeutung besitzen, mit einem den gewünschten Rest $-(A)_n-R^1$ liefernden Alkohol der allgemeinen Formel

$HO-(A)_n-R^1$ III

worin A, n und $R^1$ obige Bedeutung besitzen, umestert, oder

b) eine Carbonsäure der allgemeinen Formel

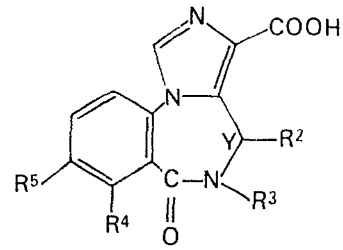

IV

worin $R^2$, $R^3$, $R^4$ und $R^5$ obige Bedeutung besitzen, mit einem den Rest $-(A)_n-R^1$ liefernden Mittel verestert, oder

c) eine Verbindung der allgemeinen Formel

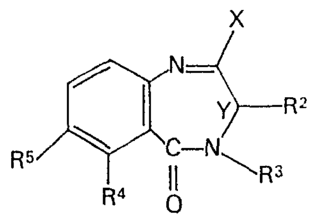

V

worin X eine Abgangsgruppe bedeutet und $R^2$, $R^3$, $R^4$ und $R^5$ obige Bedeutung besitzen, in Gegenwart einer Base mit einem Isocyanessigester der allgemeinen Formel

$CN-CH_2-COO-(A)_n-R^1$ VI

worin A, n und $R^1$ obige Bedeutung besitzen, umsetzt, oder

d) in einer Verbindung der Formel I, worin einer der Reste $R^4$ und $R^5$ Halogen und der andere Wasserstoff, Trifluormethyl, Amino, Nitro, Cyano oder niederes Alkyl bedeutet und A, n, $R^1$, $R^2$ und $R^3$ obige Bedeutung besitzen, das Halogenatom durch die Cyanogruppe ersetzt, oder

e) in einer Verbindung der Formel I, worin einer der Reste $R^4$ und $R^5$ Amino und der andere Wasserstoff, Halogen, Trifluormethyl, Nitro, Cyano oder niederes Alkyl bedeutet und A, n, $R^1$, $R^2$ und $R^3$ obige Bedeutung besitzen, die Aminogruppe durch ein Wasserstoff- oder Halogenatom oder durch eine Cyano- oder Nitrogruppe ersetzt, oder

f) eine Verbindung der Formel I, worin einer der Reste $R^4$ und $R^5$ Amino und der andere Wasserstoff bedeutet und A, n, $R^1$, $R^2$ und $R^3$ obige Bedeutung besitzen, in $\alpha$-Stellung zur Aminogruppe halogeniert, oder

g) in einer Verbindung der Formel I, worin einer der Reste $R^4$ und $R^5$ Nitro und der andere Wasserstoff bedeutet und A, n, $R^1$, $R^2$ und $R^3$ obige Bedeutung besitzen, die Nitrogruppe zur Aminogruppe reduziert, und

h) erwünschtenfalls eine erhaltene Verbindung der Formel I in ein pharmazeutisch annehmbares Säureadditionssalz überführt.

2. Verfahren gemäss Anspruch 1, dadurch gekennzeichnet, dass n die Zahl 0 bedeutet oder dass n die Zahl 1 und A gegebenenfalls durch Alkyl, das höchstens 7 Kohlenstoffatome aufweist, substituiertes Methylen oder 1,2-Äthylen bedeuten.

3. Verfahren gemäss Anspruch 1 oder 2, dadurch gekennzeichnet, dass $R^1$ Alkinyl, Phenyl oder jeweils gegebenenfalls durch Alkyl, das höchstens 7 Kohlenstoffatome aufweist, substituiertes ($C_{3-8}$)-Cycloalkyl oder ($C_{5-8}$)-Cycloalkenyl bedeutet.

4. Verfahren gemäss Anspruch 3, dadurch gekennzeichnet, dass $R^1$ gegebenenfalls durch Alkyl, das höchstens 7 Kohlenstoffatome aufweist, substituiertes ($C_{3-6}$)-Cycloalkyl bedeutet.

5. Verfahren gemäss Anspruch 4, dadurch gekennzeichnet, dass die Gruppe $-(A)_n-R^1$ Cyclohexyl, 2-Cyclohexen-1-yl, Cyclopropylmethyl, 1-Cyclopropyläthyl oder 2-Cyclopropyläthyl bedeutet.

6. Verfahren gemäss einem der Ansprüche 1 bis 5, dadurch gekennzeichnet, dass $R^2$ und $R^3$ zusammen Dimethylen, Trimethylen oder Propenylen bedeuten und die entsprechenden Verbindungen der Formel I bezüglich des mit γ bezeichneten Kohlenstoffatoms die (S)-Konfiguration aufweisen.

7. Verfahren gemäss einem der Ansprüche 1 bis 6, dadurch gekennzeichnet, dass $R^4$ Wasserstoff, Halogen, Trifluormethyl, Nitro, Cyano oder Alkyl, das höchstens 7 Kohlenstoffatome aufweist, bedeutet.

8. Verfahren gemäss einem der Ansprüche 1 bis 7, dadurch gekennzeichnet, dass $R^5$ Wasserstoff oder Halogen bedeutet.

9. Verfahren gemäss Anspruch 1, dadurch gekennzeichnet, dass man Cyclopropylmethyl (S)-8-chlor-12,12a-dihydro-9-oxo-9H,11H-azeto[2,1-c]imidazo[1,5-a][1,4]benzodiazepin-1-carboxylat herstellt.

10. Verfahren gemäss Anspruch 1, dadurch gekennzeichnet, dass man Cyclohexyl (S)-8-brom-11,12,13,13a-tetrahydro-9-oxo-9H-imidazo[1,5-a]pyrrolo[2,1-c][1,4]benzodiazepin-1-carboxylat herstellt.

11. Verfahren gemäss Anspruch 1, dadurch gekennzeichnet, dass man (R,S)-2-Cyclohexen-1-yl (S)-8-chlor-11,12,13,13a-tetrahydro-9-oxo-9H-imidazo[1,5-a]pyrrolo[2,1-c][1,4]benzodiazepin-1-carboxylat herstellt.

12. Verfahren gemäss Anspruch 1, dadurch gekennzeichnet, dass man Cyclohexyl (S)-8-chlor-12,12a-dihydro-9-oxo-9H,11H-azeto[2,1-c]imidazo[1,5-a][1,4]benzodiazepin-1-carboxylat herstellt.

13. Verfahren gemäss Anspruch 1, dadurch gekennzeichnet, dass man Cyclohexyl (S)-7-fluor-12,12a-dihydro-9-oxo-9H,11H-azeto[2,1-c]imidazo[1,5-a][1,4]benzodiazepin-1-carboxylat herstellt.

14. Verfahren gemäss Anspruch 1, dadurch gekennzeichnet, dass man Cyclohexyl (S)-8-chlor-7-fluor-11,12,13,13a-tetrahydro-9-oxo-9H-imidazo[1,5-a]pyrrolo[2,1-c][1,4]benzodiazepin-1-carboxylat herstellt.

15. Verfahren gemäss Anspruch 1, dadurch gekennzeichnet, dass man (R,S)-1-Cyclopropyläthyl (S)-8-chlor-12,12a-dihydro-9-oxo-9H,11H-azeto[2,1-c]imidazo[1,5-a][1,4]benzodiazepin-1-carboxylat herstellt.

16. Verfahren gemäss Anspruch 1, dadurch gekennzeichnet, dass man Cyclohexyl (S)-12,12a-dihydro-8-jod-9-oxo-9H,11H-azeto[2,1-c]imidazo[1,5-a][1,4]benzodiazepin-1-carboxylat herstellt.

17. Verfahren gemäss Anspruch 1, dadurch gekennzeichnet, dass man Cyclopropylmethyl (S)-7-fluor-11,12,13,13a-tetrahydro-9-oxo-9H-imidazo[1,5-a]pyrrolo[2,1-c][1,4]benzodiazepin-1-carboxylat herstellt.

18. Verfahren gemäss Anspruch 1, dadurch gekennzeichnet, dass man (R,S)-1-Cyclopropyläthyl (S)-8-chlor-11,12,13,13a-tetrahydro-9-oxo-9H-imidazo[1,5-a]pyrrolo[2,1-c][1,4]benzodiazepin-1-carboxylat herstellt.

19. Verfahren gemäss Anspruch 1, dadurch gekennzeichnet, dass man Cyclopropylmethyl (S)-7-fluor-12,12a-dihydro-9-oxo-9H,11H-azeto[2,1-c]imidazo[1,5-a][1,4]benzodiazepin-1-carboxylat herstellt.

20. Verfahren gemäss Anspruch 1, dadurch gekennzeichnet, dass man Cyclohexyl (S)-11,12,13,13a-tetrahydro-9-oxo-8-(trifluormethyl)-9H-imidazo[1,5-a]pyrrolo[2,1-c][1,4]benzodiazepin-1-carboxylat herstellt.

**Claims for the Contracting States: BE, CH, DE, FR, GB, IT, LI, LU, NL and SE**

1. Imidazobenzodiazepines of the general formula

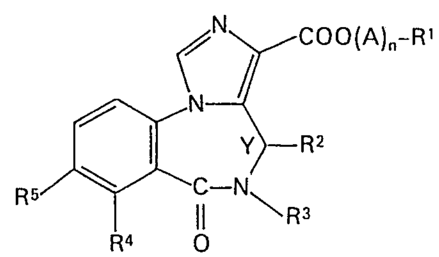

I

wherein A signifies lower alkylene, n signifies the number 0 or 1, $R^1$ signifies lower alkynyl, lower alkenyl, phenyl optionally substituted by halogen, lower alkyloxy, lower alkyl, trifluoromethyl or nitro, ($C_{3-8}$)-cycloalkyl or ($C_{5-8}$)-cycloalkenyl in each case optionally substituted by lower alkyl, or a 5- or 6-membered saturated or unsaturated heterocycle which contains an oxygen or sulphur atom as a ring member and which is optionally substituted by lower alkyl, $R^4$ and $R^5$ each signify hydrogen, halogen, trifluoromethyl, cyano, nitro, amino or lower alkyl and either $R^2$ signifies hydrogen and $R^3$ signifies lower alkyl or $R^2$ and $R^3$ together signify dimethylene, trimethylene or propenylene, whereby the compounds of formula I in which $R^2$ and $R^3$ together signify dimethylene, trimethylene of propenylene have the (S)- or (R,S)-configuration with reference to the carbon atom denoted by γ and whereby the residues denoted as «lower» have a maximum of 7 carbon atoms, and pharmaceutically acceptable acid addition salts thereof.

2. Compounds in accordance with claim 1, characterized in that n signifies the number 0 or in that n signifies the number 1 and A signifies methylene or 1,2-ethylene optionally substituted by alkyl which has a maximum of 7 carbon atoms.

3. Compounds in accordance with claim 1 or 2, characterized in that $R^1$ signifies alkynyl, phenyl, or $(C_{3-8})$-cycloalkyl or $(C_{5-8})$-cycloalkenyl in each case optionally substituted by alkyl which has a maximum of 7 carbon atoms.

4. Compounds in accordance with claim 3, characterized in that $R^1$ signifies $(C_{3-6})$-cycloalkyl optionally substituted by alkyl which has a maximum of 7 carbon atoms.

5. Compounds in accordance with claim 4, characterized in that the group $-(A)_n-R^1$ signifies cyclohexyl, 2-cyclohexen-1-yl, cyclopropylmethyl, 1-cyclopropylethyl or 2-cyclopropylethyl.

6. Compounds in accordance with any one of claims 1 to 5, characterized in that $R^2$ and $R^3$ together signify dimethylene, trimethylene or propenylene and the corresponding compounds of formula I have the (S)-configuration with reference to the carbon atom denoted by $\gamma$.

7. Compounds in accordance with any one of claims 1 to 6, characterized in that $R^4$ signifies hydrogen, halogen, trifluoromethyl, nitro, cyano or alkyl which has a maximum of 7 carbon atoms.

8. Compounds in accordance with any one of claims 1 to 7, characterized in that $R^5$ signifies hydrogen or halogen.

9. Cyclopropylmethyl (S)-8-chloro-12,12a-dihydro-9-oxo-9H,11H-azeto[2,1-c]imidazo[1,5-a][1,4]benzodiazepine-1-carboxylate.

10. Cyclohexyl (S)-8-bromo-11,12,13,13a-tetrahydro-9-oxo-9H-imidazo[1,5-a]pyrrolo[2,1-c][1,4]benzodiazepine-1-carboxylate.

11. (R,S)-2-Cyclohexen-1-yl (S)-8-chloro-11,12,13,13a-tetrahydro-9-oxo-9H-imidazo[1,5-a]pyrrolo[2,1-c][1,4]benzodiazepine-1-carboxylate.

12. Cyclohexyl (S)-8-chloro-12,12a-dihydro-9-oxo-9H,11H-azeto[2,1-c]imidazo[1,5-a][1,4]benzodiazepine-1-carboxylate.

13. Cyclohexyl (S)-7-fluoro-12,12a-dihydro-9-oxo-9H,11H-azeto[2,1-c]imidazo[1,5-a][1,4]benzodiazepine-1-carboxylate.

14. Cyclohexyl (S)-8-chloro-7-fluoro-11,12,13,13a-tetrahydro-9-oxo-9H-imidazo[1,5-a]pyrrolo[2,1-c][1,4]benzodiazepine-1-carboxylate.

15. (R,S)-1-Cyclopropylethyl (S)-8-chloro-12,12a-dihydro-9-oxo-9H,11H-azeto[2,1-c]imidazo[1,5-a][1,4]benzodiazepine-1-carboxylate.

16. Cyclohexyl (S)-12,12a-dihydro-8-iodo-9-oxo-9H,11H-azeto[2,1-c]imidazo[1,5-a][1,4]benzodiazepine-1-carboxylate.

17. Cyclopropylmethyl (S)-7-fluoro-11,12,13,13a-tetrahydro-9-oxo-9H-imidazo[1,5-a]pyrrolo[2,1-c][1,4]benzodiazepine-1-carboxylate.

18. (R,S)-1-Cyclopropylethyl (S)-8-chloro-11,12,13,13a-tetrahydro-9-oxo-9H-imidazo[1,5-a]pyrrolo[2,1-c][1,4]benzodiazepine-1-carboxylate.

19. Cyclopropylmethyl (S)-7-fluoro-12,12a-dihydro-9-oxo-9H,11H-azeto[2,1-c]imidazo[1,5-a][1,4]benzodiazepine-1-carboxylate.

20. Cyclohexyl (S)-11,12,13,13a-tetrahydro-9-oxo-8-(trifluoromethyl)-9H-imidazo[1,5-a]pyrrolo[2,1-c][1,4]benzodiazepine-1-carboxylate.

21. Cyclohexyl (S)-8-chloro-11,12,13,13a-tetrahydro-9-oxo-9H-imidazo[1,5-a]pyrrolo[2,1-c][1,4]benzodiazepine-1-carboxylate, 2-propynyl (S)-8-chloro-11,12,13,13a-tetrahydro-9-oxo-9H-imidazo[1,5-a]pyrrolo[2,1-c][1,4]benzodiazepine-1-carboxylate, cyclopropylmethyl 8-chloro-11,13a-dihydro-9-oxo-9H-imidazo[1,5-a]pyrrolo[2,1-c][1,4]benzodiazepine-1-carboxylate, cyclopropylmethyl (S)-8-chloro-11,12,13,13a-tetrahydro-9-oxo-9H-imidazo[1,5-a]pyrrolo[2,1-c][1,4]benzodiazepine-1-carboxylate, cyclopropylmethyl (S)-8-bromo-11,12,13,13a-tetrahydro-9-oxo-9H-imidazo[1,5-a]pyrrolo[2,1-c][1,4]benzodiazepine-1-carboxylate, cyclopropylmethyl (S)-11,12,13,13a-tetrahydro-8-iodo-9-oxo-9H-imidazo[1,5-a]pyrrolo[2,1-c][1,4]benzodiazepine-1-carboxylate, 2-cyclopropylethyl (S)-8-chloro-11,12,13,13a-tetrahydro-9-oxo-9H-imidazo[1,5-a]pyrrolo[2,1-c][1,4]benzodiazepine-1-carboxylate, cyclohexyl (S)-12,12a-dihydro-8-methyl-9-oxo-9H,11H-azeto[2,1-c]imidazo[1,5-a][1,4]benzodiazepine-1-carboxylate, rac-cis-3-methylcyclohexyl (S)-8-chloro-11,12,13,13a-tetrahydro-9-oxo-9H-imidazo[1,5-a]pyrrolo[2,1-c][1,4]benzodiazepine-1-carboxylate, cyclopentyl (S)-12,12a-dihydro-8-iodo-9-oxo-9H,11H-azeto[2,1-c]imidazo[1,5-a][1,4]benzodiazepine-1-carboxylate, cycloheptyl (S)-8-chloro-11,12,13,13a-tetrahydro-9-oxo-9H-imidazo[1,5-a]pyrrolo[2,1-c][1,4]benzodiazepine-1-carboxylate, benzyl (S)-8-chloro-12,12a-dihydro-9-oxo-9H,11H-azeto[2,1-c]imidazo[1,5-a][1,4]benzodiazepine-1-carboxylate, cyclopropylmethyl (S)-12,12a-dihydro-8-iodo-9-oxo-9H,11H-azeto[2,1-c]imidazo[1,5-a][1,4]benzodiazepine-1-carboxylate, cyclohexyl (S)-8-bromo-12,12a-dihydro-9-oxo-9H,11H-azeto[2,1-c]imidazo[1,5-a][1,4]benzodiazepine-1-carboxylate, cyclopropylmethyl (S)-8-bromo-12,12a-dihydro-9-oxo-9H,11H-azeto[2,1-c]imidazo[1,5-a][1,4]benzodiazepine-1-carboxylate, cyclohexyl (S)-11,12,13,13a-tetrahydro-8-nitro-9-oxo-9H-imidazo[1,5-a]pyrrolo[2,1-c][1,4]benzodiazepine-1-carboxylate and cyclohexyl (S)-8-cyano-11,12,13,13a-tetrahydro-9-oxo-9H-imidazo[1,5-a]pyrrolo[2,1-c][1,4]benzodiazepine-1-carboxylate.

22. Compounds in accordance with any one of claims 1 to 21 for use as therapeutically active substances.

23. Cyclopropylmethyl (S)-8-chloro-12,12a-dihydro-9-oxo-9H,11H-azeto[2,1-c]imidazo[1,5-a][1,4]benzodiazepine-1-carboxylate for use as a therapeutically active substance.

24. A process for the manufacture of compounds in accordance with any one of claims 1 to

21, characterized by

a) trans-esterifying a carboxylic acid ester of the general formula

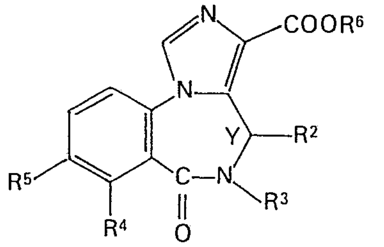

II

wherein $R^6$ signifies lower alkyl or the group $-(A)_n-R^1$ and A, n, $R^1$, $R^2$, $R^3$, $R^4$ and $R^5$ have the significance given in claim 1, with an alcohol of the general formula

$HO-(A)_n-R^1$ III

wherein A, n and $R^1$ have the significance given in claim 1, which yields the desired residue $-(A)_n-R^1$, or

b) esterifying a carboxylic acid of the general formula

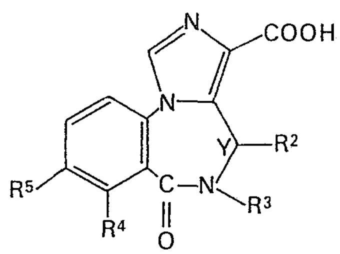

IV

wherein $R^2$, $R^3$, $R^4$ and $R^5$ have the significance given in claim 1, with an agent which yields the residue $-(A)_n-R^1$, or

c) reacting a compound of the general formula

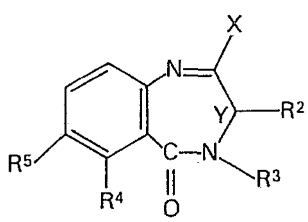

V

wherein X signifies a leaving group and $R^2$, $R^3$, $R^4$ and $R^5$ have the significance given in claim 1, in the presence of a base with an isocyanoacetic ester of the general formula

$CN-CH_2-COO-(A)_n-R^1$ VI

wherein A, n and $R^1$ have the significance given in claim 1, or

d) replacing the halogen atom in a compound of formula I in which one of the residues $R^4$ and $R^5$ signifies halogen and the other signifies hydrogen, trifluoromethyl, amino, nitro, cyano or lower alkyl and A, n, $R^1$, $R^2$ and $R^3$ have the significance given in claim 1 by the cyano group, or

e) replacing the amino group in a compound of formula I in which one of the residues $R^4$ and $R^5$ signifies amino and the other signifies hydrogen, halogen, trifluoromethyl, nitro, cyano or lower alkyl and A, n, $R^1$, $R^2$ and $R^3$ have the significance given in claim 1 by a hydrogen or halogen atom or by a cyano or nitro group, or

f) halogenating a compound of formula I in which one of the residues $R^4$ and $R^5$ signifies amino and the other signifies hydrogen and A, n, $R^1$, $R^2$ and $R^3$ have the significance given in claim 1 in the α-position to the amino group, or

g) reducing the nitro group in a compound of formula I in which one of the residues $R^4$ and $R^5$ signifies nitro and the other signifies hydrogen and A, n, $R^1$, $R^2$ and $R^3$ have the significance given in claim 1 to the amino group, and

h) if desired, converting a compound of formula I obtained into a pharmaceutically acceptable acid addition salt.

25. A medicament containing a compound in accordance with any one of claims 1 to 21 and therapeutically inert excipient.

26. A medicament containing cyclopropylmethyl (S)-8-chloro-12,12a-dihydro-9-oxo-9H,11H-azeto[2,1-c]imidazo[1,5-a][1,4]benzodiazepine-1-carboxylate.

**Revendications pour les Etats contractants: BE, CH, DE, FR, GB, IT, LI, LU, NL et SE**

1. Imidazobenzodiazépines de formule générale

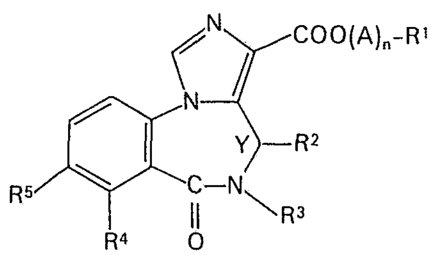

I

dans laquelle A représente un groupe alkylène inférieur, n est égal à 0 ou 1, $R^1$ représente un groupe alcynyle inférieur, alcényle inférieur, phényle éventuellement substitué par halogène, alkyloxy inférieur, alkyle inférieur, trifluorométhyle ou nitro, cycloalkyle en C3-C8 ou cycloalcényle en C5-C8 chacun éventuellement substitué par un groupe alkyle inférieur, ou un hétérocycle saturé ou insaturé à 5 ou 6 chaînons éventuellement substitué par un groupe alkyle inférieur et qui contient en tant que chaînon cyclique un atome d'oxygène ou de soufre, $R^4$ et $R^5$ représentent chacun l'hydrogène, un halogène, un groupe trifluorométhyle, cyano, nitro, amino ou alkyle inférieur et $R^2$ représente l'hydrogène et $R^3$ un groupe alkyle inférieur ou bien $R^2$ et $R^3$ forment ensemble un groupe diméthylène, triméthylène ou propénylène, les composés de formule I dans laquelle $R^2$ et $R^3$ forment ensemble un groupe diméthylène, triméthylène ou propénylène présentant la configuration (S) ou (R,S) par rapport à l'atome

de carbone marqué γ, et les groupes qualifiés d'«inférieurs» contenant au maximum 7 atomes de carbone, et leurs sels acceptables pour l'usage pharmaceutique formé par addition avec des acides.

2. Composés selon la revendication 1, caractérisés en ce que n est égal à 0 ou bien n est égal à 1 et A représente un groupe méthylène ou 1,2-éthylène éventuellement substitué par un groupe alkyle contenant au maximum 7 atomes de carbone.

3. Composés selon la revendication 1 ou 2, caractérisés en ce que $R^1$ représente un groupe alcynyle, phényle ou cycloalkyle en C3–C8 ou cycloalcényle en C5–C8, ces deux derniers éventuellement substitués par un groupe alkyle qui contient au maximum 7 atomes de carbone.

4. Composés selon la revendication 3, caractérisés en ce que $R^1$ représente un groupe cycloalkyle en C3–C6 éventuellement substitué par un groupe alkyle contenant au maximum 7 atomes de carbone.

5. Composés selon la revendication 4, caractérisés en ce que le groupe $-(A)_n-R^1$ est un groupe cyclohexyle, 2-cyclohexène-1-yle, cyclopropylméthyle, 1-cyclopropyléthyle ou 2-cyclopropyléthyle.

6. Composés selon l'une des revendications 1 à 5, caractérisés en ce que $R^2$ et $R^3$ forment ensemble un groupe diméthylène, triméthylène, ou propénylène et les composés correspondant de formule I ont la configuration (S) par rapport à l'atome de carbone marqué γ.

7. Composés selon l'une des revendications 1 à 6, caractérisés en ce que $R^4$ représente l'hydrogène, un halogène, un groupe trifluorométhyle, nitro, cyano ou alkyle contenant au maximum 7 atomes de carbone.

8. Composés selon l'une des revendications 1 à 7, caractérisés en ce que $R^5$ représente l'hydrogène ou un halogène.

9. Le (S)-8-chloro-12,12a-dihydro-9-oxo-9H,11H-azéto[2,1-c]imidazo[1,5-a][1,4]benzodiazépine-1-carboxylate de cyclopropylméthyle.

10. Le (S)-8-bromo-11,12,13,13a-tétrahydro-9-oxo-9H-imidazo[1,5-a]pyrrolo[2,1-c][1,4]benzodiazépine-1-carboxylate de cyclohexyle.

11. Le (S)-8-chloro-11,12,13,13a-tétrahydro-9-oxo-9H-imidazo[1,5-a]pyrrolo[2,1-c][1,4]benzodiazépine-1-carboxylate de (R,S)-2-cyclohexène-1-yle.

12. Le (S)-8-chloro-12,12a-dihydro-9-oxo-9H,11H-azéto[2,1-c]imidazo[1,5-a][1,4]benzodiazépine-1-carboxylate de cyclohexyle.

13. Le (S)-7-fluoro-12,12a-dihydro-9-oxo-9H,11H-azéto[2,1-c]imidazo[1,5-a][1,4]benzodiazépine-1-carboxylate de cyclohexyle.

14. Le (S)-8-chloro-7-fluoro-11,12,13,13a-tétrahydro-9-oxo-9H-imidazo[1,5-a]pyrrolo[2,1-c][1,4]benzodiazépine-1-carboxylate de cyclohexyle.

15. Le (S)-8-chloro-12,12a-dihydro-9-oxo-9H,11H-azéto[2,1-c]imidazo[1,5-a][1,4]benzodiazépine-1-carboxylate de (R,S)-1-cyclopropylèthyle.

16. Le (S)-12,12a-dihydro-8-iodo-9-oxo-9H,11H-azéto[2,1-c]imidazo[1,5-a][1,4]benzodiazépine-1-carboxylate de cyclohexyle.

17. Le (S)-7-fluoro-11,12,13,13a-tétrahydro-9-oxo-9H-imidazo[1,5-a]pyrrolo[2,1-c][1,4]benzodiazépine-1-carboxylate de cyclopropylméthyle.

18. Le (S)-8-chloro-11,12,13,13a-tétrahydro-9-oxo-9H-imidazo[1,5-a]pyrrolo[2,1-c][1,4]benzodiazépine-1-carboxylate de (R,S)-1-cyclopropyléthyle.

19. Le (S)-7-fluoro-12,12a-dihydro-9-oxo-9H,11H-azéto[2,1-c]imidazo[1,5-a][1,4]benzodiazépine-1-carboxylate de cyclopropylméthyle.

20. Le (S)-11,12,13,13a-tétrahydro-9-oxo-8-(trifluorométhyle)-9H-imidazo[1,5-a]pyrrolo[2,1-c][1,4]benzodiazépine-1-carboxylate de cyclohexyle.

21. Le (S)-8-chloro-11,12,13,13a-tétrahydro-9-oxo-9H-imidazo[1,5-a]pyrrolo[2,1-c][1,4]benzodiazépine-1-carboxylate de cyclohexyle, le (S)-8-chloro-11,12,13,13a-tétrahydro-9-oxo-9H-imidazo[1,5-a]pyrrolo[2,1-c][1,4]benzodiazépine-1-carboxylate de 2-propynyle, le 8-chloro-11,13a-dihydro-9-oxo-9H-imidazo[1,5-a]pyrrolo[2,1-c][1,4]benzodiazépine-1-carboxylate de cyclopropylméthyle, le (S)-8-chloro-11,12,13,13a-tétrahydro-9-oxo-9H-imidazo[1,5-a]pyrrolo[2,1-c][1,4]benzodiazépine-1-carboxylate de cyclopropylméthyle, le (S)-8-bromo-11,12,13,13a-tétrahydro-9-oxo-9H-imidazo[1,5-a]pyrrolo[2,1-c][1,4]benzodiazépine-1-carboxylate de cyclopropylméthyle, le (S)-11,12,13,13a-tétrahydro-8-iodo-9-oxo-9H-imidazo[1,5-a]pyrrolo[2,1-c][1,4]benzodiazépine-1-carboxylate de cyclopropylmèthyle, le (S)-8-chloro-11,12,13,13a-tétrahydro-9-oxo-9H-imidazo[1,5-a]pyrrolo[2,1-c][1,4]benzodiazépine-1-carboxylate de 2-cyclopropylèthyle, le (S)-12,12a-dihydro-8-méthyl-9-oxo-9H,11H-azéto[2,1-c]imidazo[1,5-a][1,4]benzodiazépine-1-carboxylate de cyclohexyle, le (S)-8-chloro-11,12,13,13a-tétrahydro-9-oxo-9H-imidazo[1,5-a]pyrrolo[2,1-c][1,4]benzodiazépine-1-carboxylate de rac-cis-3-méthylcyclohexyle, le (S)-12,12a-dihydro-8-iodo-9-oxo-9H,11H-azéto[2,1-c]imidazo[1,5-a][1,4]benzodiazépine-1-carboxylate de cyclopentyle, le (S)-8-chloro-11,12,13,13a-tétrahydro-9-oxo-9H-imidazo[1,5-a]pyrrolo[2,1-c][1,4]benzodiazépine-1-carboxylate de cycloheptyle, le (S)-8-chloro-12,12a-dihydro-9-oxo-9H,11H-azéto[2,1-c]imidazo[1,5-a][1,4]benzodiazépine-1-carboxylate de benzyle, le (S)-12,12a-dihydro-8-iodo-9-oxo-9H,11H-azéto[2,1-c]imidazo[1,5-a][1,4]benzodiazépine-1-carboxylate de cyclopropylméthyle, le (S)-8-bromo-12,12a-dihydro-9-oxo-9H,11H-azéto[2,1-c]imidazo[1,5-a][1,4]benzodiazépine-1-carboxylate de cyclohexyle, le (S)-8-bromo-12,12a-dihydro-9-oxo-9H,11H-azéto[2,1-c]imidazo[1,5-a][1,4]benzodiazépine-1-carboxylate de cyclopropylméthyle, le (S)-11,12,13,13a-tétrahydro-8-nitro-9-oxo-9H-imidazo[1,5-a]pyrrolo[2,1-c][1,4]benzodiazépine-1-carboxylate de cyclohexyle et le (S)-8-cyano-11,12,13,13a-tétrahydro-9-oxo-9H-imidazo[1,5-a]pyrrolo[2,1-c][1,4]benzodiazépine-1-carboxylate de cyclohexyle.

22. Composés selon l'une des revendications 1 à 21 pour l'utilisation en tant que substances actives thérapeutiques.

23. Le (S)-8-chloro-12,12a-dihydro-9-oxo-9H,11H-azéto[2,1-c]imidazo[1,5-a][1,4]benzodiazépine-1-carboxylate de cyclopropylméthyle pour l'utilisation en tant que substance active thérapeutique.

24. Procédé de préparation des composés selon l'une des revendications 1 à 21, caractérisé en ce que

a) on transestérifie un ester d'acide carboxylique de formule générale

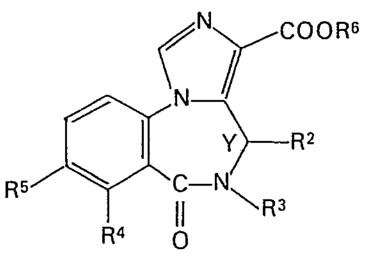

II

dans laquelle $R^6$ représente un groupe alkyle inférieur ou le groupe $-(A)_n-R^1$ et A, n, $R^1$, $R^2$, $R^3$, $R^4$ et $R^5$ ont les significations indiquées dans la revendication 1, par un alcool apportant le reste $-(A)_n-R^1$ voulu, qui répond à la formule générale

$HO-(A)_n-R^1$ III

dans laquelle A, n et $R^1$ ont les significations indiquées dans la revendication 1, ou bien

b) on estérifie un acide carboxylique de formule générale

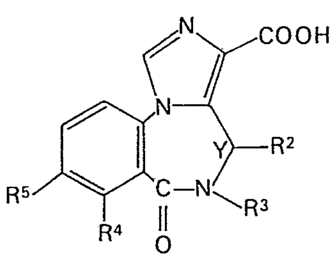

IV

dans laquelle $R^2$, $R^3$, $R^4$ et $R^5$ ont les significations indiquées dans la revendication 1, par un réactif apportant le reste $-(A)_n-R^1$, ou bien

c) on fait réagir un composé de formule générale

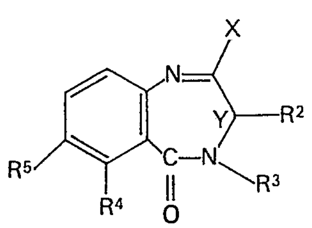

V

dans laquelle X représente un groupe éliminable et $R^2$, $R^3$, $R^4$ et $R^5$ ont les significations indiquées dans la revendication 1, en présence d'une base, avec un ester isocyanacétique de formule générale

$CN-CH_2-COO-(A)_n-R^1$ VI

dans laquelle A, n et $R^1$ ont les significations indiquées dans la revendication 1, ou bien

d) dans un composé de formule I, dans laquelle l'un des symboles $R^4$ et $R^5$ représente un halogène et l'autre l'hydrogène, un groupe trifluorométhyle, amino, nitro, cyano ou alkyle inférieur et A, n, $R^1$, $R^2$ et $R^3$ ont les significations indiquées dans la revendication 1, on remplace l'atome d'halogène par le groupe cyano; ou bien

e) dans un composé de formule I, dans laquelle l'un des symboles $R^4$ et $R^5$ représente un groupe amino et l'autre l'hydrogène, un halogène, un groupe trifluorométhyle, nitro, cyano ou alkyle inférieur et A, n, $R^1$, $R^2$ et $R^3$ ont les significations indiquées dans la revendication 1, on remplace le groupe amino par un atome d'hydrogène ou d'halogène ou par un groupe cyano ou nitro; ou bien

f) on halogène en position alpha du groupe amino un composé de formule I dans laquelle l'un des symboles $R^4$ et $R^5$ représente un groupe amino et l'autre l'hydrogène, A, n, $R^1$, $R^2$ et $R^3$ ayant les significations indiquées dans la revendication 1, ou bien

g) dans un composé de formule I dans laquelle l'un des symboles $R^4$ et $R^5$ représente un groupe nitro et l'autre l'hydrogène, A, n, $R^1$, $R^2$ et $R^3$ ayant les significations indiquées dans la revendication 1, on réduit le groupe nitro en groupe amino; et

h) si on le désire, on convertit un composé obtenu, répondant à la formule I, en un sel acceptable pour l'usage pharmaceutique par addition avec un acide.

25. Médicament contenant un composé selon l'une des revendications 1 à 21 et un excipient inerte du point de vue thérapeutique.

26. Médicament contenant du (S)-8-chloro-12,12a-dihydro-9-oxo-9H,11H-azéto[2,1-c]imidazo[1,5-a][1,4]benzodiazépine-1-carboxylate de cyclopropylméthyle.

**Claims for the Contracting State: AT**

1. A process for the manufacture of imidazobenzodiazepines of the general formula

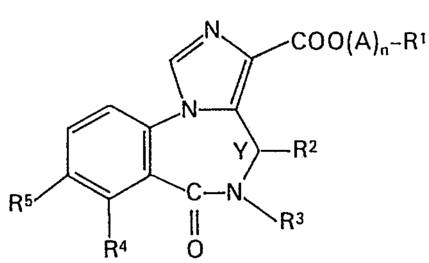

I

wherein A signifies lower alkylene, n signifies the number 0 or 1, $R^1$ signifies lower alkynyl, lower

alkenyl, phenyl optionally substituted by halogen, lower alkyloxy, lower alkyl, trifluoromethyl or nitro, $(C_{3-8})$-cycloalkyl or $(C_{5-8})$-cycloalkenyl in each case optionally substituted by lower alkyl, or a 5- or 6-membered saturated or unsaturated heterocycle which contains an oxygen or sulphur atom as a ring member and which is optionally substituted by lower alkyl, $R^4$ and $R^5$ each signify hydrogen, halogen, trifluoromethyl, cyano, nitro, amino or lower alkyl and either $R^2$ signifies hydrogen and $R^3$ signifies lower alkyl or $R^2$ and $R^3$ together signify dimethylene, trimethylene or propenylene, whereby the compounds of formula I in which $R^2$ and $R^3$ together signify dimethylene, trimethylene of propenylene have the (S)- or (R,S)-configuration with reference to the carbon atom denoted by γ and whereby the residues denoted as «lower» have a maximum of 7 carbon atoms, and pharmaceutically acceptable acid addition salts thereof, characterized by

a) trans-esterifying a carboxylic acid ester of the general formula

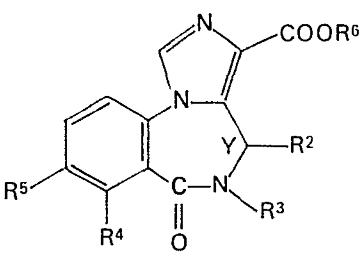

II

wherein $R^6$ signifies lower alkyl or the group $-(A)_n-R^1$ and A, n, $R^1$, $R^2$, $R^3$, $R^4$ and $R^5$ have the above significance, with an alcohol of the general formula

$HO-(A)_n-R^1$ III

wherein A, n and $R^1$ have the above significance, which yields the desired residue $-(A)_n-R^1$, or

b) esterifying a carboxylic acid of the general formula

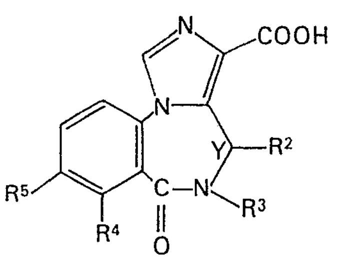

IV

wherein $R^2$, $R^3$, $R^4$ and $R^5$ have the above significance, with an agent which yields the residue $-(A)_n-R^1$, or

c) reacting a compound of the general formula

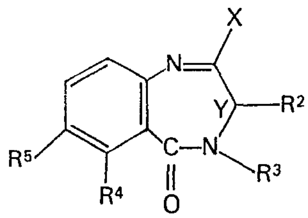

V

wherein X signifies a leaving group and $R^2$, $R^3$, $R^4$ and $R^5$ have the above significance, in the presence of a base with an isocyanoacetic ester of the general formula

$CN-CH_2-COO-(A)_n-R^1$ VI

wherein A, n and $R^1$ have the above significance, or

d) replacing the halogen atom in a compound of formula I in which one of the residues $R^4$ and $R^5$ signifies halogen and the other signifies hydrogen, trifluoromethyl, amino, nitro, cyano or lower alkyl and A, n, $R^1$, $R^2$ and $R^3$ have the above significance by the cyano group, or

e) replacing the amino group in a compound of formula I in which one of the residues $R^4$ and $R^5$ signifies amino and the other signifies hydrogen, halogen, trifluoromethyl, nitro, cyano or lower alkyl and A, n, $R^1$, $R^2$ and $R^3$ have the above significance by a hydrogen or halogen atom or by a cyano or nitro group, or

f) halogenating a compound of formula I in which one of the residues $R^4$ and $R^5$ signifies amino and the other signifies hydrogen and A, n, $R^1$, $R^2$ and $R^3$ have the above significance in the α-position to the amino group, or

g) reducing the nitro group in a compound of formula I in which one of the residues $R^4$ and $R^5$ signifies nitro and the other signifies hydrogen and A, n, $R^1$, $R^2$ and $R^3$ have the above significance to the amino group, and

h) if desired, converting a compound of formula I obtained into a pharmaceutically acceptable acid addition salt.

2. A process in accordance with claim 1, characterized in that n signifies the number 0 or in that n signifies the number 1 and A signifies methylene or 1,2-ethylene optionally substituted by alkyl which has a maximum of 7 carbon atoms.

3. A process in accordance with claim 1 or 2, characterized in that $R^1$ signifies alkynyl, phenyl, or $(C_{3-8})$-cycloalkyl or $(C_{5-8})$-cycloalkenyl in each case optionally substituted by alkyl which has a maximum of 7 carbon atoms.

4. A process in accordance with claim 3, characterized in that $R^1$ signifies $(C_{3-6})$-cycloalkyl optionally substituted by alkyl which has a maximum of 7 carbon atoms.

5. A process in accordance with claim 4, characterized in that the group $-(A)_n-R^1$ signifies cyclohexyl, 2-cyclohexen-1-yl, cyclopropylmethyl, 1-cyclopropylethyl or 2-cyclopropylethyl.

6. A process in accordance with any one of claims 1 to 5, characterized in that $R^2$ and $R^3$ together signify dimethylene, trimethylene or propenylene and the corresponding compounds of formula I have the (S)-configuration with reference to the carbon atom denoted by γ.

7. A process in accordance with any one of claims 1 to 6, characterized in that $R^4$ signifies hydrogen, halogen, trifluoromethyl, nitro, cyano or alkyl which has a maximum of 7 carbon atoms.

8. A process in accordance with any one of claims 1 to 7, characterized in that $R^5$ signifies hydrogen or halogen.

9. A process in accordance with claim 1, characterized in that cyclopropylmethyl (S)-8-chloro-12,12a-dihydro-9-oxo-9H,11H-azeto[2,1-c]imidazo[1,5-a][1,4]benzodiazepine-1-carboxylate is manufactured.

10. A process in accordance with claim 1, characterized in that cyclohexyl (S)-8-bromo-11,12,13,13a-tetrahydro-9-oxo-9H-imidazo[1,5-a]pyrrolo[2,1-c][1,4]benzodiazepine-1-carboxylate is manufactured.

11. A process in accordance with claim 1, characterized in that (R,S)-2-cyclohexen-1-yl (S)-8-chloro-11,12,13,13a-tetrahydro-9-oxo-9H-imidazo[1,5-a]pyrrolo[2,1-c][1,4]benzodiazepine-1-carboxylate is manufactured.

12. A process in accordance with claim 1, characterized in that cyclohexyl (S)-8-chloro-12,12a-dihydro-9-oxo-9H,11H-azeto[2,1-c]imidazo[1,5-a][1,4]benzodiazepine-1-carboxylate is manufactured.

13. A process in accordance with claim 1, characterized in that cyclohexyl (S)-7-fluoro-12,12a-dihydro-9-oxo-9H,11H-azeto[2,1-c]imidazo[1,5-a][1,4]benzodiazepine-1-carboxylate is manufactured.

14. A process in accordance with claim 1, characterized in that cyclohexyl (S)-8-chloro-7-fluoro-11,12,13,13a-tetrahydro-9-oxo-9H-imidazo[1,5-a]pyrrolo[2,1-c][1,4]benzodiazepine-1-carboxylate is manufactured.

15. A process in accordance with claim 1, characterized in that (R,S)-1-cyclopropylethyl (S)-8-chloro-12,12a-dihydro-9-oxo-9H,11H-azeto[2,1-c]imidazo[1,5-a][1,4]benzodiazepine-1-carboxylate is manufactured.

16. A process in accordance with claim 1, characterized in that cyclohexyl (S)-12,12a-dihydro-8-iodo-9-oxo-9H,11H-azeto[2,1-c]imidazo[1,5-a][1,4]benzodiazepine-1-carboxylate is manufactured.

17. A process in accordance with claim 1, characterized in that cyclopropylmethyl (S)-7-fluoro-11,12,13,13a-tetrahydro-9-oxo-9H-imidazo[1,5-a]pyrrolo[2,1-c][1,4]benzodiazepine-1-carboxylate is manufactured.

18. A process in accordance with claim 1, characterized in that (R,S)-1-cyclopropylethyl (S)-8-chloro-11,12,13,13a-tetrahydro-9-oxo-9H-imidazo[1,5-a]pyrrolo[2,1-c][1,4]benzodiazepine-1-carboxylate is manufactured.

19. A process in accordance with claim 1, characterized in that cyclopropylmethyl (S)-7-fluoro-12,12a-dihydro-9-oxo-9H,11H-azeto[2,1-c]imidazo[1,5-a][1,4]benzodiazepine-1-carboxylate is manufactured.

20. A process in accordance with claim 1, characterized in that cyclohexyl (S)-11,12,13,13a-tetrahydro-9-oxo-8-(trifluoromethyl)-9H-imidazo[1,5-a]pyrrolo[2,1-c][1,4]benzodiazepine-1-carboxylate is manufactured.

**Revendications pour l'Etat contractant: AT**

1. Procédé de préparation d'imidazobenzodiazépines de formule générale

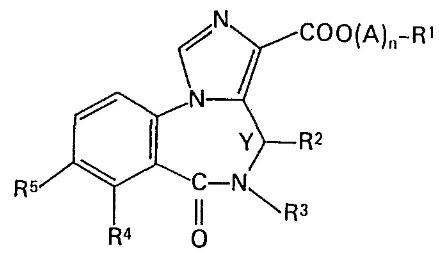

I

dans laquelle A représente un groupe alkylène inférieur, n est égal à 0 ou 1, $R^1$ représente un groupe alcynyle inférieur, alcényle inférieur, phényle éventuellement substitué par halogène, alkyloxy inférieur, alkyle inférieur, trifluorométhyle ou nitro, cycloalkyle en C3–C8 ou cycloalcényle en C5–C8 éventuellement substitué chacun par un groupe alkyle inférieur, ou un hétérocycle saturé ou insaturé à 5 ou 6 chaînons éventuellement substitué par un groupe alkyle inférieur et qui contient en tant que chaînon cyclique un atome d'oxygène ou de soufre, $R^4$ et $R^5$ représentent chacun l'hydrogène, un halogène, un groupe trifluorométhyle, cyano, nitro, amino ou alkyle inférieur, et $R^2$ représente l'hydrogène et $R^3$ un groupe alkyle inférieur ou bien $R^2$ et $R^3$ forment ensemble un groupe diméthylène, triméthylène ou propénylène, les composés de formule I dans laquelle $R^2$ et $R^3$ forment ensemble un groupe diméthylène, triméthylène ou propénylène ayant la configuration (S) ou (R,S) par rapport à l'atome de carbone marqué γ et les groupes qualifiés d'«inférieurs» contenant au maximum 7 atomes de carbone, et de leurs sels acceptables pour l'usage pharmaceutique formés par addition avec des acides, caractérisé en ce que

a) on transestérifie un ester d'acide carboxylique de formule générale

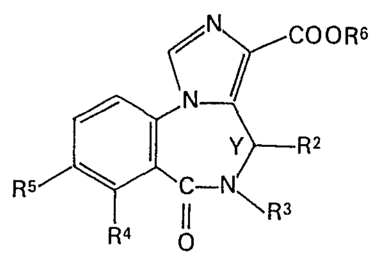

II

dans laquelle $R^6$ représente un groupe alkyle inférieur ou le groupe $-(A)_n-R^1$ et A, n, $R^1$, $R^2$, $R^3$, $R^4$ et $R^5$ ont les significations indiquées ci-dessus, par un alcool apportant le reste $-(A)_n-R^1$ voulu et répondant à la formule générale

$HO-(A)_n-R^1$ III

dans laquelle A, n et $R^1$ ont les significations indiquées ci-dessus, ou bien

b) on estérifie un acide carboxylique de formule générale

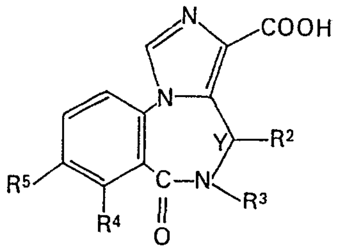

IV

dans laquelle $R^2$, $R^3$, $R^4$ et $R^5$ ont les significations indiquées ci-dessus par un réactif apportant le reste $-(A)_n-R^1$, ou bien

c) on fait réagir un composé de formule générale

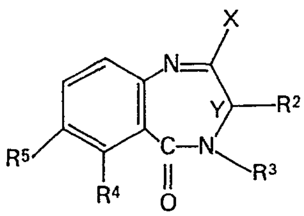

V

dans laquelle X représente un groupe éliminable et $R^2$, $R^3$, $R^4$ et $R^5$ ont les significations indiquées ci-dessus, en présence d'une base, avec un ester isocyanacétique de formule générale

$CN-CH_2-COO-(A)_n-R^1$ VI

dans laquelle A, n et $R^1$ ont les significations indiquées ci-dessus, ou bien

d) dans un composé de formule I dans laquelle l'un des symboles $R^4$ et $R^5$ représente un halogène et l'autre l'hydrogène, un groupe trifluorométhyle, amino, nitro, cyano ou alkyle inférieur, A, n, $R^1$, $R^2$ et $R^3$ ayant les significations indiquées ci-dessus, on remplace l'atome d'halogène par le groupe cyano, ou bien

e) dans un composé de formule I dans laquelle l'un des symboles $R^4$ et $R^5$ représente un groupe amino et l'autre l'hydrogène, un halogène, un groupe trifluorométhyle, nitro, cyano ou alkyle inférieur, A, n, $R^1$, $R^2$ et $R^3$ ayant les significations indiquées ci-dessus, on remplace le groupe amino par un atome d'hydrogène ou d'halogène ou par un groupe cyano ou nitro, ou bien

f) on halogène en position alpha du groupe amino un composé de formule I dans laquelle l'un des symboles $R^4$ et $R^5$ est un groupe amino et l'autre représente l'hydrogène, A, n, $R^1$, $R^2$ et $R^3$ ayant les significations indiquées ci-dessus, ou bien

g) dans un composé de formule I dans laquelle l'un des symboles $R^4$ et $R^5$ représente un groupe nitro et l'autre l'hydrogène, A, n, $R^1$, $R^2$ et $R^3$ ayant les significations indiquées ci-dessus, on réduit le groupe nitro en groupe amino, et

h) on convertit si on le désire un composé obtenu, répondant à la formule I, en un sel acceptable pour l'usage pharmaceutique par addition avec un acide.

2. Procédé selon la revendication 1, caractérisé en ce que n est égal à 0 ou en ce que n est égal à 1 et A représente un groupe méthylène ou 1,2-éthylène éventuellement substitué par un groupe alkyle contenant au maximum 7 atomes de carbone.

3. Procédé selon la revendication 1 ou 2, caractérisé en ce que $R^1$ représente un groupe alcynyle, phényle ou cycloalkyle en C3-C8 ou cycloalcényle en C5-C8, ces deux derniers éventuellement substitués chacun par un groupe alkyle contenant au maximum 7 atomes de carbone.

4. Procédé selon la revendication 3, caractérisé en ce que $R^1$ représente un groupe cycloalkyle en C3-C6 éventuellement substitué par un groupe alkyle qui contient au maximum 7 atomes de carbone.

5. Procédé selon la revendication 4, caractérisé en ce que le groupe $-(A)_n-R^1$ est un groupe cyclohexyle, 2-cyclohexène-1-yle, cyclopropylméthyle, 1-cyclopropyléthyle ou 2-cyclopropyléthyle.

6. Procédé selon l'une des revendications 1 à 5, caractérisé en ce que $R^2$ et $R^3$ forment ensemble un groupe diméthylène, triméthylène ou propénylène et les composés correspondants de formule I ont la configuration (S) par rapport à l'atome de carbone marqué $\gamma$.

7. Procédé selon l'une des revendications 1 à 6, caractérisé en ce que $R^4$ représente l'hydrogène, un halogène, un groupe trifluorométhyle, nitro, cyano ou alkyle à 7 atomes de carbone au maximum.

8. Procédé selon l'une des revendications 1 à 7, caractérisé en ce que $R^5$ représente l'hydrogène ou un halogène.

9. Procédé selon la revendication 1, caractérisé en ce que l'on prépare le (S)-8-chloro-12,12a-dihydro-9-oxo-9H,11H-azéto[2,1-c]imidazo[1,5-a][1,4]benzodiazépine-1-carboxylate de cyclopropylméthyle.

10. Procédé selon la revendication 1, caractérisé en ce que l'on prépare le (S)-8-bromo-11,12,13,13a-tétrahydro-9-oxo-9H-imidazo[1,5-a]pyrrolo[2,1-c][1,4]benzodiazépine-1-carboxylate de cyclohexyle.

11. Procédé selon la revendication 1, caractérisé en ce que l'on prépare le (S)-8-chloro-11,12,13,13a-tétrahydro-9-oxo-9H-imidazo[1,5-a]pyrrolo[2,1-c][1,4]benzodiazépine-1-carboxylate de (R,S)-2-cyclohexène-1-yle.

12. Procédé selon la revendication 1, caractérisé en ce que l'on prépare le (S)-8-chloro-12,12a-dihydro-9-oxo-9H,11H-azéto[2,1-c]imidazo[1,5-a][1,4]benzodiazépine-1-carboxylate de cyclohexyle.

13. Procédé selon la revendication 1, caractérisé en ce que l'on prépare le (S)-7-fluoro-12,12a-dihydro-9-oxo-9H,11H-azéto[2,1-c]imida-

zo[1,5-a][1,4]benzodiazépine-1-carboxylate de cyclohexyle.

14. Procédé selon la revendication 1, caractérisé en ce que l'on prépare le (S)-8-chloro-7-fluoro-11,12,13,13a-tétrahydro-9-oxo-9H-imidazo[1,5-a]pyrrolo[2,1-c][1,4]benzodiazépine-1-carboxylate de cyclohexyle.

15. Procédé selon la revendication 1, caractérisé en ce que l'on prépare le (S)-8-chloro-12,12a-dihydro-9-oxo-9H,11H-azéto[2,1-c]imidazo[1,5-a][1,4]benzodiazépine-1-carboxylate de (R,S)-1-cyclopropyléthyle.

16. Procédé selon la revendication 1, caractérisé en ce que l'on prépare le (S)-12,12a-dihydro-8-iodo-9-oxo-9H,11H-azéto[2,1-c]imidazo[1,5-a][1,4]benzodiazépine-1-carboxylate de cyclohexyle.

17. Procédé selon la revendication 1, caractérisé en ce que l'on prépare le (S)-7-fluoro-11,12,13,13a-tétrahydro-9-oxo-9H-imidazo[1,5-a]pyrrolo[2,1-c][1,4]benzodiazépine-1-carboxylate de cyclopropylméthyle.

18. Procédé selon la revendication 1, caractérisé en ce que l'on prépare le (S)-8-chloro-11,12,13,13a-tétrahydro-9-oxo-9H-imidazo[1,5-a]pyrrolo[2,1-c][1,4]benzodiazépine-1-carboxylate de (R,S)-1-cyclopropyléthyle.

19. Procédé selon la revendication 1, caractérisé en ce que l'on prépare le (S)-7-fluoro-12,12a-dihydro-9-oxo-9H,11H-azéto[2,1-c]imidazo[1,5-a][1,4]benzodiazépine-1-carboxylate de cyclopropylméthyle.

20. Procédé selon la revendication 1, caractérisé en ce que l'on prépare le (S)-11,12,13,13a-tétrahydro-9-oxo-8-(trifluorométhyl)-9H-imidazo[1,5-a]pyrrolo[2,1-c][1,4]benzodiazépine-1-carboxylate de cyclohexyle.